(19) Europäisches Patentamt
European Patent Office
Office européen des brevets

(11)  **EP 2 569 626 B1**

(12)  **EUROPEAN PATENT SPECIFICATION**

(45) Date of publication and mention
of the grant of the patent:
**27.11.2019   Bulletin 2019/48**

(21) Application number: **11781242.0**

(22) Date of filing: **11.05.2011**

(51) Int Cl.:
**G01N 33/48** *(2006.01)*      **C12Q 1/68** *(2018.01)*

(86) International application number:
**PCT/US2011/036143**

(87) International publication number:
**WO 2011/143361 (17.11.2011 Gazette 2011/46)**

(54)  **METHODS AND COMPOSITIONS FOR DIAGNOSING CONDITIONS**

VERFAHREN UND ZUSAMMENSETZUNGEN ZUR DIAGNOSE VON LEIDEN

MÉTHODES ET COMPOSITIONS UTILISÉES DANS LE CADRE DU DIAGNOSTIC DE MALADIES

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **05.10.2010  US 389810 P
11.05.2010  US 333717 P**

(43) Date of publication of application:
**20.03.2013   Bulletin 2013/12**

(73) Proprietor: **Veracyte, Inc.
South San Francisco, CA 94080 (US)**

(72) Inventors:
• **KENNEDY, Giulia, C.**
**San Francisco, CA 94116 (US)**
• **CHUDOVA, Darya, I**
**San Jose, CA 95123 (US)**
• **WILDE, Jonathan, I**
**Burlingame, CA 94010 (US)**
• **VEITCH, James, G.**
**Berkeley, CA 94705 (US)**
• **ANDERSON, Bonnie, H.**
**Half Moon Bay, CA 94019 (US)**

(74) Representative: **Avidity IP
Broers Building
Hauser Forum
21 JJ Thomson Avenue
Cambridge CB3 0FA (GB)**

(56) References cited:
**WO-A2-2010/056374      US-A1- 2005 137 805
US-A1- 2007 037 186      US-A1- 2008 145 841
US-A1- 2009 191 535      US-A1- 2010 055 704**

**US-A1- 2010 099 093**

• **DURAND S ET AL.: "Evaluation of gene
expression profiles in thyroid nodule biobsy
material to diagnose thyroid cancer", JOURNAL
OF CLINICAL ENDOCRINOLOGY &
METABOLISM, vol. 93, April 2008 (2008-04),
pages 1195-1202, XP002720663,**
• **HAUGEN B R et al.: "Development of a novel
molecular classifier to accurately identify benign
thyroid nodules in patients with inderterminate
FNA cytology", Abstract presented at 14th
International Thyroid Congres , 15 September
2010 (2010-09-15), XP002720664, Retrieved from
the Internet:
URL:https://www.google.nl/search?q=develop
ment+of+a+novel+molecular+classifier+to+ac
curately+identify+benign+thyroid+nodules&i
e=utf-8&oe=utf-8&rls=org.mozilla:en-GB:off
icial&client=firefox-a&gfe_rd=ctrl&ei=jm4E
U5Qpw-P8BqmVgMgl&gws_rd=cr [retrieved on
2014-02-19]**
• **GRIFFITH O L ET AL: "Meta-analysis and
meta-review of thyroid cancer gene expression
profiling studies identifies important diagnostic
biomarkers", JOURNAL OF CLINICAL
ONCOLOGY, AMERICAN SOCIETY OF CLINICAL
ONCOLOGY, US, vol. 24, no. 31, 1 November 2006
(2006-11-01), pages 5043-5051, XP002499803,
ISSN: 0732-183X, DOI: 10.1200/JCO.2006.06.7330**

EP 2 569 626 B1

**(Cont. next page)**

- HUANG Y ET AL: "Gene expression in papillary thyroid carcinoma reveals highly consistent profiles", PROCEEDINGS OF THE NATIONAL ACADEMY OF SCIENCES, NATIONAL ACADEMY OF SCIENCES, US, vol. 98, no. 26, 18 December 2001 (2001-12-18), pages 15044-15049, XP002343971, ISSN: 0027-8424, DOI: 10.1073/PNAS.251547398
- MAZZANTI C ET AL: "Using gene expression profiling to differentiate benign versus malignant thyroid tumors", CANCER RESEARCH, AMERICAN ASSOCIATION FOR CANCER RESEARCH, US, vol. 64, 15 April 2004 (2004-04-15), pages 2898-2903, XP002343973, ISSN: 0008-5472, DOI: 10.1158/0008-5472.CAN-03-3811
- BARDEN C B ET AL.: "Classification of follicular thyroid tumors by molecular signature: results of gene profiling", CLINICAL CANCER RESEARCH, vol. 9, May 2003 (2003-05), pages 1792-1800, XP002720665,
- PUSKAS L G ET AL.: "Gene profiling identifies genes specific for well-differentiated epithelial thyroid tumors", CELLULAR AND MOLECULAR BIOLOGY, vol. 51, 2005, pages 177-186, XP002720666,
- SAEYS Y ET AL: "A review of feature selection techniques in bioinformatics", BIOINFORMATICS, OXFORD UNIVERSITY PRESS, SURREY, GB, vol. 23, no. 19, 1 October 2007 (2007-10-01), pages 2507-2517, XP002601323, ISSN: 1367-4803, DOI: 10.1093/BIOINFORMATICS/BTM344 [retrieved on 2007-08-24]
- DÍAZ-URIARTE RAMÓN ET AL: "Gene selection and classification of microarray data using random forest", BMC BIOINFORMATICS, BIOMED CENTRAL, LONDON, GB, vol. 7, no. 1, 6 January 2006 (2006-01-06), page 3, XP021001113, ISSN: 1471-2105, DOI: 10.1186/1471-2105-7-3
- CHUDOVA D ET AL.: "Molecular classification of thyroid nodules using high-dimensionality genomic data", JOURNAL OF CLINICAL ENDOCRINOLOGY & METABOLISM, vol. 95, no. 12, 19 February 2014 (2014-02-19), pages 5296-5304, XP002720667,
- WU, M. ET AL.: 'A Comparative Study of 200 Fine Needle Aspiration Biopsies Performed by Clinicians and Cytopathologists (Abstract).' THE LARYNGOSCOPE vol. 116, no. 7, July 2006, pages 1212 - 1215, XP008158131

**Description**

**BACKGROUND OF THE INVENTION**

There is a need in the art for more accurate methods of classifying, characterizing, and diagnosing diseases or disorders. For example, cancer is one of the leading causes of mortality worldwide; yet for many patients, the process of simply clearing the first step of obtaining an accurate diagnosis is often a frustrating and time-consuming experience. This is true of many cancers, including thyroid cancer. This is also particularly true of relatively rare diseases, such as Hurthle cell adenomas and carcinomas, which account for approximately 5% of thyroid neoplasms.

An inaccurate diagnosis of cancer can lead to unnecessary follow-up procedures, including costly surgical procedures, not to mention unnecessary emotional distress to the patient. In the case of thyroid cancer, it is estimated that out of the approximately 130,000 thyroid removal surgeries performed each year due to suspected malignancy in the United States, only about 54,000 are necessary; therefore, tens of thousands of unnecessary thyroid removal surgeries are performed annually. Continued treatment costs and complications due to the need for lifelong drug therapy to replace the lost thyroid function adds can cause further economic and physical harm. Accordingly, there is a strong need for improved methods for detecting and/or diagnosing diseases such as cancer, as well as other disorders. Durand et al. Journal of Clinical Endocrinology & Metabolism, vol. 93, pages 1195 - 1202 describes evaluation of gene expression profiles in thyroid nodule biopsy material to diagnose thyroid cancer.

**SUMMARY OF THE INVENTION**

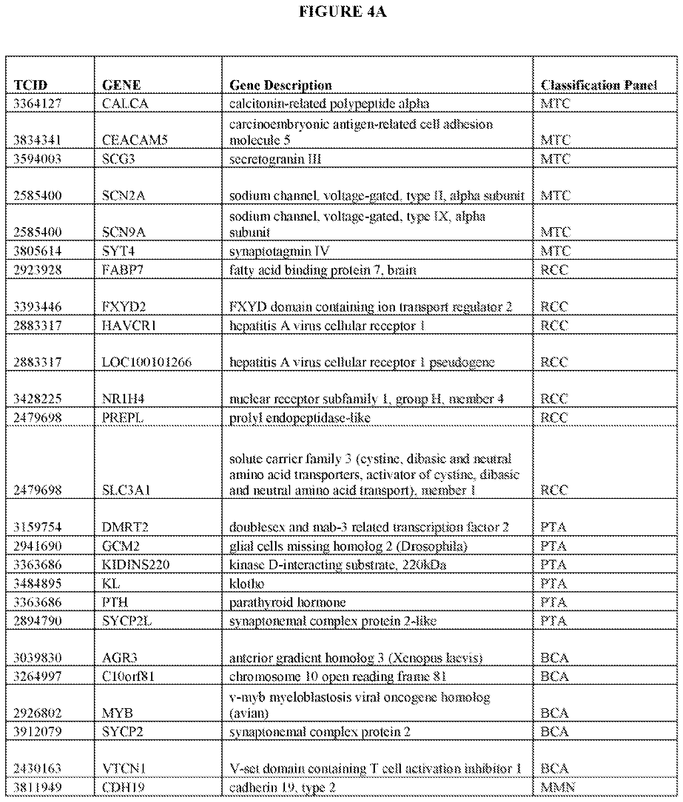

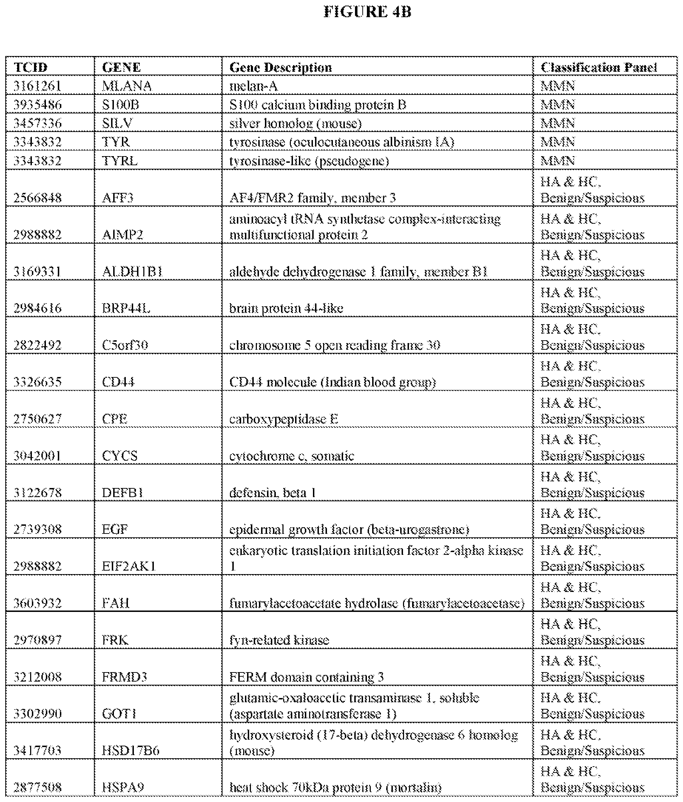

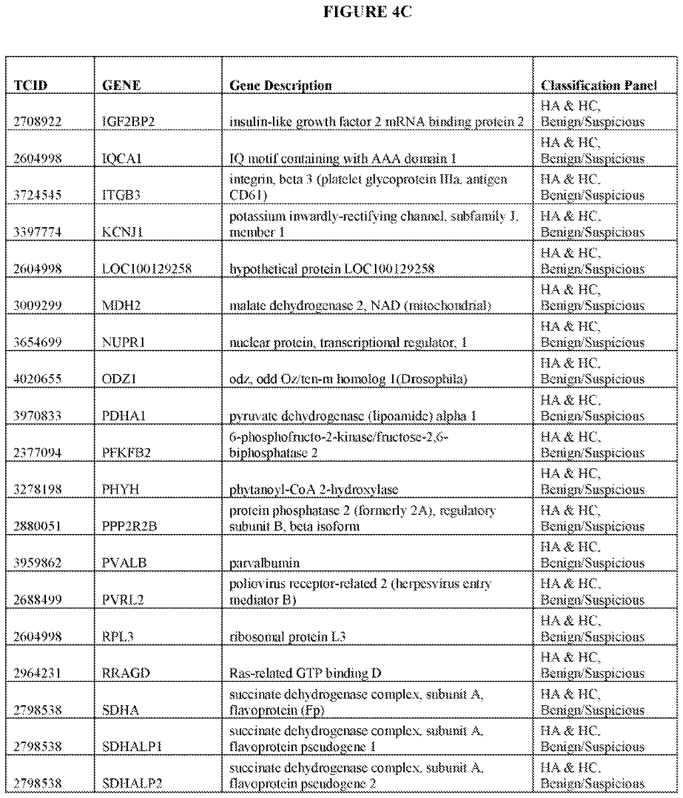

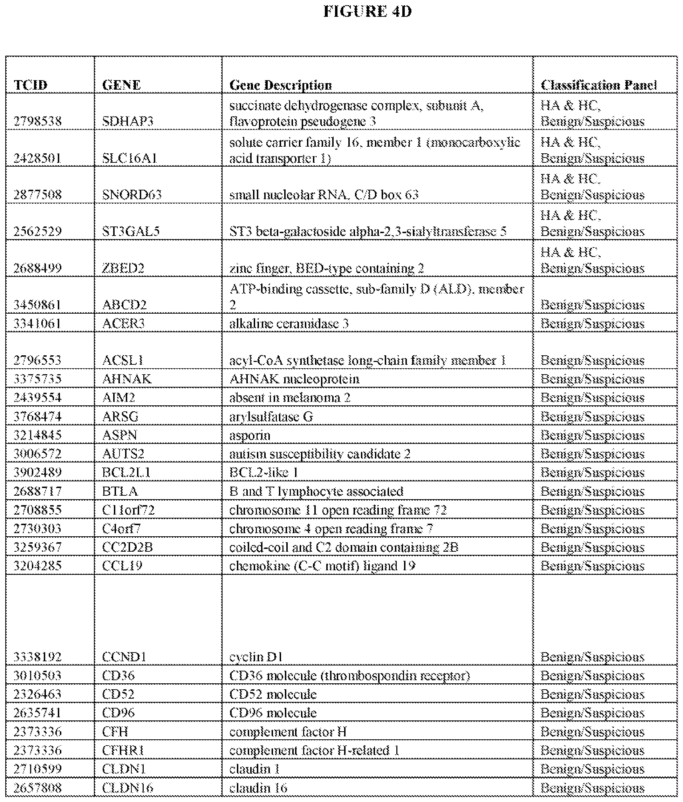

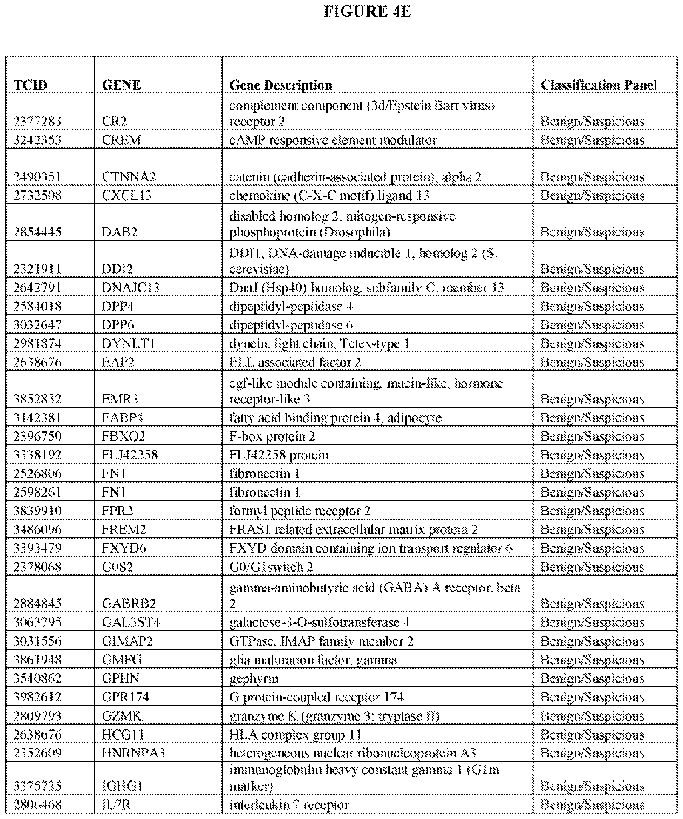

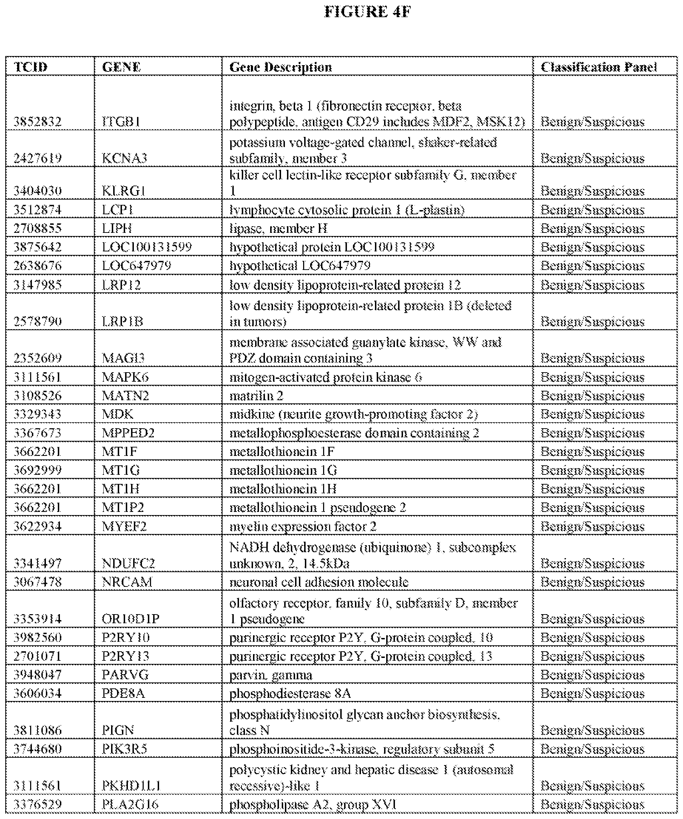

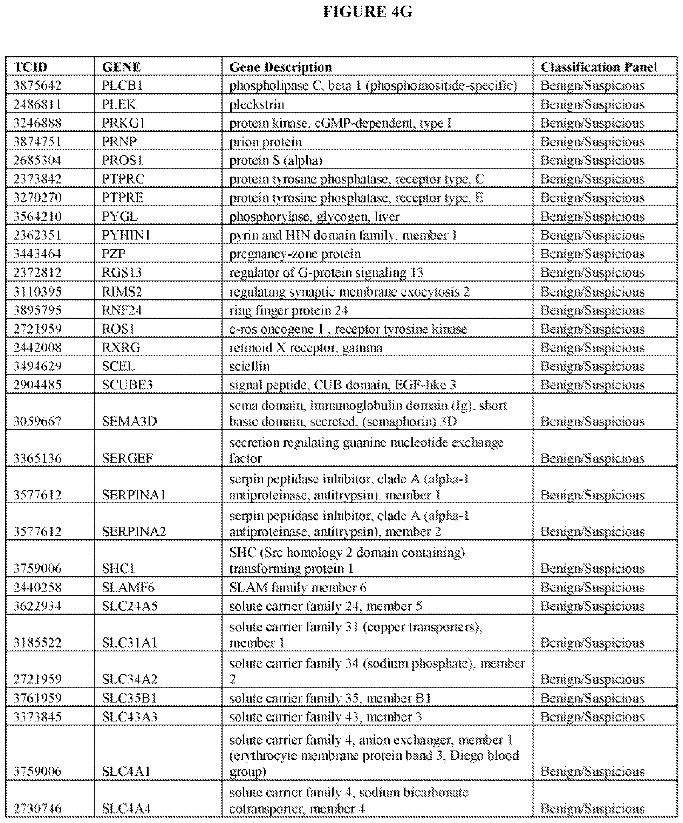

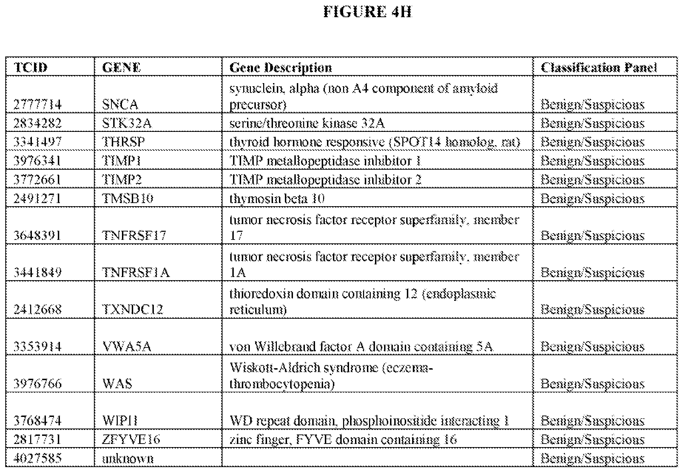

The invention provides a method for evaluating a fine needle aspiration (FNA) thyroid tissue sample obtained from a subject, comprising: (a) assaying expression levels of at least a first panel of at least 5 genes selected from Figure 4 and of at least a second panel of at least 5 genes selected from Figure 4 in said FNA thyroid tissue sample, wherein said first panel is different than said second panel; and (b) using a machine learning algorithm that has been trained using a plurality of reference samples comprising surgical biopsy and FNA samples to classify the FNA thyroid tissue sample as benign or malignant at a negative predictive value (NPV) of at least 95% by (i) first comparing said expression levels to at least a first reference set of gene expression data of said first panel obtained from said plurality of reference samples, wherein said at least one first reference set comprises gene expression levels associated with a given thyroid tissue type, and wherein said first comparing does not rule-in or rule-out said FNA thyroid tissue sample as benign or malignant, and (ii) subsequently comparing said expression levels to a second reference set of gene expression data of said second panel obtained from said plurality of reference samples, wherein said subsequently comparing does rule-in or rule-out said FNA thyroid tissue sample as benign or malignant, thereby classifying said FNA thyroid tissue sample. In some embodiments, the method further comprises providing a thyroid tissue sample collected from a subject for use in step (a). In some embodiments, the sequential comparison ends with comparing said expression level to gene expression data for a final set of biomarkers by analyzing said expression level using a main classifier, said main classifier obtained from gene expression data from one or more sets of biomarkers. In some embodiments, the main classifier is obtained from gene expression data comprising one or more reference gene expression levels correlated with the presence of one or more of the following tissue types: follicular thyroid adenoma, follicular thyroid carcinoma, nodular hyperplasia, papillary thyroid carcinoma, follicular variant of papillary carcinoma, Hurthle cell carcinoma, Hurthle cell adenoma, and lymphocytic thyroiditis. In some embodiments, the sequential comparing begins with comparing said expression level to one or more sets of biomarkers comprising one or more reference gene expression levels correlated with the presence of one or more of the following tissue types: medullary thyroid carcinoma, renal carcinoma metastasis to the thyroid, parathyroid, breast carcinoma metastasis to the thyroid, and melanoma metastasis to the thyroid. In some embodiments, the sequentially comparing comprises inputting said thyroid tissue sample expression level to a computer system comprising gene expression data corresponding to said plurality of reference gene expression levels. In some embodiments, the sequentially comparing is performed by an algorithm trained by said gene expression data obtained from said plurality of reference samples. The algorithm may be trained using a plurality of clinical samples, such as more than 200 clinical samples, which clinical samples may comprise one or more thyroid tissue sample obtained by fine needle aspiration (FNA) and one or more thyroid tissue sample obtained by surgical biopsy. In some embodiments, the algorithm is trained using samples derived from at least 5 different geographical locations.

In one embodiment, the method comprises (a) determining an expression level for one or more gene expression products from said thyroid tissue sample; and (b) identifying the presence of Hurthle cell adenoma or Hurthle cell carcinoma in the thyroid tissue sample by comparing the expression level to a plurality of reference gene expression levels correlated with the presence or absence of Hurthle cell adenoma or Hurthle cell carcinoma. In some embodiments, the method further comprises providing a thyroid tissue sample collected from a subject for use in step (a). In some embodiments, the comparing step comprises inputting the thyroid tissue sample expression level to a computer system comprising gene expression data corresponding to said plurality of reference gene expression levels. In some embodiments, the comparing step is performed by an algorithm trained by the expression data obtained from the plurality of reference samples. In some embodiments, the reference gene expression levels are obtained from at least one surgical reference thyroid tissue sample collected by surgical biopsy and at least one FNA reference thyroid tissue sample collected by fine needle aspiration. In some embodiments, the at least one surgical reference thyroid tissue sample and/or the one or more RNA reference thyroid tissue sample does not comprise Hurthle cell adenoma tissue and/or Hurthle cell carcinoma tissue. In some embodiments, the one or more gene expression product corresponds to one or more genes selected from the group consisting of AFF3, AIMP2, ALDH1B1, BRP44L, C5orf30, CD44, CPE, CYCS, DEFB1, EGF, EIF2AK1, FAH, FRK, FRMD3, GOT1, HSD17B6, HSPA9, IGF2BP2, IQCA1, ITGB3, KCNJ1, LOC100129258, MDH2, NUPR1, ODZ1, PDHA1, PFKFB2, PHYH, PPP2R2B, PVALB, PVRL2, RPL3, RRAGD, SDHA, SDHALP1, SDHALP2, SDHAP3, SLC16A1, SNORD63, ST3GAL5, and ZBED2.

A method is described comprising (a) obtaining an expression level for two or more gene expression products of a thyroid tissue sample from said subject, wherein the two or more gene expression products correspond to two or more genes selected from Figure 4; and (b) identifying the biological sample as having a thyroid condition by correlating the gene expression level with the presence of a thyroid condition in the thyroid tissue sample. In some embodiments, the method has a specificity of at least 50% and/or an NPV of at least 95%. In some embodiments, the thyroid condition is a malignant thyroid condition. In some embodiments, the one or more gene expression products correspond to at least 10, or at least 20 genes selected from Figure 4.

A method is described comprising the steps of: (a) determining an expression level for one or more gene expression products from said thyroid tissue sample; (b) comparing the expression level of step (a) with gene expression data obtained from a plurality of reference samples, wherein said plurality of reference samples comprises a reference thyroid sample obtained by surgical biopsy of thyroid tissue and a reference thyroid sample obtained by fine needle aspiration of thyroid tissue; and (c) based on said correlating, (i) identifying said thyroid tissue sample as malignant, (ii) identifying said thyroid tissue sample as benign, (iii) identifying said thyroid tissue sample as non-cancerous, (iv) identifying said thyroid tissue sample as non-malignant, or (v) identifying said thyroid tissue sample as normal. In some embodiments, the method further comprises providing a thyroid tissue sample collected from a subject for use in step (a). In some embodiments, the comparing is performed by an algorithm trained by the gene expression data obtained from the plurality of reference samples, such as more than 200 samples. In some embodiments, the plurality of reference samples have pathologies selected from the group consisting of follicular thyroid adenoma, follicular thyroid carcinoma, nodular hyperplasia, papillary thyroid carcinoma, follicular variant of papillary carcinoma, lymphocytic thyroiditis, Hurthle cell adenoma, and Hurthle cell carcinoma. In some embodiments, the comparing step comprises comparing said expression level to gene expression data for at least two different sets of biomarkers, the gene expression data for each set of biomarkers comprising one or more reference gene expression levels correlated with the presence of one or more tissue types, wherein said expression level is compared to gene expression data for said at least two sets of biomarkers sequentially.

Also described is a method of selecting a treatment for a subject, such as a human subject, having or suspected of having a thyroid condition. In one embodiment, the method comprises (a) obtaining an expression level for two or more gene expression products of a thyroid tissue sample from said subject, wherein the two or more gene expression products correspond to two or more genes selected from Figure 4; and (b) selecting a treatment for said subject based on correlating the gene expression level with the presence of a thyroid condition in the thyroid tissue sample. In some embodiments, the treatment is selected from the group consisting of radioactive iodine ablation, surgery, thyroidectomy, and administering a therapeutic agent. In some embodiments, the correlating step comprises comparing said expression level to gene expression data for at least two different sets of biomarkers, the gene expression data for each set of biomarkers comprising one or more reference gene expression levels correlated with the presence of one or more tissue types, wherein said expression level is compared to gene expression data for said at least two sets of biomarkers sequentially. In some embodiments, the sequential comparison ends with comparing said expression level to gene expression data for a final set of biomarkers by analyzing said expression level using a main classifier, said main classifier obtained from gene expression data from one or more sets of biomarkers. In some embodiments, the main classifier is obtained from gene expression data comprising one or more reference gene expression levels correlated with the presence of one or more of the following tissue types: follicular thyroid adenoma, follicular thyroid carcinoma, nodular hyperplasia, papillary thyroid carcinoma, follicular variant of papillary carcinoma, Hurthle cell carcinoma, Hurthle cell adenoma, and lymphocytic thyroiditis. The thyroid condition may be selected from the group consisting of follicular thyroid adenoma, nodular hyperplasia, lymphocytic thyroiditis, Hurthle cell adenoma, follicular thyroid carcinoma, papillary thyroid carcinoma, follicular variant of papillary carcinoma, medullary thyroid carcinoma, Hurthle cell carcinoma, anaplastic thyroid carcinoma, renal carcinoma metastasis to the thyroid, breast carcinoma metastasis to the thyroid, melanoma metastasis to the thyroid, B cell lymphoma metastasis to the thyroid. The correlating may be performed by an algorithm trained by expression data obtained from a plurality of reference samples.

In some embodiments of the methods of the invention, one or more of the at least two sets of biomarkers comprises one or more gene expression product levels correlated with the presence of one or more tissue types selected from the group consisting of normal thyroid, follicular thyroid adenoma, nodular hyperplasia, lymphocytic thyroiditis, Hurthle cell adenoma, follicular thyroid carcinoma, papillary thyroid carcinoma, follicular variant of papillary carcinoma, medullary thyroid carcinoma, Hurthle cell carcinoma, anaplastic thyroid carcinoma, renal carcinoma metastasis to the thyroid, breast carcinoma metastasis to the thyroid, melanoma metastasis to the thyroid, B cell lymphoma metastasis to the thyroid, and parathyroid. In some embodiments, one or more of the at least two sets of biomarkers comprises one or more gene expression product levels correlated with the presence of one or more tissue types selected from the group consisting of follicular thyroid adenoma, follicular thyroid carcinoma, nodular hyperplasia, papillary thyroid carcinoma, follicular variant of papillary carcinoma, lymphocytic thyroiditis, Hurthle cell adenoma, and Hurthle cell carcinoma. In some embodiments, one or more of the at least two sets of biomarkers comprises one or more gene expression product levels correlated with the presence of one or more tissue types selected from the group consisting of medullary thyroid carcinoma, renal carcinoma metastasis to the thyroid, parathyroid, breast carcinoma metastasis to the thyroid, melanoma metastasis to the thyroid, Hurthle cell adenoma, and Hurthle cell carcinoma. In some embodiments, a first of the at least two sets of biomarkers comprises one or more gene expression product levels correlated with the presence of one or more tissue types selected from the group consisting of medullary thyroid carcinoma, renal carcinoma metastasis to the thyroid, parathyroid, breast carcinoma metastasis to the thyroid, melanoma metastasis to the thyroid, Hurthle cell adenoma, and Hurthle cell; and a second of the at least two sets of biomarkers comprises one or more gene expression product levels correlated with the presence of one or more tissue types selected from the group consisting of follicular thyroid adenoma, follicular thyroid carcinoma, nodular hyperplasia, papillary thyroid carcinoma, follicular variant of papillary carcinoma, lymphocytic thyroiditis, Hurthle cell adenoma, and Hurthle cell carcinoma. In some embodiments, one or more of said at least two sets of biomarkers comprises one or more gene expression product levels correlated with the presence of Hurthle cell adenoma and/or Hurthle cell carcinoma. In some embodiments of the methods of the invention, a result of classifying or identifying a sample is reported to a user via a display device.

In some embodiments of the methods of the invention, reference gene expression levels are obtained from at least one surgical reference thyroid tissue sample collected by surgical biopsy and at least one FNA reference thyroid tissue sample collected by fine needle aspiration, which may comprise at least 200 surgical biopsy samples and/or at least 200 FNA fine needle aspiration samples.. In some embodiments, the one or more gene expression products correspond to genes selected from the group consisting of AFF3, AIMP2, ALDH1B1, BRP44L, C5orf30, CD44, CPE, CYCS, DEFB1, EGF, EIF2AK1, FAH, FRK, FRMD3, GOT1, HSD17B6, HSPA9, IGF2BP2, IQCA1, ITGB3, KCNJ1, LOC100129258, MDH2, NUPR1, ODZ1, PDHA1, PFKFB2, PHYH, PPP2R2B, PVALB, PVRL2, RPL3, RRAGD, SDHA, SDHALP1, SDHALP2, SDHAP3, SLC16A1, SNORD63, ST3GAL5, ZBED2, ABCD2, ACER3, ACSL1, AHNAK, AIM2, ARSG, ASPN, AUTS2, BCL2L1, BTLA, C11orf72, C4orf7, CC2D2B, CCL19, CCND1, CD36, CD52, CD96, CFH, CFHR1, CLDN1, CLDN16, CR2, CREM, CTNNA2, CXCL13, DAB2, DDI2, DNAJC13, DPP4, DPP6, DYNLT1, EAF2, EMR3, FABP4, FBXO2, FLJ42258, FN1, FN1, FPR2, FREM2, FXYD6, G0S2, GABRB2, GAL3ST4, GIMAP2, GMFG, GPHN, GPR174, GZMK, HCG11, HNRNPA3, IGHG1, IL7R, ITGB1, KCNA3, KLRG1, LCP1, LIPH, LOC100131599, LOC647979, LRP12, LRP1B, MAGI3, MAPK6, MATN2, MDK, MPPED2, MT1F, MT1G, MT1H, MT1P2, MYEF2, NDUFC2, NRCAM, OR10D1P, P2RY10, P2RY13, PARVG, PDE8A, PIGN, PIK3R5, PKHD1L1, PLA2G16, PLCB1, PLEK, PRKG1, PRNP, PROS1, PTPRC, PTPRE, PYGL, PYHIN1, PZP, RGS13, RIMS2, RNF24, ROS1, RXRG, SCEL, SCUBE3, SEMA3D, SERGEF, SERPINA1, SERPINA2, SHC1, SLAMF6, SLC24A5, SLC31A1, SLC34A2, SLC35B1, SLC43A3, SLC4A1, SLC4A4, SNCA, STK32A, THRSP, TIMP1, TIMP2, TMSB10, TNFRSF17, TNFRSF1A, TXNDC12, VWA5A, WAS, WIPI1, and ZFYVE16. The thyroid tissue sample may be a human thyroid tissue sample. A thyroid tissue sample may be obtained by needle aspiration, fine needle aspiration, core needle biopsy, vacuum assisted biopsy, large core biopsy, incisional biopsy, excisional biopsy, punch biopsy, shave biopsy, or skin biopsy. Gene expression products for use in the methods of the invention include, but are not limited to RNA, such as mRNA, rRNA, tRNA, or miRNA. In some embodiments, RNA expression level is measured by microarray, SAGE, blotting, RT-PCR, sequencing, or quantitative PCR.

## BRIEF DESCRIPTION OF THE DRAWINGS

The novel features of the invention are set forth with particularity in the appended claims. A better understanding of the features and advantages of the present disclosure will be obtained by reference to the following detailed description that sets forth illustrative embodiments, in which the principles of the disclosure are utilized, and the accompanying drawings of which:

Figure 1A and 1B are flow charts depicting embodiments of the disclosure.
Figure 1C depicts one embodiment of an architecture of a system for conducting the methods of the disclosure.
Figure 2 is a table that lists 16 biomarker panels that can be used to diagnose a thyroid condition.
Figure 3 is a table that lists 7 classification panels that can be used to diagnose a thyroid condition. Classifier 7 is at times herein referred to as "main classifier."

Figure 4 is a single table that lists biomarkers that can be assigned to the indicated classification panel. Subparts A-H are arbitrary divisions of the table and do not necessarily represent individual sets of biomarkers.

Figure 5 is a single table providing a model of a gene expression matrix that differentiates between malignant and benign thyroid fine needle aspirates (FNA) using a hypothetical panel of 20 biomarkers. Subparts A-B are arbitrary divisions of the table.

Figure 6 is a single table providing a model of a gene expression matrix that differentiates between malignant and benign thyroid FNA samples using a panel of 20 biomarkers. This figure has the identical biomarker signature to that displayed in Figure 5, except that the individual biomarkers are different. Subparts A-B are arbitrary divisions of the table.

Figure 7 is a single table providing a model of a gene expression matrix that differentiates between malignant and benign thyroid FNA samples using a panel of 20 biomarkers. This table uses genetic markers that differ from those in Figures 5 and 6 and that also provide a different biomarker signature from that in Figures 5 and 6. Subparts A-B are arbitrary divisions of the table.

Figure 8 is a table providing an example list of biomarkers useful in the methods of the present invention, especially for identifying the presence of Hurthle cell adenoma and/or Hurthle cell carcinoma in a thyroid tissue sample.

Figure 9 illustrates Receiver Operator Characteristic (ROC) curves for classifiers trained according to the methods of the disclosure.

Figures 10A and 10B illustrate comparisons of molecular classifiers trained according to the methods of the disclosure, including measures of sensitivity and specificity with regard to performance on two independent test sets.

Figures 10C and 10D show subtype distribution of the two independent data sets and classifier prediction for each sample.

Figure 11 is a table showing the composition of samples used in algorithm training and testing, by subtype, as defined by expert post-surgical histopathology review.

Figure 12A shows a comparison of composite follicular (FOL) and lymphocytic (LCT) scores across surgical tissue.

Figure 12B shows a comparison of composite follicular (FOL) and lymphocytic (LCT) scores across fine needle aspirates.

Figure 13 illustrate the effect of *in silico* simulated mixtures and *in vitro* mixtures on classifier performance.

Figure 14 is a table showing the results of over-representation analysis of top differentially expressed genes.

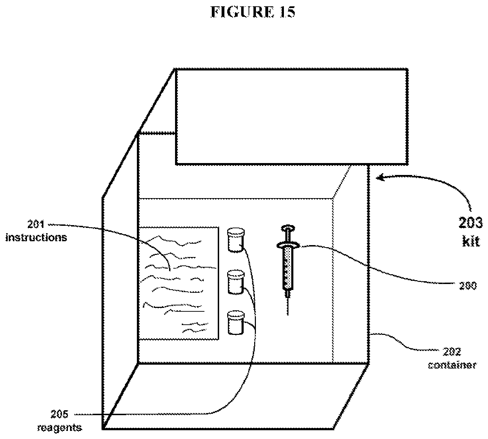

Figure 15 is an embodiment of a kit of the present disclosure.

Figure 16 depicts a computer useful for displaying, storing, retrieving, or calculating diagnostic results from the methods of the disclosure; displaying, storing, retrieving, or calculating raw data from genomic or nucleic acid expression analysis; or displaying, storing, retrieving, or calculating any sample or customer information useful in the methods of the present disclosure

## DETAILED DESCRIPTION OF THE INVENTION

### I. Introduction

The present disclosure provides novel methods for identifying abnormal cellular proliferation in a biological test sample, and related kits and compositions. Methods of differentiating benign from suspicious (or malignant) tissue are provided, as well as methods of identifying definitive benign tissue, and related kits, compositions and business methods. Sets of biomarkers useful for identifying benign or suspicious tissue are provided, as well as methods of obtaining such sets of biomarkers. For example, this disclosure provides novel classification panels that can be obtained from gene expression analysis of sample cohorts exhibiting different pathologies. This disclosure also provides methods of reclassifying an indeterminate biological sample (e.g., surgical tissue, thyroid tissue, thyroid FNA sample, etc.) into a benign versus suspicious (or malignant) category, and related compositions, business methods and kits. In some cases, this disclosure provides a "main classifier" obtained from expression analysis using panels of biomarkers, and that can be used to designate a sample as benign or suspicious (or malignant). This disclosure also provides a series of steps that may precede applying a main classifier to expression level data from a biological sample, such as a clinical sample. Such series of steps may include an initial cytology or histopathology study of the biological sample, followed by analysis of gene (or other biomarker) expression levels in the sample. In some embodiments, the cytology or histopathology study occurs concurrently or after the step of applying any of the classifiers described herein.

Expression levels for a sample may be compared to gene expression data for two or more different sets of biomarkers, the gene expression data for each set of biomarkers comprising one or more reference gene expression levels correlated with the presence of one or more tissue types, wherein the expression level is compared to gene expression data for the two or more biomarkers in sequential fashion. Comparison of expression levels to gene expression data for sets of biomarkers may comprise the application of a classifier. For example, analysis of the gene expression levels may involve sequential application of different classifiers described herein to the gene expression data. Such sequential analysis may involve applying a classifier obtained from gene expression analysis of cohorts of diseased tissue, followed by applying a classifier obtained from analysis of a mixture of different biological samples, some of such samples containing diseased tissues and others containing benign tissue. In preferred embodiments, the diseased tissue is malignant or cancerous tissue (including tissue that has metastasized from another organ). In more preferred embodiments, the diseased tissue is thyroid cancer or a non-thyroid cancer that has metastasized to the thyroid. In some embodiments, the classifier is obtained from gene expression analysis of samples hosting foreign tissue (e.g, a thyroid tissue sample containing parathyroid tissue).

Classifiers used early in the sequential analysis may be used to either rule-in or rule-out a sample as benign or suspicious. In some embodiments, such sequential analysis ends with the application of a "main" classifier to data from samples that have not been ruled out by the preceding classifiers, wherein the main classifier is obtained from data analysis of gene expression levels in multiple types of tissue and wherein the main classifier is capable of designating the sample as benign or suspicious (or malignant).

One example of a condition that can be identified or characterized using the subject methods is thyroid cancer. The thyroid has at least two kinds of cells that make hormones. Follicular cells make thyroid hormone, which affects heart rate, body temperature, and energy level. C cells make calcitonin, a hormone that helps control the level of calcium in the blood. Abnormal growth in the thyroid can result in the formation of nodules, which can be either benign or suspicious (or malignant). Thyroid cancer includes at least four different kinds of malignant tumors of the thyroid gland: papillary, follicular, medullary and anaplastic.

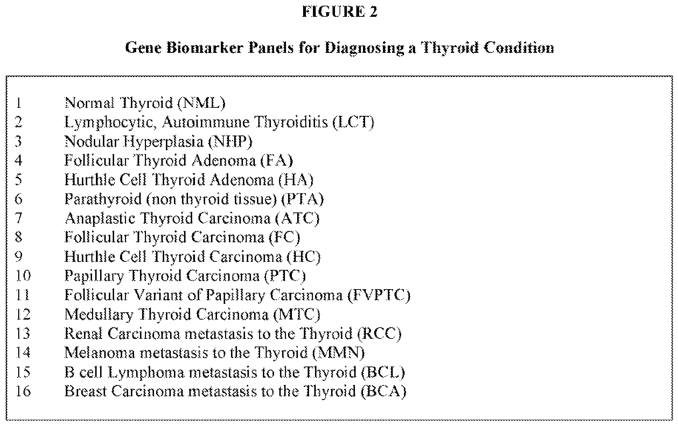

Expression profiling using panels of biomarkers can be used to characterize thyroid tissue as benign, suspicious, and/or malignant. Panels may be derived from analysis of gene expression levels of cohorts containing benign (non-cancerous) thyroid subtypes including follicular adenoma (FA), nodular hyperplasia (NHP), lymphocytic thyroiditis (LCT), and Hurthle cell adenoma (HA); malignant subtypes including follicular carcinoma (FC), papillary thyroid carcinoma (PTC), follicular variant of papillary carcinoma (FVPTC), medullary thyroid carcinoma (MTC), Hürthle cell carcinoma (HC), and anaplastic thyroid carcinoma (ATC). Such panels may also be derived from non-thyroid subtypes including renal carcinoma (RCC), breast carcinoma (BCA), melanoma (MMN), B cell lymphoma (BCL), and parathyroid (PTA). Biomarker panels associated with normal thyroid tissue (NML) may also be used in the methods and compositions provided herein. Exemplary panels of biomarkers are provided in Figure 2, and will be described further herein. Of note, each panel listed in Figure 2, relates to a signature, or pattern of biomarker expression (e.g., gene expression), that correlates with samples of that particular pathology or description.

The present disclosure also provides novel methods and compositions for identification of types of aberrant cellular proliferation through an iterative process (e.g. differential diagnosis) such as carcinomas including follicular carcinomas (FC), follicular variant of papillary thyroid carcinomas (FVPTC), Hurthle cell carcinomas (HC), Hurthle cell adenomas (HA); papillary thyroid carcinomas (PTC), medullary thyroid carcinomas (MTC), and anaplastic carcinomas (ATC); adenomas including follicular adenomas (FA); nodule hyperplasias (NHP); colloid nodules (CN); benign nodules (BN); follicular neoplasms (FN); lymphocytic thyroiditis (LCT), including lymphocytic autoimmune thyroiditis; parathyroid tissue; renal carcinoma metastasis to the thyroid; melanoma metastasis to the thyroid; B-cell lymphoma metastasis to the thyroid; breast carcinoma to the thyroid; benign (B) tumors, malignant (M) tumors, and normal (N) tissues. The present disclosure further provides novel gene expression markers and novel groups of genes and markers useful for the characterization, diagnosis, and/or treatment of cellular proliferation. Additionally the present disclosure provides business methods for providing enhanced diagnosis, differential diagnosis, monitoring, and treatment of cellular proliferation.

The present disclosure provides lists of specific biomarkers useful for classifying thyroid tissue. However, the present disclosure is not meant to be limited solely to the specific biomarkers disclosed herein. Rather, it is understood that any biomarker, gene, group of genes or group of biomarkers identified through methods described herein is encompassed by the present disclosure.

In some cases, the method provides a number, or a range of numbers, of biomarkers (including gene expression products) that are used to diagnose or otherwise characterize a biological sample. For example, in some embodiments, at least 1, 2, 3, 4, 5, 6, 7, 8, 9, 10, 15, 20, 25, 30, 33, 35, 38, 40, 43, 45, 48, 50, 53, 58, 63, 65, 68, 100, 120, 140, 142, 145, 147, 150, 152, 157, 160, 162, 167, 175, 180, 185, 190, 195, 200, or 300 total biomarkers are used. In other embodiments, at most 1, 2, 3, 4, 5, 6, 7, 8, 9, 10, 15, 20, 25, 30, 33, 35, 38, 40, 43, 45, 48, 50, 53, 58, 63, 65, 68, 100, 120, 140, 142, 145, 147, 150, 152, 157, 160, 162, 167, 175, 180, 185, 190, 195, 200, or 300 total biomarkers are used.

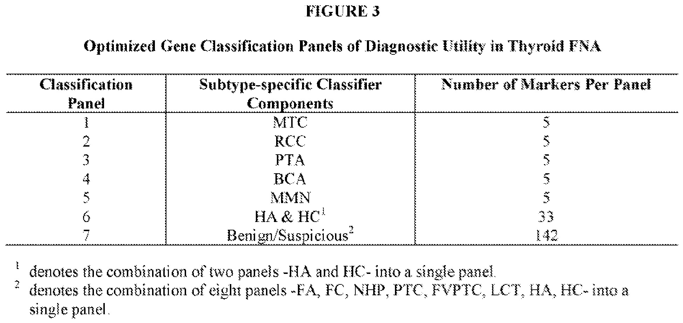

The present methods and compositions also relate to the use of "biomarker panels" for purposes of identification, classification, diagnosis, or to otherwise characterize a biological sample. The methods and compositions may also use groups of biomarker panels, herein described as "classification panels," examples of which can be found in Figure 3. Often the pattern of levels of gene expression of biomarkers in a panel (also known as a signature) is determined and then used to evaluate the signature of the same panel of biomarkers in a biological sample, such as by a measure of similarity between the sample signature and the reference signature. In some embodiments, the method involves measuring (or obtaining) the levels of two or more gene expression products that are within a biomarker panel and/or within a classification panel. For example, in some embodiments, a biomarker panel or a classification panel may contain at least 1, 2, 3, 4, 5, 6, 7, 8, 9, 10, 15, 20, 25, 30, 33, 35, 38, 40, 43, 45, 48, 50, 53, 58, 63, 65, 68, 100, 120, 140, 142, 145, 147, 150, 152, 157, 160, 162, 167, 175, 180, 185, 190, 195, 200, or 300 biomarkers. In some embodiments, a biomarker panel or a classification panel contains no more than 1, 2, 3, 4, 5, 6, 7, 8, 9, 10, 15, 20, 25, 30, 33, 35, 38, 40, 43, 45, 48, 50, 53, 58, 63, 65, 68, 100, 120, 140, 142, 145, 147, 150, 152, 157, 160, 162, 167, 175, 180, 185, 190, 195, 200, or 300 biomarkers. In some embodiments, a classification panel contains at least 1, 2, 3, 4, 5, 6, 7, 8, 9, 10, 15, 20, or 25 different biomarker panels. In other embodiments, a classification panel contains no more than 1, 2, 3, 4, 5, 6, 7, 8, 9, 10, 15, 20, 25 different biomarker panels.

In some embodiments, the present disclosure provides a method of identifying, classifying, or diagnosing cancer comprising the steps of: obtaining an expression level for one or more gene expression products of a biological sample; and identifying the biological sample as benign wherein the gene expression level indicates a lack of cancer in the biological sample. In some embodiments, the present disclosure provides a method of identifying, classifying, or diagnosing cancer comprising the steps of: obtaining an expression level for one or more gene expression products of a biological sample; and identifying the biological sample as malignant or suspicious wherein the gene expression level is indicative of a cancer in the biological sample. For example, this can be done by correlating the patterns of gene expression levels, as defined in classification panels described herein, with the gene expression level in the sample, in order to identify (or rule out) the presence of thyroid cancer in the biological sample. In some embodiments, the gene expression products are associated with biomarkers selected from Figure 4.

In some embodiments, the present disclosure provides a method of identifying, classifying, or diagnosing cancer that gives a specificity and a sensitivity that each are at least 50%, or 70%, using the subject methods described herein, wherein the gene expression product levels are compared between the biological sample and a biomarker panel, or between the biological sample and a classification panel; and identifying the biological sample as cancerous, suspicious, or benign based on the comparison of gene expression profiles. In some embodiments, the specificity of the present method is at least 50%, 60%, 70%, 75%, 80%, 85%, 86%, 87%, 88%, 89%, 90%, 91%, 92%, 93%, 94%, 95%, 96%, 97%, 98%, or 99%. In some embodiments, the sensitivity of the present method is at least 50%, 60%, 70%, 75%, 80%, 85%, 86%, 87%, 88%, 89%, 90%, 91%, 92%, 93%, 94%, 95%, 96%, 97%, 98%, or 99%. In some embodiments, the specificity is at least 50% and the sensitivity of the present method is at least 50%.. In some embodiments, the specificity of the present method is at least 70% and the sensitivity of the present method is at least 70%. In some embodiments, the specificity is at least 50%, and the sensitivity is at least 70%.

In some embodiments, the nominal specificity is greater than or equal to 50%. In some embodiments, the nominal specificity is greater than or equal to 70%. In some embodiments, the nominal negative predictive value (NPV) is greater than or equal to 95%. In some embodiments, the NPV is at least 90%, 91%, 92%, 93%, 94%, 95%, 95.5%, 96%, 96.5%, 97%, 97.5%, 98%, 98.5%, 99%, 99.5% (e.g., 90%, 91%, 92%, 93%, 94%, 95%, 95.5%, 96%, 96.5%, 97%, 97.5%, 98%, 98.5%, 99%, 99.5%, or 100%) and the specificity (or positive predictive value (PPV)) is at least 30%, 35%, 40%, 50%, 60%, 70%, 80%, 90%, 95%, 95.5%, 96%, 96.5%, 97%, 97.5%, 98%, 98.5%, 99%, or 99.5% (e.g., 30%, 35%, 40%, 50%, 60%, 70%, 80%, 90%, 95%, 95.5%, 96%, 96.5%, 97%, 97.5%, 98%, 98.5%, 99%, 99.5%, or 100%). In some cases the NPV is at least 95%, and the specificity is at least 50%. In some cases the NPV is at least 95% and the specificity is at least 70%.

Marker panels are chosen to accommodate adequate separation of benign from non-benign or suspicious expression profiles. Training of this multi-dimensional classifier, i.e., algorithm, can be performed on numerous biological samples, such as at least 50, 100, 200, 300, 400, 500, 600, 700, 800, 900, 1000, 1500, 2000, 2500, 3000, 3500, or 4000 biological samples (e.g., thyroid samples). The total sample population can consist of samples obtained from FNAs, or the sample population may be a mixture of samples obtained by FNAs and by other methods, e.g., post-surgical tissue. The percent of the total sample population that is obtained by FNA's may be greater than 10, 20, 30, 40, 50, 60, 70, 80, 90, or 95%. In some embodiments, many training/test sets are used to develop the preliminary algorithm. The overall algorithm error rate may be shown as a function of gene number for benign vs. non-benign samples. In some embodiments, other performance metric may be used, such as a performance metric that is a function of gene number for either subtypes or benign vs. malignant (B vs. M). Such performance metric may be obtained using CV, or other method known in the art. All results may be obtained using a support vector machine model which is trained and tested in a cross-validated mode on the samples.

In some embodiments, there is a specific (or range of) difference in gene expression between subtypes or sets of samples being compared to one another. In some examples, the gene expression of some similar subtypes are merged to form a super-class that is then compared to another subtype, or another super-class, or the set of all other subtypes. In some embodiments, the difference in gene expression level is at least 5%, 10%, 15%, 20%, 25%, 30%, 35%, 40%, 45% or 50% or more. In some embodiments, the difference in gene expression level is at least 2, 3, 4, 5, 6, 7, 8, 9, 10 fold or more.

In some embodiments, the biological sample is identified as suspicious (e.g., potentially malignant) with an accuracy of at least 50%, 60%, 70%, 75%, 80%, 85%, 90%, 95%, 99% or more. In some embodiments, the biological sample is identified as benign with an accuracy of greater than 50%, 60%, 70%, 75%, 80%, 85%, 90%, 95%, 99% or more. In some embodiments, the accuracy is calculated using a trained algorithm. In some embodiments, the biological sample is identified as cancerous with a sensitivity of greater than 50% or 70%. In some embodiments, the biological sample is identified as cancerous with a specificity of greater than 50% or 70%. In some embodiments, the biological sample is identified as cancerous with a sensitivity of greater than 50% and a specificity of greater than 70%. In some embodiments, the biological sample is identified as benign with a sensitivity of greater than 50%. In some embodiments, the biological sample is identified as benign with a specificity of greater than 50%. In some embodiments, the biological sample is identified as benign with a sensitivity of greater than 50% and a specificity of greater than 50%. In some embodiments, method uses a panel of biomarkers (e.g., biomarker panel, classification panel, classifier) such that the method has a specificity of greater than 50%, 70%, 75%, 80%, 85%, 86%, 87%, 88%, 89%, 90%, 91%, 92%, 93%, 94%, 95%, 96%, 97%, 98%, 99%, or 99.5%, and a sensitivity of greater than 50%, 70%, 75%, 80%, 85%, 86%, 87%, 88%, 89%, 90%, 91%, 92%, 93%, 94%, 95%, 96%, 97%, 98%, 99%, or 99.5%. In some embodiments, the method uses a panel of biomarkers (e.g., biomarker panel, classification panel, classifier) such that the method has a positive predictive value of at least 95%, 95.5%, 96%, 96.5%, 97%, 97.5%, 98%, 98.5%, 99%, 99.5% or more; and/or a negative predictive value of at least 95%, 95.5%, 96%, 96.5%, 97%, 97.5%, 98%, 98.5%, 99%, 99.5% or more. In some embodiments, the method uses a panel of biomarkers (e.g., biomarker panel, classification panel, classifier) such that the method has a specificity or sensitivity of greater than 50%, 70%, 75%, 80%, 85%, 86%, 87%, 88%, 89%, 90%, 91%, 92%, 93%, 94%, 95%, 96%, 97%, 98%, 99%, or 99.5%, and a positive predictive value or negative predictive value of at least 95%, 95.5%, 96%, 96.5%, 97%, 97.5%, 98%, 98.5%, 99%, 99.5% or more. In some embodiments, the method uses a panel of biomarkers (e.g., biomarker panel, classification panel, classifier) such that the method has a negative predictive value of at least 95%, 95.5%, 96%, 96.5%, 97%, 97.5%, 98%, 98.5%, 99%, 99.5% or more.

The present invention provides gene expression products corresponding to biomarkers selected from Figure 4. The methods and compositions provided herein can include gene expression products corresponding to any or all of the biomarkers selected from Figure 4, as well as any subset thereof, in any combination. For example, the methods may use gene expression products corresponding to at least 1, 2, 3, 4, 5, 6, 7, 8, 9, 10, 15, 20, 25, 30, 35, 40, 45 or 50, 100, 120, 140, 160 of the genetic markers provided in Figure 4. In some cases, certain biomarkers may be excluded or substituted with other biomarkers, for example with biomarkers that exhibit a similar expression level profile with respect to a particular tissue type or sub-type.

In some embodiments, the methods of the present invention seek to improve upon the accuracy of current methods of cancer diagnosis. In some embodiments, the methods provide improved accuracy of identifying benign, or definitively benign, samples (e.g., thyroid samples). Improved accuracy may be obtained by using algorithms trained with specific sample cohorts, high numbers of samples, and/or samples from individuals located in diverse geographical regions. The sample cohort may be from at least 1, 2, 3, 4, 5, 6, 67, 8, 9, 10, 15, 20, 25, 30, 35, 40, 45, 50, 55, 60, 65, 70, 75, or 80 different geographical locations (e.g., sites spread out across a nation, such as the United States, across a continent, or across the world). Geographical locations include, but are not limited to, test centers, medical facilities, medical offices, post office addresses, cities, counties, states, nations, and continents. In some embodiments, a classifier that is trained using sample cohorts from the United States may need to be re-trained for use on sample cohorts from other geographical regions (e.g., India, Asia, Europe, Africa, etc.).

In some embodiments, the present disclosure provides a method of classifying cancer comprising the steps of: obtaining a biological sample comprising gene expression products; determining the expression level for one or more gene expression products of the biological sample that are differentially expressed in different subtypes of a cancer; and identifying the biological sample as cancerous wherein the gene expression level is indicative of a subtype of cancer. In some embodiments, the subject methods distinguish follicular carcinoma from medullary carcinoma. In some embodiments, the subject methods are used to classify a thyroid tissue sample as comprising one or more benign or malignant tissue types (e.g. a cancer subtype), including but not limited to follicular adenoma (FA), nodular hyperplasia (NHP), lymphocytic thyroiditis (LCT), and Hurthle cell adenoma (HA), follicular carcinoma (FC), papillary thyroid carcinoma (PTC), follicular variant of papillary carcinoma (FVPTC), medullary thyroid carcinoma (MTC), Hürthle cell carcinoma (HC), and anaplastic thyroid carcinoma (ATC), renal carcinoma (RCC), breast carcinoma (BCA), melanoma (MMN), B cell lymphoma (BCL), and parathyroid (PTA). In some embodiments, the subject methods are used to classify a sample of thyroid tissue as comprising HC and/or HA tissue types. In some embodiments, the subject methods distinguish a benign thyroid disease from a malignant thyroid tumor/carcinoma.

In some embodiments of the disclosure, the biological sample is classified as cancerous or positive for a subtype of cancer with an accuracy of greater than 75%, 80%, 85%, 86%, 87%, 88%, 89%, 90%, 91%, 92%, 93%, 94%, 95%, 96%, 97%, 98%, 99%, or 99.5%. The classification accuracy as used herein includes specificity, sensitivity, positive predictive value, negative predictive value, and/or false discovery rate.

When a range of values is indicated herein, and the range begins with a modifier such as "greater than," "at least", "more than," etc., the modifier is meant to be included for every value in the range, unless where otherwise indicated. For example, "at least 1, 2, or 3" means "at least 1, at least 2, or at least 3," as used herein.

In some embodiments, gene expression product markers of the present disclosure may provide increased accuracy of a disease or cancer identification or diagnosis through the use of multiple gene expression product markers in low quantity and quality, and statistical analysis using the algorithms of the present invention. In particular, the present disclosure provides, but is not limited to, methods of characterizing, classifying, or diagnosing gene expression profiles associated with thyroid cancers. The present disclosure also provides algorithms for characterizing and classifying thyroid tissue samples, and kits and compositions useful for the application of said methods. The disclosure further includes methods for running a molecular profiling business.

In one embodiment of the disclosure, markers and genes can be identified to have differential expression in thyroid cancer samples compared to thyroid benign samples. Illustrative examples having a benign pathology include follicular adenoma, Hurthle cell adenoma, lymphocytic thyroiditis, and nodular hyperplasia. Illustrative examples having a malignant pathology include follicular carcinoma, follicular variant of papillary thyroid carcinoma, medullary carcinoma, and papillary thyroid carcinoma.

Biological samples may be treated to extract nucleic acid such as DNA or RNA. The nucleic acid may be contacted with an array of probes of the present disclosure under conditions to allow hybridization, or the nucleic acids may be sequenced by any method known in the art. The degree of hybridization may be assayed in a quantitative matter using a number of methods known in the art. In some cases, the degree of hybridization at a probe position may be related to the intensity of signal provided by the assay, which therefore is related to the amount of complementary nucleic acid sequence present in the sample. Software can be used to extract, normalize, summarize, and analyze array intensity data from probes across the human genome or transcriptome including expressed genes, exons, introns, and miRNAs. In some embodiments, the intensity of a given probe in either the benign or malignant samples can be compared against a reference set to determine whether differential expression is occuring in a sample. An increase or decrease in relative intensity at a marker position on an array corresponding to an expressed sequence is indicative of an increase or decrease respectively of expression of the corresponding expressed sequence. Alternatively, a decrease in relative intensity may be indicative of a mutation in the expressed sequence.

The resulting intensity values for each sample can be analyzed using feature selection techniques including filter techniques which assess the relevance of features by looking at the intrinsic properties of the data, wrapper methods which embed the model hypothesis within a feature subset search, and embedded techniques in which the search for an optimal set of features is built into a classifier algorithm.

Filter techniques useful in the methods of the present invention include (1) parametric methods such as the use of two sample t-tests, ANOVA analyses, Bayesian frameworks, and Gamma distribution models (2) model free methods such as the use of Wilcoxon rank sum tests, between-within class sum of squares tests, rank products methods, random permutation methods, or TNoM which involves setting a threshold point for fold-change differences in expression between two datasets and then detecting the threshold point in each gene that minimizes the number of misclassifications (3) and multivariate methods such as bivariate methods, correlation based feature selection methods (CFS), minimum redundancy maximum relevance methods (MRMR), Markov blanket filter methods, and uncorrelated shrunken centroid methods. Wrapper methods useful in the methods of the present invention include sequential search methods, genetic algorithms, and estimation of distribution algorithms. Embedded methods useful in the methods of the present invention include random forest algorithms, weight vector of support vector machine algorithms, and weights of logistic regression algorithms. Bioinformatics. 2007 Oct 1;23(19):2507-17 provides an overview of the relative merits of the filter techniques provided above for the analysis of intensity data.

Selected features may then be classified using a classifier algorithm. Illustrative algorithms include but are not limited to methods that reduce the number of variables such as principal component analysis algorithms, partial least squares methods, and independent component analysis algorithms. Illustrative algorithms further include but are not limited to methods that handle large numbers of variables directly such as statistical methods and methods based on machine learning techniques. Statistical methods include penalized logistic regression, prediction analysis of microarrays (PAM), methods based on shrunken centroids, support vector machine analysis, and regularized linear discriminant analysis. Machine learning techniques include bagging procedures, boosting procedures, random forest algorithms, and combinations thereof. Cancer Inform. 2008; 6: 77-97 provides an overview of the classification techniques provided above for the analysis of microarray intensity data.

The markers and genes of the present disclosure can be utilized to characterize the cancerous or non-cancerous status of cells or tissues. The present invention includes a method for distinguishing between benign tissues or cells and malignant tissues or cells comprising determining the differential expression of one or more markers or genes in a thyroid sample of a subject wherein said markers or genes are listed in Figure 4. The present invention also includes methods for identifying thyroid pathology subtypes comprising determining the differential expression of one or more markers or genes in a thyroid sample of a subject wherein said markers or genes are listed in Figure 4 along with the corresponding sub-type, as indicated in Figure 4.

In accordance with the foregoing, the differential expression of a gene, genes, markers, mRNA, miRNAs, or a combination thereof as disclosed herein may be determined using northern blotting and employing the sequences as identified herein to develop probes for this purpose. Such probes may be composed of DNA or RNA or synthetic nucleotides or a combination of these and may advantageously be comprised of a contiguous stretch of nucleotide residues matching, or complementary to, a sequence corresponding to a genetic marker identified in Figure 4. Such probes will most usefully comprise a contiguous stretch of at least 15-200 residues or more including 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 80, 85, 90, 95, 100, 110, 120, 130, 140, 150, 160, 175, or 200 nucleotides or more, derived from one or more of the sequences corresponding to a genetic marker identified in Figure 4. Thus, where a single probe binds multiple times to the transcriptome of a sample of cells that are cancerous, or are suspected of being cancerous, or predisposed to become cancerous, whereas binding of the same probe to a similar amount of transcriptome derived from the genome of otherwise non-cancerous cells of the same organ or tissue results in observably more or less binding, this is indicative of differential expression of a gene, multiple genes, markers, or miRNAs comprising, or corresponding to, the sequences corresponding to a genetic marker identified in Figure 4 from which the probe sequenced was derived.

In one such embodiment, the elevated expression, as compared to normal cells and/or tissues of the same organ, is determined by measuring the relative rates of transcription of RNA, such as by production of corresponding cDNAs and then analyzing the resulting DNA using probes developed from the gene sequences as corresponding to a genetic marker identified in Figure 4. Thus, the levels of cDNA produced by use of reverse transcriptase with the full RNA complement of a cell suspected of being cancerous produces a corresponding amount of cDNA that can then be amplified using polymerase chain reaction, or some other means, such as linear amplification, isothermal amplification, NASB, or rolling circle amplification, to determine the relative levels of resulting cDNA and, thereby, the relative levels of gene expression.

Increased expression may also be determined using agents that selectively bind to, and thereby detect, the presence of expression products of the genes disclosed herein. For example, an antibody, possibly a suitably labeled antibody, such as where the antibody is bound to a fluorescent label or radiolabel, may be generated against one of the polypeptides that is a gene product of one of the gene sequences corresponding to a genetic marker identified in Figure 4, and said antibody will then react with, binding either selectively or specifically, to a polypeptide encoded by one of the genes that corresponds to a sequence disclosed herein. Such antibody binding, especially the relative extent of such binding in samples derived from suspected cancerous, as opposed to otherwise non-cancerous, cells and tissues, can then be used as a measure of the extent of expression, or differential expression, of the cancer-related genes identified herein. Thus, the genes identified herein as being differentially expressed in cancerous cells and tissues may be differentially expressed due to increased copy number, decreased copy number, or due to over- or under-transcription, such as where the over-expression is due to over- or under-production of a transcription factor that activates or represses the gene and leads to repeated binding of RNA polymerase, thereby generating large than normal amounts of RNA transcripts, which are subsequently translated into polypeptides, such as the polypeptides comprising amino acid sequences corresponding to gene products (e.g., polypeptides) of a sequence corresponding to a genetic marker identified in Figure 4. Such analysis provides an additional means of ascertaining the expression of the genes identified according to the disclosure and thereby determining the presence of a cancerous state in a sample derived from a patient to be tested, or the predisposition to develop cancer at a subsequent time in said patient.

In employing the methods of the invention, the gene or marker expression indicative of a cancerous state need not be characteristic of every cell found to be cancerous. Thus, the methods disclosed herein are useful for detecting the presence of a cancerous condition within a tissue where less than all cells exhibit the complete pattern of differential expression. For example, a set of selected genes or markers, comprising sequences homologous under stringent conditions, or at least 90%, preferably 95%, identical to at least one of the sequences corresponding to a genetic marker identified in Figure 4, or probe sequences complementary to all or a portion thereof, may be found, using appropriate probes (e.g. DNA or RNA probes) to be present in about, less than about, or more than about 20%, 30%, 40%, 50%, 60%, 70%, 80%, 90%, or more of cells derived from a sample of tumorous or malignant tissue. In some embodiments, a set of selected genes or markers correlated with a cancerous condition, and forming an expression pattern, may be absent from about, less than about, or more than about 20%, 30%, 40%, 50%, 60%, 70%, 80%, 90%, or more cells derived from corresponding non-cancerous, or otherwise normal, tissue. In one embodiment, an expression pattern of a cancerous condition is detected in at least 70% of cells drawn from a cancerous tissue and absent from at least 70% of a corresponding normal, non-cancerous, tissue sample. In some embodiments, such expression pattern is found to be present in at least 80% of cells drawn from a cancerous tissue and absent from at least 80% of a corresponding normal, non-cancerous, tissue sample. In some embodiments, such expression pattern is found to be present in at least 90% of cells drawn from a cancerous tissue and absent from at least 90% of a corresponding normal, non-cancerous, tissue sample. In some embodiments, such expression pattern is found to be present in at least 100% of cells drawn from a cancerous tissue and absent from at least 100% of a corresponding normal, non-cancerous, tissue sample, although the latter embodiment may represent a rare occurrence. It should also be noted that the expression pattern may be either completely present, partially present, or absent within affected cells, as well as unaffected cells. Therefore, in some embodiments, the expression pattern is present in variable amounts within affected cells; in some embodiments, the expression pattern is present in variable amounts within unaffected cells.

In some embodiments molecular profiling includes detection, analysis, or quantification of nucleic acid (DNA, or RNA), protein, or a combination thereof. The diseases or conditions to be diagnosed by the methods of the present disclosure include for example conditions of abnormal growth in one or more tissues of a subject including but not limited to skin, heart, lung, kidney, breast, pancreas, liver, muscle, smooth muscle, bladder, gall bladder, colon, intestine, brain, esophagus, or prostate. The tissues analyzed by the methods of the present invention include thyroid tissues.

## II. Obtaining a Biological Sample

In some embodiments, the methods of the present disclosure provide for obtaining a sample from a subject. As used herein, the term subject refers to any animal (e.g. a mammal), including but not limited to humans, non-human primates, rodents, dogs, cats, pigs, fish, and the like. In preferred embodiments, the present methods and compositions apply to biological samples from humans. In some embodiments, the human is a child, an adolescent, or an adult. In some cases, the human is more than 1, 2, 5, 10, 20, 30, 40, 50, 60, 65, 70, 75, or 80 years of age.

The methods of obtaining provided herein include methods of biopsy including fine needle aspiration, core needle biopsy, vacuum assisted biopsy, incisional biopsy, excisional biopsy, punch biopsy, shave biopsy or skin biopsy. In some cases, the classifiers provided herein are applied to data only from biological samples obtained by FNA. In some cases, the classifiers provided herein are applied to data only from biological samples obtained by FNA or surgical biopsy. In some cases, the classifiers provided herein are applied to data only from biological samples obtained by surgical biopsy. In some cases, the classifiers themselves are obtained from analysis of data from samples obtained by a specific procedure. For example, a cohort of samples, wherein some were obtained by FNA, and others were obtained by surgical biopsy, may be the source of the samples that are analyzed for the classifiers used herein. In other cases, only data from samples obtained by FNA are used to obtain the classifiers herein. In other cases, only data from samples obtained by surgical procedures are used to obtain the classifiers herein.

The sample may be obtained from any of the tissues provided herein including but not limited to skin, heart, lung, kidney, breast, pancreas, liver, muscle, smooth muscle, bladder, gall bladder, colon, intestine, brain, prostate, esophagus, or thyroid. Alternatively, the sample may be obtained from any other source including but not limited to blood, sweat, hair follicle, buccal tissue, tears, menses, feces, or saliva. In some embodiments of the present disclosure, a medical professional may obtain a biological sample for testing. In some cases the medical professional may refer the subject to a testing center or laboratory for submission of the biological sample. In other cases, the subject may provide the sample. In some cases, a molecular profiling business of the present disclosure may obtain the sample. In some cases, a molecular profiling business obtains data regarding the biological sample, such as biomarker expression level data, or analysis of such data.

The sample may be obtained by methods known in the art such as the biopsy methods provided herein, swabbing, scraping, phlebotomy, or any other methods known in the art. In some cases, the sample may be obtained, stored, or transported using components of a kit of the present disclosure. In some cases, multiple samples, such as multiple thyroid samples may be obtained for diagnosis by the methods of the present disclosure. In some cases, multiple samples, such as one or more samples from one tissue type (e.g. thyroid) and one or more samples from another tissue (e.g. buccal) may be obtained for diagnosis by the methods of the present disclosure. In some cases, multiple samples such as one or more samples from one tissue type (e.g. thyroid) and one or more samples from another tissue (e.g. buccal) may be obtained at the same or different times. In some cases, the samples obtained at different times are stored and/or analyzed by different methods. For example, a sample may be obtained and analyzed by cytological analysis (routine staining). In some cases, a further sample may be obtained from a subject based on the results of a cytological analysis. The diagnosis of cancer may include an examination of a subject by a physician, nurse or other medical professional. The examination may be part of a routine examination, or the examination may be due to a specific complaint including but not limited to one of the following: pain, illness, anticipation of illness, presence of a suspicious lump or mass, a disease, or a condition. The subject may or may not be aware of the disease or condition. The medical professional may obtain a biological sample for testing. In some cases the medical professional may refer the subject to a testing center or laboratory for submission of the biological sample.

In some cases, the subject may be referred to a specialist such as an oncologist, surgeon, or endocrinologist for further diagnosis. The specialist may likewise obtain a biological sample for testing or refer the individual to a testing center or laboratory for submission of the biological sample. In any case, the biological sample may be obtained by a physician, nurse, or other medical professional such as a medical technician, endocrinologist, cytologist, phlebotomist, radiologist, or a pulmonologist. The medical professional may indicate the appropriate test or assay to perform on the sample, or the molecular profiling business of the present disclosure may consult on which assays or tests are most appropriately indicated. The molecular profiling business may bill the individual or medical or insurance provider thereof for consulting work, for sample acquisition and or storage, for materials, or for all products and services rendered.

In some embodiments of the present disclosure, a medical professional need not be involved in the initial diagnosis or sample acquisition. An individual may alternatively obtain a sample through the use of an over the counter kit. Said kit may contain a means for obtaining said sample as described herein, a means for storing said sample for inspection, and instructions for proper use of the kit. In some cases, molecular profiling services are included in the price for purchase of the kit. In other cases, the molecular profiling services are billed separately.

A sample suitable for use by the molecular profiling business may be any material containing tissues, cells, nucleic acids, genes, gene fragments, expression products, gene expression products, or gene expression product fragments of an individual to be tested. Methods for determining sample suitability and/or adequacy are provided. A sample may include but is not limited to, tissue, cells, or biological material from cells or derived from cells of an individual. The sample may be a heterogeneous or homogeneous population of cells or tissues. The biological sample may be obtained using any method known to the art that can provide a sample suitable for the analytical methods described herein.

The sample may be obtained by non-invasive methods including but not limited to: scraping of the skin or cervix, swabbing of the cheek, saliva collection, urine collection, feces collection, collection of menses, tears, or semen. In other cases, the sample is obtained by an invasive procedure including but not limited to: biopsy, alveolar or pulmonary lavage, needle aspiration, or phlebotomy. The method of biopsy may further include incisional biopsy, excisional biopsy, punch biopsy, shave biopsy, or skin biopsy. The method of needle aspiration may further include fine needle aspiration, core needle biopsy, vacuum assisted biopsy, or large core biopsy. In some embodiments, multiple samples may be obtained by the methods herein to ensure a sufficient amount of biological material. Methods of obtaining suitable samples of thyroid are known in the art and are further described in the ATA Guidelines for thyroid nodule management (Cooper et al. Thyroid Vol. 16 No. 2 2006). Generic methods for obtaining biological samples are also known in the art and further described in for example Ramzy, Ibrahim Clinical Cytopathology and Aspiration Biopsy 2001. In one embodiment, the sample is a fine needle aspirate of a thyroid nodule or a suspected thyroid tumor. In some cases, the fine needle aspirate sampling procedure may be guided by the use of an ultrasound, X-ray, or other imaging device.

In some embodiments of the present disclosure, the molecular profiling business may obtain the biological sample from a subject directly, from a medical professional, from a third party, or from a kit provided by the molecular profiling business or a third party. In some cases, the biological sample may be obtained by the molecular profiling business after the subject, a medical professional, or a third party acquires and sends the biological sample to the molecular profiling business. In some cases, the molecular profiling business may provide suitable containers, and excipients for storage and transport of the biological sample to the molecular profiling business.

### III. Storing the sample

In some embodiments, the methods of the present disclosure provide for storing the sample for a time such as seconds, minutes, hours, days, weeks, months, years or longer after the sample is obtained and before the sample is analyzed by one or more methods of the disclosure. In some cases, the sample obtained from a subject is subdivided prior to the step of storage or further analysis such that different portions of the sample are subject to different downstream methods or processes including but not limited to storage, cytological analysis, adequacy tests, nucleic acid extraction, molecular profiling or a combination thereof.

In some cases, a portion of the sample may be stored while another portion of said sample is further manipulated. Such manipulations may include but are not limited to molecular profiling; cytological staining; nucleic acid (RNA or DNA) extraction, detection, or quantification; gene expression product (RNA or Protein) extraction, detection, or quantification; fixation (e.g. formalin fixed paraffin embedded samples); and examination. The sample may be fixed prior to or during storage by any method known to the art such as using glutaraldehyde, formaldehyde, or methanol. In other cases, the sample is obtained and stored and subdivided after the step of storage for further analysis such that different portions of the sample are subject to different downstream methods or processes including but not limited to storage, cytological analysis, adequacy tests, nucleic acid extraction, molecular profiling or a combination thereof. In some cases, samples are obtained and analyzed by for example cytological analysis, and the resulting sample material is further analyzed by one or more molecular profiling methods of the present invention. In such cases, the samples may be stored between the steps of cytological analysis and the steps of molecular profiling. Samples may be stored upon acquisition to facilitate transport, or to wait for the results of other analyses. In another embodiment, samples may be stored while awaiting instructions from a physician or other medical professional.

The acquired sample may be placed in a suitable medium, excipient, solution, or container for short term or long term storage. Said storage may require keeping the sample in a refrigerated, or frozen environment. The sample may be quickly frozen prior to storage in a frozen environment. The frozen sample may be contacted with a suitable cryopreservation medium or compound including but not limited to: glycerol, ethylene glycol, sucrose, or glucose. A suitable medium, excipient, or solution may include but is not limited to: hanks salt solution, saline, cellular growth medium, an ammonium salt solution such as ammonium sulphate or ammonium phosphate, or water. Suitable concentrations of ammonium salts include solutions of about 0.1g/ml, 0.2g/ml, 0.3g/ml, 0.4g/ml, 0.5g/ml, 0.6 g/ml, 0.7g/ml, 0.8 g/ml, 0.9g/ml, 1.0 g/ml, 1.1 g/ml, 1.2 g/ml, 1.3g/ml, 1.4g/ml, 1.5g/ml, 1.6 g/ml, 1.7 g/ml, 1.8 g/ml, 1.9 g/ml, 2.0 g/ml, 2.2 g/ml, 2.3g/ml, 2.5 g/ml or higher. The medium, excipient, or solution may or may not be sterile.

The sample may be stored at room temperature or at reduced temperatures such as cold temperatures (e.g. between about 20°C and about 0°C), or freezing temperatures, including for example 0°C, -1°C, -2°C, -3°C, -4°C, -5°C, -6°C, -7°C, -8°C, -9°C, -10°C, -12°C, -14°C, -15°C, -16°C, -20°C, - 22°C, -25°C, -28°C, -30°C, -35°C, -40°C, -45°C, -50°C, -60°C, -70°C, -80°C, -100°C, -120°C, -140°C, -180°C, -190°C, or about -200°C. In some cases, the samples may be stored in a refrigerator, on ice or a frozen gel pack, in a freezer, in a cryogenic freezer, on dry ice, in liquid nitrogen, or in a vapor phase equilibrated with liquid nitrogen.

The medium, excipient, or solution may contain preservative agents to maintain the sample in an adequate state for subsequent diagnostics or manipulation, or to prevent coagulation. Said preservatives may include citrate, ethylene diamine tetraacetic acid, sodium azide, or thimersol. The medium, excipient or solution may contain suitable buffers or salts such as Tris buffers or phosphate buffers, sodium salts (e.g. NaCl), calcium salts, magnesium salts, and the like. In some cases, the sample may be stored in a commercial preparation suitable for storage of cells for subsequent cytological analysis such as but not limited to Cytyc ThinPrep, SurePath, or Monoprep.

The sample container may be any container suitable for storage and or transport of the biological sample including but not limited to: a cup, a cup with a lid, a tube, a sterile tube, a vacuum tube, a syringe, a bottle, a microscope slide, or any other suitable container. The container may or may not be sterile.

## IV. Transportation of the Sample

The methods of the present disclosure provide for transport of the sample. In some cases, the sample is transported from a clinic, hospital, doctor's office, or other location to a second location whereupon the sample may be stored and/or analyzed by for example, cytological analysis or molecular profiling. In some cases, the sample may be transported to a molecular profiling company in order to perform the analyses described herein. In other cases, the sample may be transported to a laboratory such as a laboratory authorized or otherwise capable of performing the methods of the present disclosure such as a Clinical Laboratory Improvement Amendments (CLIA) laboratory. The sample may be transported by the individual from whom the sample derives. Said transportation by the individual may include the individual appearing at a molecular profiling business or a designated sample receiving point and providing a sample. Said providing of the sample may involve any of the techniques of sample acquisition described herein, or the sample may have already have been acquired and stored in a suitable container as described herein. In other cases the sample may be transported to a molecular profiling business using a courier service, the postal service, a shipping service, or any method capable of transporting the sample in a suitable manner. In some cases, the sample may be provided to a molecular profiling business by a third party testing laboratory (e.g. a cytology lab). In other cases, the sample may be provided to a molecular profiling business by the subject's primary care physician, endocrinologist or other medical professional. The cost of transport may be billed to the individual, medical provider, or insurance provider. The molecular profiling business may begin analysis of the sample immediately upon receipt, or may store the sample in any manner described herein. The method of storage may or may not be the same as chosen prior to receipt of the sample by the molecular profiling business.

The sample may be transported in any medium or excipient including any medium or excipient provided herein suitable for storing the sample such as a cryopreservation medium or a liquid based cytology preparation. In some cases, the sample may be transported frozen or refrigerated such as at any of the suitable sample storage temperatures provided herein.

Upon receipt of the sample by the molecular profiling business, a representative or licensee thereof, a medical professional, researcher, or a third party laboratory or testing center (e.g. a cytology laboratory) the sample may be assayed using a variety of routine analyses known to the art such as cytological assays, and genomic analysis. Such tests may be indicative of cancer, the type of cancer, any other disease or condition, the presence of disease markers, or the absence of cancer, diseases, conditions, or disease markers. The tests may take the form of cytological examination including microscopic examination as described below. The tests may involve the use of one or more cytological stains. The biological material may be manipulated or prepared for the test prior to administration of the test by any suitable method known to the art for biological sample preparation. The specific assay performed may be determined by the molecular profiling company, the physician who ordered the test, or a third party such as a consulting medical professional, cytology laboratory, the subject from whom the sample derives, or an insurance provider. The specific assay may be chosen based on the likelihood of obtaining a definite diagnosis, the cost of the assay, the speed of the assay, or the suitability of the assay to the type of material provided.

## V. Test for adequacy

Subsequent to or during sample acquisition, including before or after a step of storing the sample, the biological material may be collected and assessed for adequacy, for example, to assess the suitability of the sample for use in the methods and compositions of the present disclosure. The assessment may be performed by the individual who obtains the sample, the molecular profiling business, the individual using a kit, or a third party such as a cytological lab, pathologist, endocrinologist, or a researcher. The sample may be determined to be adequate or inadequate for further analysis due to many factors including but not limited to: insufficient cells, insufficient genetic material, insufficient protein, DNA, or RNA, inappropriate cells for the indicated test, or inappropriate material for the indicated test, age of the sample, manner in which the sample was obtained, or manner in which the sample was stored or transported. Adequacy may be determined using a variety of methods known in the art such as a cell staining procedure, measurement of the number of cells or amount of tissue, measurement of total protein, measurement of nucleic acid, visual examination, microscopic examination, or temperature or pH determination. In one embodiment, sample adequacy will be determined from the results of performing a gene expression product level analysis experiment. In another embodiment sample adequacy will be determined by measuring the content of a marker of sample adequacy. Such markers include elements such as iodine, calcium, magnesium, phosphorous, carbon, nitrogen, sulfur, iron etc.; proteins such as but not limited to thyroglobulin; cellular mass; and cellular components such as protein, nucleic acid, lipid, or carbohydrate.

In some cases, iodine may be measured by a chemical method such as described in US Pat. No. 3645691 or other chemical methods known in the art for measuring iodine content. Chemical methods for iodine measurement include but are not limited to methods based on the Sandell and Kolthoff reaction. Said reaction proceeds according to the following equation:

$$2\ Ce^{4+} + As^3 + \rightarrow 2\ Ce^{3+} + As^5 + I.$$

Iodine has a catalytic effect upon the course of the reaction, i.e., the more iodine present in the preparation to be analyzed, the more rapidly the reaction proceeds. The speed of reaction is proportional to the iodine concentration. In some cases, this analytical method may carried out in the following manner:

A predetermined amount of a solution of arsenous oxide $As_2O_3$ in concentrated sulfuric or nitric acid is added to the biological sample and the temperature of the mixture is adjusted to reaction temperature, i.e., usually to a temperature between 20° C. and 60° C. A predetermined amount of a cerium (IV) sulfate solution in sulfuric or nitric acid is added thereto. Thereupon, the mixture is allowed to react at the predetermined temperature for a definite period of time. Said reaction time is selected in accordance with the order of magnitude of the amount of iodine to be determined and with the respective selected reaction temperature. The reaction time is usually between about 1 minute and about 40 minutes. Thereafter, the content of the test solution of cerium (IV) ions is determined photometrically. The lower the photometrically determined cerium (IV) ion concentration is, the higher is the speed of reaction and, consequently, the amount of catalytic agent, i.e., of iodine. In this manner the iodine of the sample can directly and quantitatively be determined.

In other cases, iodine content of a sample of thyroid tissue may be measured by detecting a specific isotope of iodine such as for example $^{123}I$, $^{124}I$, $^{125}I$, and $^{131}I$. In still other cases, the marker may be another radioisotope such as an isotope of carbon, nitrogen, sulfur, oxygen, iron, phosphorous, or hydrogen. The radioisotope in some instances may be administered prior to sample collection. Methods of radioisotope administration suitable for adequacy testing are well known in the art and include injection into a vein or artery, or by ingestion. A suitable period of time between administration of the isotope and acquisition of thyroid nodule sample so as to effect absorption of a portion of the isotope into the thyroid tissue may include any period of time between about a minute and a few days or about one week including about 1 minute, 2 minutes, 5 minutes, 10 minutes, 15 minutes, ½ an hour, an hour, 8 hours, 12 hours, 24 hours, 48 hours, 72 hours, or about one, one and a half, or two weeks, and may readily be determined by one skilled in the art. Alternatively, samples may be measured for natural levels of isotopes such as radioisotopes of iodine, calcium, magnesium, carbon, nitrogen, sulfur, oxygen, iron, phosphorous, or hydrogen.

## (i) Cell and/or Tissue Content Adequacy Test

Methods for determining the amount of a tissue include but are not limited to weighing the sample or measuring the volume of sample. Methods for determining the amount of cells include but are not limited to counting cells which may in some cases be performed after dis-aggregation with for example an enzyme such as trypsin or collagenase or by physical means such as using a tissue homogenizer for example. Alternative methods for determining the amount of cells recovered include but are not limited to quantification of dyes that bind to cellular material, or measurement of the volume of cell pellet obtained following centrifugation. Methods for determining that an adequate number of a specific type of cell is present include PCR, Q-PCR, RT-PCR, immuno-histochemical analysis, cytological analysis, microscopic, and or visual analysis.

(ii) Nucleic Acid Content Adequacy Test

Samples may be analyzed by determining nucleic acid content after extraction from the biological sample using a variety of methods known to the art. In some cases, nucleic acids such as RNA or mRNA is extracted from other nucleic acids prior to nucleic acid content analysis. Nucleic acid content may be extracted, purified, and measured by ultraviolet absorbance, including but not limited to absorbance at 260 nanometers using a spectrophotometer. In other cases nucleic acid content or adequacy may be measured by fluorometer after contacting the sample with a stain. In still other cases, nucleic acid content or adequacy may be measured after electrophoresis, or using an instrument such as an agilent bioanalyzer for example. It is understood that the methods of the present invention are not limited to a specific method for measuring nucleic acid content and or integrity.

In some embodiments, the RNA quantity or yield from a given sample is measured shortly after purification using a NanoDrop spectrophotometer in a range of nano- to micrograms. In some embodiments, RNA quality is measured using an Agilent 2100 Bioanalyzer instrument, and is characterized by a calculated RNA Integrity Number (RIN, 1-10). The NanoDrop is a cuvette-free spectrophotometer. It uses 1 microliter to measure from 5 ng/$\mu$l to 3,000 ng/$\mu$l of sample. The key features of NanoDrop include low volume of sample and no cuvette; large dynamic range 5 ng/$\mu$l to 3,000 ng/$\mu$l; and it allows quantitation of DNA, RNA and proteins. NanoDrop™ 2000c allows for the analysis of 0.5 $\mu$l - 2.0 $\mu$l samples, without the need for cuvettes or capillaries.

RNA quality can be measured by a calculated RNA Integrity Number (RIN). The RNA integrity number (RIN) is an algorithm for assigning integrity values to RNA measurements. The integrity of RNA is a major concern for gene expression studies and traditionally has been evaluated using the 28S to 18S rRNA ratio, a method that has been shown to be inconsistent. The RIN algorithm is applied to electrophoretic RNA measurements and based on a combination of different features that contribute information about the RNA integrity to provide a more robust universal measure. In some embodiments, RNA quality is measured using an Agilent 2100 Bioanalyzer instrument. The protocols for measuring RNA quality are known and available commercially, for example, at Agilent website. Briefly, in the first step, researchers deposit total RNA sample into an RNA Nano LabChip. In the second step, the LabChip is inserted into the Agilent bioanalyzer and let the analysis run, generating a digital electropherogram. In the third step, the new RIN algorithm then analyzes the entire electrophoretic trace of the RNA sample, including the presence or absence of degradation products, to determine sample integrity. Then, The algorithm assigns a 1 to 10 RIN score, where level 10 RNA is completely intact. Because interpretation of the electropherogram is automatic and not subject to individual interpretation, universal and unbiased comparison of samples is enabled and repeatability of experiments is improved. The RIN algorithm was developed using neural networks and adaptive learning in conjunction with a large database of eukaryote total RNA samples, which were obtained mainly from human, rat, and mouse tissues. Advantages of RIN include obtain a numerical assessment of the integrity of RNA; directly comparing RNA samples, e.g. before and after archival, compare integrity of same tissue across different labs; and ensuring repeatability of experiments, e.g. if RIN shows a given value and is suitable for microarray experiments, then the RIN of the same value can always be used for similar experiments given that the same organism/tissue/extraction method is used (Schroeder A, et al. BMC Molecular Biology 2006, 7:3 (2006)).

In some embodiments, RNA quality is measured on a scale of RIN 1 to 10, 10 being highest quality. In one aspect, the present invention provides a method of analyzing gene expression from a sample with an RNA RIN value equal or less than 6.0. In some embodiments, a sample containing RNA with an RIN number of 1.0, 2.0, 3.0, 4.0, 5.0 or 6.0 is analyzed for microarray gene expression using the subject methods and algorithms of the present invention. In the method of the invention, the sample is a fine needle aspirate of thyroid tissue. The sample can be degraded with an RIN as low as 2.0.

Determination of gene expression in a given sample can be a complex, dynamic, and expensive process. RNA samples with RIN ≤5.0 are typically not used for multi-gene microarray analysis, and may instead be used only for single-gene RT-PCR and/or TaqMan assays. This dichotomy in the usefulness of RNA according to quality has thus far limited the usefulness of samples and hampered research efforts. The present invention provides methods via which low quality RNA can be used to obtain meaningful multi-gene expression results from samples containing low concentrations of RNA, for example, thyroid FNA samples.

In addition, samples having a low and/or un-measurable RNA concentration by NanoDrop normally deemed inadequate for multi-gene expression profiling can be measured and analyzed using the subject methods and algorithms of the present invention. A sensitive apparatus that can be used to measure nucleic acid yield is the NanoDrop spectrophotometer. Like many quantitative instruments of its kind, the accuracy of a NanoDrop measurement decreases significantly with very low RNA concentration. The minimum amount of RNA necessary for input into a microarray experiment also limits the usefulness of a given sample. In the present invention, a sample containing a very low amount of nucleic acid can be estimated using a combination of the measurements from both the NanoDrop and the Bioanalyzer instruments, thereby optimizing the sample for multi-gene expression assays and analysis.

### (iii) Protein Content Adequacy Test

In some cases, protein content in the biological sample may be measured using a variety of methods known to the art, including but not limited to: ultraviolet absorbance at 280 nanometers, cell staining as described herein, or protein staining with for example coomassie blue, or bichichonic acid. In some cases, protein is extracted from the biological sample prior to measurement of the sample. In some cases, multiple tests for adequacy of the sample may be performed in parallel, or one at a time. In some cases, the sample may be divided into aliquots for the purpose of performing multiple diagnostic tests prior to, during, or after assessing adequacy. In some cases, the adequacy test is performed on a small amount of the sample which may or may not be suitable for further diagnostic testing. In other cases, the entire sample is assessed for adequacy. In any case, the test for adequacy may be billed to the subject, medical provider, insurance provider, or government entity.

In some embodiments of the present invention, the sample may be tested for adequacy soon or immediately after collection. In some cases, when the sample adequacy test does not indicate a sufficient amount sample or sample of sufficient quality, additional samples may be taken.

### VI. Analysis of Sample

In one aspect, the present invention provides methods for performing microarray gene expression analysis with low quantity and quality of polynucleotide, such as DNA or RNA. In some embodiments, the present disclosure describes methods of diagnosing, characterizing and/or monitoring a cancer by analyzing gene expression with low quantity and/or quality of RNA. In one embodiment, the cancer is thyroid cancer. Thyroid RNA can be obtained from fine needle aspirates (FNA). In some embodiments, gene expression profile is obtained from degraded samples with an RNA RIN value of about or less than about 9.0, 8.0, 7.0, 6.0, 5.0, 4.0, 3.0, 2.0, 1.0 or less. In particular embodiments, a gene expression profile is obtained from a sample with an RIN of equal or less than 6, i.e. 6.0, 5.0, 4.0, 3.0, 2.0, 1.0 or less. Provided by the present invention are methods by which low quality RNA can be used to obtain meaningful gene expression results from samples containing low concentrations of nucleic acid, such as thyroid FNA samples.

Another estimate of sample usefulness is RNA yield, typically measured in nanogram to microgram amounts for gene expression assays. An apparatus that can be used to measure nucleic acid yield in the laboratory is the NanoDrop spectrophotometer. Like many quantitative instruments of its kind, the accuracy of a NanoDrop measurement decreases significantly with very low RNA concentration. The minimum amount of RNA necessary for input into a microarray experiment also limits the usefulness of a given sample. In some aspects, the present invention solves the low RNA concentration problem by estimating sample input using a combination of the measurements from both the NanoDrop and the Bioanalyzer instruments. Since the quality of data obtained from a gene expression study is dependent on RNA quantity, meaningful gene expression data can be generated from samples having a low or un-measurable RNA concentration as measured by NanoDrop.

The subject methods and algorithms enable: 1) gene expression analysis of samples containing low amount and/or low quality of nucleic acid; 2) a significant reduction of false positives and false negatives, 3) a determination of the underlying genetic, metabolic, or signaling pathways responsible for the resulting pathology, 4) the ability to assign a statistical probability to the accuracy of the diagnosis of genetic disorders, 5) the ability to resolve ambiguous results, and 6) the ability to distinguish between sub-types of cancer.

### Cytological Analysis

Samples may be analyzed by cell staining combined with microscopic examination of the cells in the biological sample. Cell staining, or cytological examination, may be performed by a number of methods and suitable reagents known to the art including but not limited to: EA stains, hematoxylin stains, cytostain, papanicolaou stain, eosin, nissl stain, toluidine blue, silver stain, azocarmine stain, neutral red, or janus green. In some cases the cells are fixed and/or permeablized with for example methanol, ethanol, glutaraldehyde or formaldehyde prior to or during the staining procedure. In some cases, the cells are not fixed. In some cases, more than one stain is used in combination. In other cases no stain is used at all. In some cases measurement of nucleic acid content is performed using a staining procedure, for example with ethidium bromide, hematoxylin, nissl stain or any nucleic acid stain known to the art.

In some embodiments of the present invention, cells may be smeared onto a slide by standard methods well known in the art for cytological examination. In other cases, liquid based cytology (LBC) methods may be utilized. In some cases, LBC methods provide for an improved means of cytology slide preparation, more homogenous samples, increased sensitivity and specificity, and improved efficiency of handling of samples. In liquid based cytology methods, biological samples are transferred from the subject to a container or vial containing a liquid cytology preparation solution such as for example Cytyc ThinPrep, SurePath, or Monoprep or any other liquid based cytology preparation solution known in the art. Additionally, the sample may be rinsed from the collection device with liquid cytology preparation solution into the container or vial to ensure substantially quantitative transfer of the sample. The solution containing the biological sample in liquid based cytology preparation solution may then be stored and/or processed by a machine or by one skilled in the art to produce a layer of cells on a glass slide. The sample may further be stained and examined under the microscope in the same way as a conventional cytological preparation.

In some embodiments of the present invention, samples may be analyzed by immuno-histochemical staining. Immuno-histochemical staining provides for the analysis of the presence, location, and distribution of specific molecules or antigens by use of antibodies in a biological sample (e.g. cells or tissues). Antigens may be small molecules, proteins, peptides, nucleic acids or any other molecule capable of being specifically recognized by an antibody. Samples may be analyzed by immuno-histochemical methods with or without a prior fixing and/or permeabilization step. In some cases, the antigen of interest may be detected by contacting the sample with an antibody specific for the antigen and then non-specific binding may be removed by one or more washes. The specifically bound antibodies may then be detected by an antibody detection reagent such as for example a labeled secondary antibody, or a labeled avidin/streptavidin. In some cases, the antigen specific antibody may be labeled directly instead. Suitable labels for immuno-histochemistry include but are not limited to fluorophores such as fluoroscein and rhodamine, enzymes such as alkaline phosphatase and horse radish peroxidase, and radionuclides such as $^{32}$P and $^{125}$I. Gene product markers that may be detected by immuno-histochemical staining include but are not limited to Her2/Neu, Ras, Rho, EGFR, VEGFR, UbcH10, RET/PTC1, cytokeratin 20, calcitonin, GAL-3, thyroid peroxidase, and thyroglobulin.

### VII. Assay Results

The results of routine cytological or other assays may indicate a sample as negative (cancer, disease or condition free), ambiguous or suspicious (suggestive of the presence of a cancer, disease or condition), diagnostic (positive diagnosis for a cancer, disease or condition), or non diagnostic (providing inadequate information concerning the presence or absence of cancer, disease, or condition). The diagnostic results may be further classified as malignant or benign. The diagnostic results may also provide a score indicating for example, the severity or grade of a cancer, or the likelihood of an accurate diagnosis, such as via a p-value, a corrected p-value, or a statistical confidence indicator. In some cases, the diagnostic results may be indicative of a particular type of a cancer, disease, or condition, such as for example follicular adenoma (FA), nodular hyperplasia (NHP), lymphocytic thyroiditis (LCT), Hurthle cell adenoma (HA), follicular carcinoma (FC), papillary thyroid carcinoma (PTC), follicular variant of papillary carcinoma (FVPTC), medullary thyroid carcinoma (MTC), Hürthle cell carcinoma (HC), anaplastic thyroid carcinoma (ATC), renal carcinoma (RCC), breast carcinoma (BCA), melanoma (MMN), B cell lymphoma (BCL), parathyroid (PTA), hyperplasia, papillary carcinoma, or any of the diseases or conditions provided herein. In some cases, the diagnostic results may be indicative of a particular stage of a cancer, disease, or condition. The diagnostic results may inform a particular treatment or therapeutic intervention for the condition (e.g. type or stage of the specific cancer disease or condition) diagnosed. In some embodiments, the results of the assays performed may be entered into a database. The molecular profiling company may bill the individual, insurance provider, medical provider, or government entity for one or more of the following: assays performed, consulting services, reporting of results, database access, or data analysis. In some cases all or some steps other than molecular profiling are performed by a cytological laboratory or a medical professional.

### VIII. Molecular Profiling

Cytological assays mark the current diagnostic standard for many types of suspected tumors including for example thyroid tumors or nodules. In some embodiments of the present invention, samples that assay as negative, indeterminate, diagnostic, or non diagnostic may be subjected to subsequent assays to obtain more information. In the present invention, these subsequent assays comprise the steps of molecular profiling of genomic DNA, RNA, mRNA expression product levels, miRNA levels, gene expression product levels or gene expression product alternative splicing. In some embodiments of the present invention, molecular profiling means the determination of the number (e.g. copy number) and/or type of genomic DNA in a biological sample. In some cases, the number and/or type may further be compared to a control sample or a sample considered normal. In some embodiment, genomic DNA can be analyzed for copy number variation, such as an increase (amplification) or decrease in copy number, or variants, such as insertions, deletions, truncations and the like. Molecular profiling may be performed on the same sample, a portion of the same sample, or a new sample may be acquired using any of the methods described herein. A molecular profiling company may request an additional sample by directly contacting the individual or through an intermediary such as a physician, third party testing center or laboratory, or a medical professional. In some cases, samples are assayed using methods and compositions of the invention in combination with some or all cytological staining or other diagnostic methods. In other cases, samples are directly assayed using the methods and compositions of the invention without the previous use of routine cytological staining or other diagnostic methods. In some cases the results of molecular profiling alone or in combination with cytology or other assays may enable those skilled in the art to characterize a tissue sample, diagnose a subject, or suggest treatment for a subject. In some cases, molecular profiling may be used alone or in combination with cytology to monitor tumors or suspected tumors over time for malignant changes.

The molecular profiling methods of the present invention provide for extracting and analyzing protein or nucleic acid (RNA or DNA) from one or more biological samples from a subject. In some cases, nucleic acid is extracted from the entire sample obtained. In other cases, nucleic acid is extracted from a portion of the sample obtained. In some cases, the portion of the sample not subjected to nucleic acid extraction may be analyzed by cytological examination or immuno-histochemistry. Methods for RNA or DNA extraction from biological samples are well known in the art and include for example the use of a commercial kit, such as the Qiagen DNeasy Blood and Tissue Kit, or the Qiagen EZ1 RNA Universal Tissue Kit.

(i)Tissue-type fingerprinting

In many cases, biological samples such as those provided by the methods of the present invention may contain several cell types or tissues, including but not limited to thyroid follicular cells, thyroid medullary cells, blood cells (RBCs, WBCs, platelets), smooth muscle cells, ducts, duct cells, basement membrane, lumen, lobules, fatty tissue, skin cells, epithelial cells, and infiltrating macrophages and lymphocytes. In the case of thyroid samples, diagnostic classification of the biological samples may involve for example primarily follicular cells (for cancers derived from the follicular cell such as papillary carcinoma, follicular carcinoma, and anaplastic thyroid carcinoma) and medullary cells (for medullary cancer). The diagnosis of indeterminate biological samples from thyroid biopsies in some cases concerns the distinction of follicular adenoma vs. follicular carcinoma. The molecular profiling signal of a follicular cell for example may thus be diluted out and possibly confounded by other cell types present in the sample. Similarly diagnosis of biological samples from other tissues or organs often involves diagnosing one or more cell types among the many that may be present in the sample.

In some embodiments, the methods of the present invention provide for an upfront method of determining the cellular make-up of a particular biological sample so that the resulting molecular profiling signatures can be calibrated against the dilution effect due to the presence of other cell and/or tissue types. In one aspect, this upfront method is an algorithm that uses a combination of known cell and/or tissue specific gene expression patterns as an upfront mini-classifier for each component of the sample. This algorithm utilizes this molecular fingerprint to pre-classify the samples according to their composition and then apply a correction/normalization factor. This data may in some cases then feed in to a final classification algorithm which would incorporate that information to aid in the final diagnosis.

(ii) Genomic Analysis

In some embodiments, genomic sequence analysis, or genotyping, may be performed on the sample. This genotyping may take the form of mutational analysis such as single nucleotide polymorphism (SNP) analysis, insertion deletion polymorphism (InDel) analysis, variable number of tandem repeat (VNTR) analysis, copy number variation (CNV) analysis or partial or whole genome sequencing. Methods for performing genomic analyses are known to the art and may include high throughput sequencing such as but not limited to those methods described in US Patent Nos. 7,335,762; 7,323,305; 7,264,929; 7,244,559; 7,211,390; 7,361,488; 7,300,788; and 7,280,922. Methods for performing genomic analyses may also include microarray methods as described hereinafter. In some cases, genomic analysis may be performed in combination with any of the other methods herein. For example, a sample may be obtained, tested for adequacy, and divided into aliquots. One or more aliquots may then be used for cytological analysis of the present invention, one or more may be used for RNA expression profiling methods of the present invention, and one or more can be used for genomic analysis. It is further understood that the present invention anticipates that one skilled in the art may wish to perform other analyses on the biological sample that are not explicitly provided herein.

(iii) Expression Product Profiling

Gene expression profiling generally comprises the measurement of the activity (or the expression) of a plurality of genes (e.g.at least 10, 50, 100, 200, 300, 400, 500, 600, 700, 800, 1000, 2000, 3000, 4000, 5000, 10000, 15000, 20000, or more genes) at once, to create a global picture of cellular function. Gene expression profiles can be used, for example, to distinguish between cells that are actively dividing, or to show how the cells react to a particular treatment. Many experiments of this sort measure an entire genome simultaneously, that is, every gene present in a particular cell. Microarray technology can be used to measure the relative activity of previously identified target genes and other expressed sequences. Sequence based techniques, like serial analysis of gene expression (SAGE, SuperSAGE) are also used for gene expression profiling. SuperSAGE is especially accurate and can measure any active gene, not just a predefined set. In an RNA, mRNA or gene expression profiling microarray, the expression levels of thousands of genes can be simultaneously monitored to study the effects of certain treatments, diseases, and developmental stages on gene expression. For example, microarray-based gene expression profiling can be used to characterize gene signatures of a genetic disorder disclosed herein, or different cancer types, subtypes of a cancer, and/or cancer stages.

RNA (including mRNA, miRNA, siRNA, and cRNA) can be measured by one or more of the following: microarray, SAGE, blotting, RT-PCR, quantitative PCR, sequencing, RNA sequencing, DNA sequencing (e.g., sequencing of cDNA obtained from RNA); Next-Gen sequencing, nanopore sequencing, pyrosequencing, or Nanostring sequencing.

Expression profiling experiments often involve measuring the relative amount of gene expression products, such as mRNA, expressed in two or more experimental conditions. This is because altered levels of a specific sequence of a gene expression product can suggest a changed need for the protein coded for by the gene expression product, perhaps indicating a homeostatic response or a pathological condition. For example, if breast cancer cells express higher levels of mRNA associated with a particular transmembrane receptor than normal cells do, it might be that this receptor plays a role in breast cancer. One aspect of the present invention encompasses gene expression profiling as part of a process of identification or characterization of a tissue sample, such as a diagnostic test for genetic disorders and cancers, particularly, thyroid cancer.

In some embodiments, RNA samples with RIN ≤5.0 are typically not used for multi-gene microarray analysis, and may instead be used only for single-gene RT-PCR and/or TaqMan assays. Microarray, RT-PCR and TaqMan assays are standard molecular techniques well known in the relevant art. TaqMan probe-based assays are widely used in real-time PCR including gene expression assays, DNA quantification and SNP genotyping.

In one embodiment, gene expression products related to cancer that are known to the art are profiled. Such gene expression products have been described and include but are not limited to the gene expression products detailed in US patent Nos. 7,358,061; 7,319,011; 5,965,360; 6,436,642; and US patent applications 2003/0186248, 2005/0042222, 2003/0190602, 2005/0048533, 2005/0266443, 2006/0035244, 2006/083744, 2006/0088851, 2006/0105360, 2006/0127907, 2007/0020657, 2007/0037186, 2007/0065833, 2007/0161004, 2007/0238119, and 2008/0044824.

It is further anticipated that other gene expression products related to cancer may become known, and that the methods and compositions described herein may include such newly discovered gene expression products.

In some embodiments of the present invention gene expression products are analyzed alternatively or additionally for characteristics other than expression level. For example, gene products may be analyzed for alternative splicing. Alternative splicing, also referred to as alternative exon usage, is the RNA splicing variation mechanism wherein the exons of a primary gene transcript, the pre-mRNA, are separated and reconnected (i.e. spliced) so as to produce alternative mRNA molecules from the same gene. In some cases, these linear combinations then undergo the process of translation where a specific and unique sequence of amino acids is specified by each of the alternative mRNA molecules from the same gene resulting in protein isoforms. Alternative splicing may include incorporating different exons or different sets of exons, retaining certain introns, or utilizing alternate splice donor and acceptor sites.

In some cases, markers or sets of markers may be identified that exhibit alternative splicing that is diagnostic for benign, malignant or normal samples. Additionally, alternative splicing markers may further provide an identifier for a specific type of thyroid cancer (e.g. papillary, follicular, medullary, or anaplastic). Alternative splicing markers diagnostic for malignancy known to the art include those listed in US Pat. No. 6,436,642.

In some cases, expression of gene expression products that do not encode for proteins such as miRNAs, and siRNAs may be assayed by the methods of the present invention. Differential expression of these gene expression products may be indicative of benign, malignant or normal samples. Differential expression of these gene expression products may further be indicative of the subtype of the benign sample (e.g. FA, NHP, LCT, BN, CN, HA) or malignant sample (e.g. FC, PTC, FVPTC, ATC, MTC). In some cases, differential expression of miRNAs, siRNAs, alternative splice RNA isoforms, mRNAs or any combination thereof may be assayed by the methods of the present invention.

(1) In Vitro methods of determining expression product levels

The general methods for determining gene expression product levels are known to the art and may include but are not limited to one or more of the following: additional cytological assays, assays for specific proteins or enzyme activities, assays for specific expression products including protein or RNA or specific RNA splice variants, *in situ* hybridization, whole or partial genome expression analysis, microarray hybridization assays, SAGE, enzyme linked immuno-absorbance assays, mass-spectrometry, immuno-histochemistry, blotting, sequencing, RNA sequencing, DNA sequencing (e.g., sequencing of cDNA obtained from RNA); Next-Gen sequencing, nanopore sequencing, pyrosequencing, or Nanostring sequencing. Gene expression product levels may be normalized to an internal standard such as total mRNA or the expression level of a particular gene including but not limited to glyceraldehyde 3 phosphate dehydrogenase, or tublin.

In some embodiments, the gene expression product of the subject methods is a protein, and the amount of protein in a particular biological sample is analyzed using a classifier derived from protein data obtained from cohorts of samples. The amount of protein can be determined by one or more of the following: ELISA, mass spectrometry, blotting, or immunohistochemistry.

In some embodiments of the present invention, gene expression product markers and alternative splicing markers may be determined by microarray analysis using, for example, Affymetrix arrays, cDNA microarrays, oligonucleotide microarrays, spotted microarrays, or other microarray products from Biorad, Agilent, or Eppendorf. Microarrays provide particular advantages because they may contain a large number of genes or alternative splice variants that may be assayed in a single experiment. In some cases, the microarray device may contain the entire human genome or transcriptome or a substantial fraction thereof allowing a comprehensive evaluation of gene expression patterns, genomic sequence, or alternative splicing. Markers may be found using standard molecular biology and microarray analysis techniques as described in Sambrook Molecular Cloning a Laboratory Manual 2001 and Baldi, P., and Hatfield, W.G., DNA Microarrays *and* Gene Expression 2002.

Microarray analysis generally begins with extracting and purifying nucleic acid from a biological sample, (e.g. a biopsy or fine needle aspirate) using methods known to the art. For expression and alternative splicing analysis it may be advantageous to extract and/or purify RNA from DNA. It may further be advantageous to extract and/or purify mRNA from other forms of RNA such as tRNA and rRNA.

Purified nucleic acid may further be labeled with a fluorescent label, radionuclide, or chemical label such as biotin, digoxigenin, or digoxin for example by reverse transcription, PCR, ligation, chemical reaction or other techniques. The labeling can be direct or indirect which may further require a coupling stage. The coupling stage can occur before hybridization, for example, using aminoallyl-UTP and NHS amino-reactive dyes (like cyanine dyes) or after, for example, using biotin and labelled streptavidin. In one example, modified nucleotides (e.g. at a 1 aaUTP: 4 TTP ratio) are added enzymatically at a lower rate compared to normal nucleotides, typically resulting in 1 every 60 bases (measured with a spectrophotometer). The aaDNA may then be purified with, for example, a column or a diafiltration device. The aminoallyl group is an amine group on a long linker attached to the nucleobase, which reacts with a reactive label (e.g. a fluorescent dye).

The labeled samples may then be mixed with a hybridization solution which may contain SDS, SSC, dextran sulfate, a blocking agent (such as COT1 DNA, salmon sperm DNA, calf thymum DNA, PolyA or PolyT), Denhardt's solution, formamine, or a combination thereof.

A hybridization probe is a fragment of DNA or RNA of variable length, which is used to detect in DNA or RNA samples the presence of nucleotide sequences (the DNA target) that are complementary to the sequence in the probe. The probe thereby hybridizes to single-stranded nucleic acid (DNA or RNA) whose base sequence allows probe-target base pairing due to complementarity between the probe and target. The labeled probe is first denatured (by heating or under alkaline conditions) into single DNA strands and then hybridized to the target DNA.

To detect hybridization of the probe to its target sequence, the probe is tagged (or labeled) with a molecular marker; commonly used markers are $^{32}$P or Digoxigenin, which is non-radioactive antibody-based marker. DNA sequences or RNA transcripts that have moderate to high sequence complementarity (e.g. at least 70%, 80%, 90%, 95%, 96%, 97%, 98%, 99%, or more complementarity) to the probe are then detected by visualizing the hybridized probe via autoradiography or other imaging techniques. Detection of sequences with moderate or high complementarity depends on how stringent the hybridization conditions were applied - high stringency, such as high hybridization temperature and low salt in hybridization buffers, permits only hybridization between nucleic acid sequences that are highly similar, whereas low stringency, such as lower temperature and high salt, allows hybridization when the sequences are less similar. Hybridization probes used in DNA microarrays refer to DNA covalently attached to an inert surface, such as coated glass slides or gene chips, and to which a mobile cDNA target is hybridized.

A mix comprising target nucleic acid to be hybridized to probes on an array may be denatured by heat or chemical means and added to a port in a microarray. The holes may then be sealed and the microarray hybridized, for example, in a hybridization oven, where the microarray is mixed by rotation, or in a mixer. After an overnight hybridization, non specific binding may be washed off (e.g. with SDS and SSC). The microarray may then be dried and scanned in a machine comprising a laser that excites the dye and a detector that measures emission by the dye. The image may be overlaid with a template grid and the intensities of the features (e.g. a feature comprising several pixels) may be quantified.

Various kits can be used for the amplification of nucleic acid and probe generation of the subject methods. Examples of kit that can be used in the present invention include but are not limited to Nugen WT-Ovation FFPE kit, cDNA amplification kit with Nugen Exon Module and Frag/Label module. The NuGEN WT-Ovation™ FFPE System V2 is a whole transcriptome amplification system that enables conducting global gene expression analysis on the vast archives of small and degraded RNA derived from FFPE samples. The system is comprised of reagents and a protocol required for amplification of as little as 50 ng of total FFPE RNA. The protocol can be used for qPCR, sample archiving, fragmentation, and labeling. The amplified cDNA can be fragmented and labeled in less than two hours for GeneChip® 3' expression array analysis using NuGEN's FL-Ovation™ cDNA Biotin Module V2. For analysis using Affymetrix GeneChip® Exon and Gene ST arrays, the amplified cDNA can be used with the WT-Ovation Exon Module, then fragmented and labeled using the FL-Ovation™ cDNA Biotin Module V2. For analysis on Agilent arrays, the amplified cDNA can be fragmented and labeled using NuGEN's FL-Ovation™ cDNA Fluorescent Module. More information on Nugen WT-Ovation FFPE kit can be obtained at www.nugeninc.com/nugen/index.cfirn/products/amplification-systems/wt-ovation-ffpe/.

In some embodiments, Ambion WT-expression kit can be used. Ambion WT-expression kit allows amplification of total RNA directly without a separate ribosomal RNA (rRNA) depletion step. With the Ambion® WT Expression Kit, samples as small as 50 ng of total RNA can be analyzed on Affymetrix® GeneChip® Human, Mouse, and Rat Exon and Gene 1.0 ST Arrays. In addition to the lower input RNA requirement and high concordance between the Affymetrix® method and TaqMan® real-time PCR data, the Ambion® WT Expression Kit provides a significant increase in sensitivity. For example, a greater number of probe sets detected above background can be obtained at the exon level with the Ambion® WT Expression Kit as a result of an increased signal-to-noise ratio. Ambion WT-expression kit may be used in combination with additional Affymetrix labeling kit.

In some embodiments, AmpTec Trinucleotide Nano mRNA Amplification kit (6299-A15) can be used in the subject methods. The ExpressArt® TR*inucleotide* mRNA amplification Nano kit is suitable for a wide range, from 1 ng to 700 ng of input total RNA. According to the amount of input total RNA and the required yields of aRNA, it can be used for 1-round (input >300 ng total RNA) or 2-rounds (minimal input amount 1 ng total RNA), with aRNA yields in the range of >10 $\mu$g. AmpTec's proprietary TR*inucleotide* priming technology results in preferential amplification of mRNAs (independent of the universal eukaryotic 3'-poly(A)-sequence), combined with selection against rRNAs. More information on AmpTec Trinucleotide Nano mRNA Amplification kit can be obtained at www.amp-tec.com/products.htm. This kit can be used in combination with cDNA conversion kit and Affymetrix labeling kit.

The raw data may then be normalized, for example, by subtracting the background intensity and then dividing the intensities making either the total intensity of the features on each channel equal or the intensities of a reference gene and then the t-value for all the intensities may be calculated. More sophisticated methods, include z-ratio, loess and lowess regression and RMA (robust multichip analysis), such as for Affymetrix chips.

### (2) In Vivo methods of determining gene expression product levels

It is further anticipated that the methods and compositions of the present disclosure may be used to determine gene expression product levels in an individual without first obtaining a sample. For example, gene expression product levels may be determined *in vivo,* that is in the individual. Methods for determining gene expression product levels *in vivo* are known to the art and include imaging techniques such as CAT, MRI; NMR; PET; and optical, fluorescence, or biophotonic imaging of protein or RNA levels using antibodies or molecular beacons. Such methods are described in US 2008/0044824, US 2008/0131892. Additional methods for *in vivo* molecular profiling are contemplated to be within the scope of the present disclosure.

In some embodiments of the present invention, molecular profiling includes the step of binding the sample or a portion of the sample to one or more probes of the present invention. Suitable probes bind to components of the sample, e.g. gene products, that are to be measured and include but are not limited to antibodies or antibody fragments, aptamers, nucleic acids, and oligonucleotides. The binding of the sample to the probes of the present invention represents a transformation of matter from sample to sample bound to one or more probes. In one embodiment, the method of identifying, characterizing, or diagnosing cancer based on molecular profiling further comprises the steps of detecting gene expression products (i.e. mRNA or protein) and levels of the sample; and classifying the test sample by inputting one or more differential gene expression product levels to a trained algorithm of the present invention; validating the sample classification using the selection and classification algorithms of the present invention; and identifying the sample as positive for a genetic disorder or a type of cancer.

### (i) Comparison of sample to normal

Results of molecular profiling performed on a sample from a subject (test sample) may be compared to a biological sample that is known or suspected to be normal. In some embodiments, a normal sample is a sample that does not comprise or is expected to not comprise one or more cancers, diseases, or conditions under evaluation, or would test negative in the molecular profiling assay for the one or more cancers, diseases, or conditions under evaluation. In some embodiments, a normal sample is that which is or is expected to be free of any cancer, disease, or condition, or a sample that would test negative for any cancer disease or condition in the molecular profiling assay. The normal sample may be from a different subject from the subject being tested, or from the same subject. In some cases, the normal sample is a sample obtained from a buccal swab of an subject such as the subject being tested for example. The normal sample may be assayed at the same time, or at a different time from the test sample.

The results of an assay on the test sample may be compared to the results of the same assay on a normal sample. In some cases the results of the assay on the normal sample are from a database, or a reference. In some cases, the results of the assay on the normal sample are a known or generally accepted value or range of values by those skilled in the art. In some cases the comparison is qualitative. In other cases the comparison is quantitative. In some cases, qualitative or quantitative comparisons may involve but are not limited to one or more of the following: comparing fluorescence values, spot intensities, absorbance values, chemiluminescent signals, histograms, critical threshold values, statistical significance values, gene product expression levels, gene product expression level changes, alternative exon usage, changes in alternative exon usage, protein levels, DNA polymorphisms, copy number variations, indications of the presence or absence of one or more DNA markers or regions, or nucleic acid sequences.

(ii) Evaluation of results

In some embodiments, the molecular profiling results are evaluated using methods known to the art for correlating gene product expression levels or alternative exon usage with specific phenotypes such as malignancy, the type of malignancy (e.g. follicular carcinoma), benignancy, or normalcy (e.g. disease or condition free). In some cases, a specified statistical confidence level may be determined in order to provide a diagnostic confidence level. For example, it may be determined that a confidence level of greater than 90% may be a useful predictor of malignancy, type of malignancy, or benignancy. In other embodiments, more or less stringent confidence levels may be chosen. For example, a confidence level of about or at least about 50%, 60%, 70%, 75%, 80%, 85%, 90%, 95%, 97.5%, 99%, 99.5%, or 99.9% may be chosen as a useful phenotypic predictor. The confidence level provided may in some cases be related to the quality of the sample, the quality of the data, the quality of the analysis, the specific methods used, and/or the number of gene expression products analyzed. The specified confidence level for providing a diagnosis may be chosen on the basis of the expected number of false positives or false negatives and/or cost. Methods for choosing parameters for achieving a specified confidence level or for identifying markers with diagnostic power include but are not limited to Receiver Operating Characteristic (ROC) curve analysis, binormal ROC, principal component analysis, partial least squares analysis, singular value decomposition, least absolute shrinkage and selection operator analysis, least angle regression, and the threshold gradient directed regularization method.

(iii) Data analysis

Raw gene expression level and alternative splicing data may in some cases be improved through the application of algorithms designed to normalize and or improve the reliability of the data. In some embodiments of the present invention the data analysis requires a computer or other device, machine or apparatus for application of the various algorithms described herein due to the large number of individual data points that are processed. A "machine learning algorithm" refers to a computational-based prediction methodology, also known to persons skilled in the art as a "classifier", employed for characterizing a gene expression profile. The signals corresponding to certain expression levels, which are obtained by, e.g., microarray-based hybridization assays, are typically subjected to the algorithm in order to classify the expression profile. Supervised learning generally involves "training" a classifier to recognize the distinctions among classes and then "testing" the accuracy of the classifier on an independent test set. For new, unknown samples the classifier can be used to predict the class in which the samples belong.

In some cases, the robust multi-array Average (RMA) method may be used to normalize raw data. The RMA method begins by computing background-corrected intensities for each matched cell on a number of microarrays. The background corrected values are restricted to positive values as described by Irizarry et al. Biostatistics 2003 April 4 (2): 249-64. After background correction, the base-2 logarithm of each background corrected matched-cell intensity is then obtained. The background corrected, log-transformed, matched intensity on each microarray is then normalized using the quantile normalization method in which for each input array and each probe expression value, the array percentile probe value is replaced with the average of all array percentile points, this method is more completely described by Bolstad et al. Bioinformatics 2003. Following quantile normalization, the normalized data may then be fit to a linear model to obtain an expression measure for each probe on each microarray. Tukey's median polish algorithm (Tukey, J.W., Exploratory Data Analysis. 1977) may then be used to determine the log-scale expression level for the normalized probe set data.

Data may further be filtered to remove data that may be considered suspect. In some embodiments, data deriving from microarray probes that have fewer than about 4, 5, 6, 7 or 8 guanosine + cytosine nucleotides may be considered to be unreliable due to their aberrant hybridization propensity or secondary structure issues. Similarly, data deriving from microarray probes that have more than about 12, 13, 14, 15, 16, 17, 18, 19, 20, 21, or 22 guanosine + cytosine nucleotides may be considered unreliable due to their aberrant hybridization propensity or secondary structure issues.

In some cases, unreliable probe sets may be selected for exclusion from data analysis by ranking probe-set reliability against a series of reference datasets. For example, RefSeq or Ensembl (EMBL) are considered very high quality reference datasets. Data from probe sets matching RefSeq or Ensembl sequences may in some cases be specifically included in microarray analysis experiments due to their expected high reliability. Similarly data from probe-sets matching less reliable reference datasets may be excluded from further analysis, or considered on a case by case basis for inclusion. In some cases, the Ensembl high throughput cDNA (HTC) and/or mRNA reference datasets may be used to determine the probe-set reliability separately or together. In other cases, probe-set reliability may be ranked. For example, probes and/or probe-sets that match perfectly to all reference datasets such as for example RefSeq, HTC, and mRNA, may be ranked as most reliable (1). Furthermore, probes and/or probe-sets that match two out of three reference datasets may be ranked as next most reliable (2), probes and/or probe-sets that match one out of three reference datasets may be ranked next (3) and probes and/or probe sets that match no reference datasets may be ranked last (4). Probes and or probe-sets may then be included or excluded from analysis based on their ranking. For example, one may choose to include data from category 1, 2, 3, and 4 probe-sets; category 1, 2, and 3 probe-sets; category 1 and 2 probe-sets; or category 1 probe-sets for further analysis. In another example, probe-sets may be ranked by the number of base pair mismatches to reference dataset entries. It is understood that there are many methods understood in the art for assessing the reliability of a given probe and/or probe-set for molecular profiling and the methods of the present invention encompass any of these methods and combinations thereof.

In some embodiments of the present invention, data from probe-sets may be excluded from analysis if they are not expressed or expressed at an undetectable level (not above background). A probe-set is judged to be expressed above background if for any group:

Integral from TO to Infinity of the standard normal distribution < Significance (0.01) Where:

TO = Sqr(GroupSize) (T - P) / Sqr(Pvar),
GroupSize = Number of CEL files in the group,
T = Average of probe scores in probe-set,
P = Average of Background probes averages of GC content, and
Pvar = Sum of Background probe variances / (Number of probes in probe-set)^2,

This allows including probe-sets in which the average of probe-sets in a group is greater than the average expression of background probes of similar GC content as the probe-set probes as the center of background for the probe-set and enables one to derive the probe-set dispersion from the background probe-set variance.

In some embodiments of the present invention, probe-sets that exhibit no, or low variance may be excluded from further analysis. Low-variance probe-sets are excluded from the analysis via a Chi-Square test. A probe-set is considered to be low-variance if its transformed variance is to the left of the 99 percent confidence interval of the Chi-Squared distribution with (N-1) degrees of freedom.

$$(N\text{-}1) * \text{Probe-set Variance} / (\text{Gene Probe-set Variance}) \sim \text{Chi-Sq}(N\text{-}1)$$

where N is the number of input CEL files, (N-1) is the degrees of freedom for the Chi-Squared distribution, and the 'probe-set variance for the gene' is the average of probe-set variances across the gene.

In some embodiments of the present invention, probe-sets for a given gene or transcript cluster may be excluded from further analysis if they contain less than a minimum number of probes that pass through the previously described filter steps for GC content, reliability, variance and the like. For example in some embodiments, probe-sets for a given gene or transcript cluster may be excluded from further analysis if they contain less than about 1, 2, 3, 4, 5, 6, 7, 8, 9, 10, 11, 12, 13, 14, 15, or less than about 20 probes.

Methods of data analysis of gene expression levels or of alternative splicing may further include the use of a feature selection algorithm as provided herein. In some embodiments of the present invention, feature selection is provided by use of the LIMMA software package (Smyth, G. K. (2005). Limma: linear models for microarray data. In: Bioinformatics and Computational Biology Solutions using R and Bioconductor, R. Gentleman, V. Carey, S. Dudoit, R. Irizarry, W. Huber (eds.), Springer, New York, pages 397-420).

Methods of data analysis of gene expression levels and or of alternative splicing may further include the use of a pre-classifier algorithm. For example, an algorithm may use a cell-specific molecular fingerprint to pre-classify the samples according to their composition and then apply a correction/normalization factor. This data/information may then be fed in to a final classification algorithm which would incorporate that information to aid in the final diagnosis.

Methods of data analysis of gene expression levels and/or of alternative splicing may further include the use of a classifier algorithm as provided herein. In some embodiments of the present invention a diagonal linear discriminant analysis, k-nearest neighbor algorithm, support vector machine (SVM) algorithm, linear support vector machine, random forest algorithm, or a probabilistic model-based method or a combination thereof is provided for classification of microarray data. In some embodiments, identified markers that distinguish samples (e.g. benign vs. malignant, normal vs. malignant) or distinguish subtypes (e.g. PTC vs. FVPTC) are selected based on statistical significance of the difference in expression levels between classes of interest. In some cases, the statistical significance is adjusted by applying a Benjamini Hochberg or another correction for false discovery rate (FDR).

In some cases, the classifier algorithm may be supplemented with a meta-analysis approach such as that described by Fishel and Kaufman et al. 2007 Bioinformatics 23(13): 1599-606. In some cases, the classifier algorithm may be supplemented with a meta-analysis approach such as a repeatability analysis. In some cases, the repeatability analysis selects markers that appear in at least one predictive expression product marker set.

Methods for deriving and applying posterior probabilities to the analysis of microarray data are known in the art and have been described for example in Smyth, G.K. 2004 Stat. Appl. Genet. Mol. Biol. 3: Article 3. In some cases, the posterior probabilities may be used to rank the markers provided by the classifier algorithm. In some cases, markers may be ranked according to their posterior probabilities and those that pass a chosen threshold may be chosen as markers whose differential expression is indicative of or diagnostic for samples that are for example benign, malignant, normal, ATC, PTC, MTC, FC, FN, FA, FVPTC, RCC, BCA, MMN, BCL, PTA, CN, HA, HC, LCT, or NHP. Illustrative threshold values include prior probabilities of 0.7, 0.75, 0.8, 0.85, 0.9, 0.925, 0.95, 0.975, 0.98, 0.985, 0.99, 0.995 or higher.

A statistical evaluation of the results of the molecular profiling may provide a quantitative value or values indicative of one or more of the following: the likelihood of diagnostic accuracy; the likelihood of cancer, disease or condition; the likelihood of a particular cancer, disease or condition (e.g. tissue type or cancer subtype); and the likelihood of the success of a particular therapeutic intervention. Thus a physician, who is not likely to be trained in genetics or molecular biology, need not understand the raw data. Rather, the data is presented directly to the physician in its most useful form to guide patient care. The results of the molecular profiling can be statistically evaluated using a number of methods known to the art including, but not limited to: the students T test, the two sided T test, pearson rank sum analysis, hidden markov model analysis, analysis of q-q plots, principal component analysis, one way ANOVA, two way ANOVA, LIMMA and the like.

In some embodiments of the present disclosure, the use of molecular profiling alone or in combination with cytological analysis may provide a classification, identification, or diagnosis that is between about 85% accurate and about 99% or about 100% accurate. In some cases, the molecular profiling process and/or cytology provide a classification, identification, diagnosis of malignant, benign, or normal that is about, or at least about 85%, 86%, 87%, 88%, 90%, 91%, 92%, 93%, 94%, 95%, 96%, 97%, 97.5%, 98%, 98.5%, 99%, 99.5%, 99.75%, 99.8%, 99.85%, or 99.9% accurate. In some embodiments, the molecular profiling process and/or cytology provide a classification, identification, or diagnosis of the presence of a particular tissue type (e.g. NML, FA, NHP, LCT, HA, FC, PTC, FVPTC, MTC, HC, ATC, RCC, BCA, MMN, BCL, and/or PTA) that is about, or at least about 85%, 86%, 87%, 88%, 90%, 91%, 92%, 93%, 94%, 95%, 96%, 97%, 97.5%, 98%, 98.5%, 99%, 99.5%, 99.75%, 99.8%, 99.85%, or 99.9% accurate.

In some cases, accuracy may be determined by tracking the subject over time to determine the accuracy of the original diagnosis. In other cases, accuracy may be established in a deterministic manner or using statistical methods. For example, receiver operator characteristic (ROC) analysis may be used to determine the optimal assay parameters to achieve a specific level of accuracy, specificity, positive predictive value, negative predictive value, and/or false discovery rate. Methods for using ROC analysis in cancer diagnosis are known in the art and have been described for example in US Patent Application No. 2006/019615.

In some embodiments of the present invention, gene expression products and compositions of nucleotides encoding for such products which are determined to exhibit the greatest difference in expression level or the greatest difference in alternative splicing between benign and normal, benign and malignant, or malignant and normal may be chosen for use as molecular profiling reagents of the present invention. Such gene expression products may be particularly useful by providing a wider dynamic range, greater signal to noise, improved diagnostic power, lower likelihood of false positives or false negative, or a greater statistical confidence level than other methods known or used in the art.

In other embodiments of the present invention, the use of molecular profiling alone or in combination with cytological analysis may reduce the number of samples scored as non-diagnostic by about, or at least about 100%, 99%, 95%, 90%, 80%, 75%, 70%, 65%, or about 60% when compared to the use of standard cytological techniques known to the art. In some cases, the methods of the present invention may reduce the number of samples scored as intermediate or suspicious by about, or at least about100%, 99%, 98%, 97%, 95%, 90%, 85%, 80%, 75%, 70%, 65%, or about 60%, when compared to the standard cytological methods used in the art.

In some cases the results of the molecular profiling assays, are entered into a database for access by representatives or agents of a molecular profiling business, the individual, a medical provider, or insurance provider. In some cases assay results include sample classification, identification, or diagnosis by a representative, agent or consultant of the business, such as a medical professional. In other cases, a computer or algorithmic analysis of the data is provided automatically. In some cases the molecular profiling business may bill the individual, insurance provider, medical provider, researcher, or government entity for one or more of the following: molecular profiling assays performed, consulting services, data analysis, reporting of results, or database access.

In some embodiments of the present invention, the results of the molecular profiling are presented as a report on a computer screen or as a paper record. In some cases, the report may include, but is not limited to, such information as one or more of the following: the number of genes differentially expressed, the suitability of the original sample, the number of genes showing differential alternative splicing, a diagnosis, a statistical confidence for the diagnosis, the likelihood of cancer or malignancy, and indicated therapies.

**(iv) Categorization of samples based on molecular profiling results**

The results of the molecular profiling may be classified into one of the following: benign (free of a malignant cancer, disease, or condition), malignant (positive diagnosis for a cancer, disease, or condition), or non diagnostic (providing inadequate information concerning the presence or absence of a cancer, disease, or condition). In some cases, the results of the molecular profiling may be classified into benign versus suspicious (suspected to be positive for a cancer, disease, or condition) categories. In some cases, a diagnostic result may further classify the type of cancer, disease or condition, such as by identifying the presence or absence of one or more types of tissues, including but not limited to NML, FA, NHP, LCT, HA, FC, PTC, FVPTC, MTC, HC, ATC, RCC, BCA, MMN, BCL, and PTA. In other cases, a diagnostic result may indicate a certain molecular pathway involved in the cancer disease or condition, or a certain grade or stage of a particular cancer disease or condition. In still other cases a diagnostic result may inform an appropriate therapeutic intervention, such as a specific drug regimen like a kinase inhibitor such as Gleevec or any drug known to the art, or a surgical intervention like a thyroidectomy or a hemithyroidectomy.

In some embodiments of the present invention, results are classified using a trained algorithm. Trained algorithms of the present invention include algorithms that have been developed using a reference set of known malignant, benign, and normal samples including but not limited to samples with one or more histopathologies listed in Figure 2. In some embodiments, the algorithm is further trained using one or more of the classification panels in Figure 3, in any combination. In some embodiments, training comprises comparison of gene expression product levels in a first set of one or more tissue types to gene expression product levels in a second set of one or more tissue types, where the first set of tissue types includes at least one tissue type that is not in the second set. In some embodiments, either the entire algorithm or portions of the algorithm can be trained using comparisons of expression levels of biomarker panels within a classification panel against all other biomarker panels (or all other biomarker signatures) used in the algorithm. The first set of tissue types and/or the second set of tissue types may include 1, 2, 3, 4, 5, 6, 7, 8, 9, 10, 11, 12, 13, 14, or 15 of the types selected from NML, FA, NHP, LCT, HA, FC, PTC, FVPTC, MTC, HC, ATC, RCC, BCA, MMN, BCL, and PTA, in any combination, and from any source, including surgical and/or FNA samples.

Algorithms suitable for categorization of samples include but are not limited to k-nearest neighbor algorithms, support vector algorithms, naive Bayesian algorithms, neural network algorithms, hidden Markov model algorithms, genetic algorithms, or any combination thereof.

In some cases, trained algorithms of the present invention may incorporate data other than gene expression or alternative splicing data such as but not limited to DNA polymorphism data, sequencing data, scoring or diagnosis by cytologists or pathologists of the present invention, information provided by the pre-classifier algorithm of the present invention, or information about the medical history of the subject of the present invention.

When classifying a biological sample for diagnosis of cancer, there are typically two possible outcomes from a binary classifier. When a binary classifier is compared with actual true values (e.g., values from a biological sample), there are typically four possible outcomes. If the outcome from a prediction is *p* (where "p" is a positive classifier output, such as a malignancy, or presence of a particular disease tissue as described herein) and the actual value is also *p*, then it is called a *true positive* (TP); however if the actual value is *n* then it is said to be a *false positive* (FP). Conversely, a *true negative* (e.g., definitive benign) has occurred when both the prediction outcome and the actual value are *n* (where "n" is a negative classifier output, such as benign, or absence of a particular disease tissue as described herein), and *false negative* is when the prediction outcome is *n* while the actual value is *p*. In one embodiment, consider a diagnostic test that seeks to determine whether a person has a certain disease. A false positive in this case occurs when the person tests positive, but actually does not have the disease. A false negative, on the other hand, occurs when the person tests negative, suggesting they are healthy, when they actually do have the disease. In some embodiments, a Receiver Operator Characteristic (ROC) curve assuming real-world prevalence of subtypes can be generated by re-sampling errors achieved on available samples in relevant proportions.

The positive predictive value (PPV), or precision rate, or post-test probability of disease, is the proportion of patients with positive test results who are correctly diagnosed. It is the most important measure of a diagnostic method as it reflects the probability that a positive test reflects the underlying condition being tested for. Its value does however depend on the prevalence of the disease, which may vary. In one example, FP (false positive); TN (true negative); TP (true positive); FN (false negative).

$$\text{False positive rate } (\alpha) = FP / (FP + TN) - \text{specificity}$$

$$\text{False negative rate } (\beta) = FN / (TP + FN) -- \text{sensitivity}$$

$$Power = sensitivity = 1 - \beta$$

$$Likelihood\text{-}ratio\ positive = sensitivity / (1 - specificity)$$

$$Likelihood\text{-}ratio\ negative = (1 - sensitivity) / specificity$$

The negative predictive value is the proportion of patients with negative test results who are correctly diagnosed. PPV and NPV measurements can be derived using appropriate disease subtype prevalence estimates. An estimate of the pooled malignant disease prevalence can be calculated from the pool of indeterminates which roughly classify into B vs M by surgery. For subtype specific estimates, in some embodiments, disease prevalence may sometimes be incalculable because there are not any available samples. In these cases, the subtype disease prevalence can be substituted by the pooled disease prevalence estimate.

In some embodiments, the level of expression products or alternative exon usage is indicate of one or the following: NML, FA, NHP, LCT, HA, FC, PTC, FVPTC, MTC, HC, ATC, RCC, BCA, MMN, BCL, and PTA. In some embodiments, the level of expression products or alternative exon usage is indicative of one of the following: follicular cell carcinoma, anaplastic carcinoma, medullary carcinoma, or papillary carcinoma. In some embodiments, the level of gene expression products or alternative exon usage in indicative of Hurthle cell carcinoma or Hurthle cell adenoma. In some embodiments, the one or more genes selected using the methods of the present invention for diagnosing cancer contain representative sequences corresponding to a set of metabolic or signaling pathways indicative of cancer.

In some embodiments, the results of the expression analysis of the subject methods provide a statistical confidence level that a given diagnosis is correct. In some embodiments, such statistical confidence level is at least about, or more than about 85%, 90%, 91%, 92%, 93%, 94%, 95%, 96%, 97%, 98%, 99% 99.5%, or more.

In another aspect, the present disclosure provides a composition for diagnosing cancer comprising oligonucleotides comprising a portion of one or more of the genes listed in Figure 4 or their complement, and a substrate upon which the oligonucleotides are covalently attached. The composition of the present disclosure is suitable for use in diagnosing cancer at a specified confidence level using a trained algorithm. In one example, the composition of the present disclosure is used to diagnose thyroid cancer.

For example, in the specific case of thyroid cancer, molecular profiling of the present invention may further provide a diagnosis for the specific type of thyroid cancer (e.g. papillary, follicular, medullary, or anaplastic), or other tissue type selected from NML, FA, NHP, LCT, HA, FC, PTC, FVPTC, MTC, HC, ATC, RCC, BCA, MMN, BCL, and PTA. In some embodiments, the methods of the invention provide a diagnosis of the presence or absence of Hurthle cell carcinoma or Hurthle cell adenoma. The results of the molecular profiling may further allow one skilled in the art, such as a scientist or medical professional to suggest or prescribe a specific therapeutic intervention. Molecular profiling of biological samples may also be used to monitor the efficacy of a particular treatment after the initial diagnosis. It is further understood that in some cases, molecular profiling may be used in place of, rather than in addition to, established methods of cancer diagnosis.

**(v) Monitoring of Subjects or Therapeutic Interventions via Molecular Profiling**

In some embodiments, a subject may be monitored using methods of the present invention. For example, a subject may be diagnosed with cancer or a genetic disorder. This initial diagnosis may or may not involve the use of molecular profiling. The subject may be prescribed a therapeutic intervention such as a thyroidectomy for a subject suspected of having thyroid cancer. The results of the therapeutic intervention may be monitored on an ongoing basis by molecular profiling to detect the efficacy of the therapeutic intervention. In another example, a subject may be diagnosed with a benign tumor or a precancerous lesion or nodule, and the tumor, nodule, or lesion may be monitored on an ongoing basis by molecular profiling to detect any changes in the state of the tumor or lesion.

Molecular profiling may also be used to ascertain the potential efficacy of a specific therapeutic intervention prior to administering to a subject. For example, a subject may be diagnosed with cancer. Molecular profiling may indicate the upregulation of a gene expression product known to be involved in cancer malignancy, such as for example the RAS oncogene. A tumor sample may be obtained and cultured *in vitro* using methods known to the art. The application of various inhibitors of the aberrantly activated or dysregulated pathway, or drugs known to inhibit the activity of the pathway may then be tested against the tumor cell line for growth inhibition. Molecular profiling may also be used to monitor the effect of these inhibitors on for example down-stream targets of the implicated pathway.

**(vi) Molecular Profiling as a Research Tool**

In some embodiments, molecular profiling may be used as a research tool to identify new markers for diagnosis of suspected tumors; to monitor the effect of drugs or candidate drugs on biological samples such as tumor cells, cell lines, tissues, or organisms; or to uncover new pathways for oncogenesis and/or tumor suppression.

**(vii) Biomarker groupings based on molecular profiling**

The current invention provides groupings or panels of biomarkers that may be used to characterize, rule in, rule out, identify, and/or diagnose pathology within the thyroid. Such biomarker panels are obtained from correlations between patterns of gene (or biomarker) expression levels and specific types of samples (e.g., malignant subtypes, benign subtypes, normal tissue, or samples with foreign tissue). The panels of biomarkers may also be used to characterize, rule in, rule out, identify, and/or diagnose benign conditions of the thyroid. In some cases, the number of panels of biomarkers is greater than 1,2, 3, 4, 5, 6, 7, 8, 9,10, 11, 12, 13, 14, 15, 16, 17, 18, 19, 20, 25, 30, 40, 50, 60, 70, 80, 90, or 100 panels of biomarkers. In preferred embodiments, the number of panels of biomarkers is greater than 12 panels, (e.g., 16 panels of biomarkers). Examples of sixteen panels of biomarkers include, but are not limited to the following (they are also provided in Figure 2):

1    Normal Thyroid (NML)

2    Lymphocytic, Autoimmune Thyroiditis (LCT)

3    Nodular Hyperplasia (NHP)

4    Follicular Thyroid Adenoma (FA)

5    Hurthle Cell Thyroid Adenoma (HC)

6    Parathyroid (non thyroid tissue)

7    Anaplastic Thyroid Carcinoma (ATC)

8    Follicular Thyroid Carcinoma (FC)

9    Hurthle Cell Thyroid Carcinoma (HC)

10    Papillary Thyroid Carcinoma (PTC)

11    Follicular Variant of Papillary Carcinoma (FVPTC)

12    Medullary Thyroid Carcinoma (MTC)

13    Renal Carcinoma metastasis to the Thyroid (RCC)

14    Melanoma metastasis to the Thyroid (MMN)

15    B cell Lymphoma metastasis to the Thyroid (BCL)

16    Breast Carcinoma metastasis to the Thyroid (BCA)

Each panel includes a set of biomarkers (e.g. gene expression products or alternatively spliced exons associated with the particular cell type) that can be used to characterize, rule in, rule out, and/or diagnose a given pathology (or lack thereof) within the thyroid. Biomarkers may be associated with more than one cell type. Panels 1-6 describe benign pathology, while panels 7-16 describe malignant pathology. These multiple panels can be combined (each in different proportion) to create optimized panels that are useful in a two-class classification system (e.g., benign versus malignant). Alternatively, biomarker panels may be used alone or in any combination as a reference or classifier in the classification, identification, or diagnosis of a thyroid tissue sample as comprising one or more tissues selected from NML, FA, NHP, LCT, HA, FC, PTC, FVPTC, MTC, HC, ATC, RCC, BCA, MMN, BCL, and PTA. Combinations of biomarker panels may contain at least about 1, 2, 3, 4, 5, 6, 7, 8, 9, 10, 11, 12, 13, 14, 15, 16, or more biomarker panels. In some embodiments, where two are more panels are used in the classification, identification, or diagnosis, the comparison is sequential. Sequential comparison may comprise 1, 2, 3, 4, 5, 6, 7, 8, 9, 10, or more sets comprising 2, 3, 4, 5, 6, 7, 8, 9, 10, or more biomarker panels that are compared simultaneously as a step in the sequential comparison, each set comprising at least one different biomarker panel than compared at other steps in the sequence (and may optionally be completely non-overlapping).

The biological nature of the thyroid and each pathology found within it suggest there may be some redundancy between the plurality of biomarkers in one panel versus the plurality of biomarkers in another panel. In some embodiments, for each pathology subtype, each diagnostic panel is heterogeneous and semi-redundant, or not redundant, with the biomarkers in another panel. In general, heterogeneity and redundancy reflect the biology of the tissues samples in a given thyroid sample (e.g. surgical or FNA sample) and the differences in gene expression that differentiates each pathology subtype from one another.

In one aspect, the diagnostic value of the present disclosure lies in the comparison of i) one or more markers in one panel, versus ii) one or more markers in each additional panel.

The pattern of gene expression demonstrated by a particular biomarker panel reflects the "signature" of each panel. For example, the panel of Lymphocytic Autoimmune Thyroiditis (LCT) may have certain sets of biomarkers that display a particular pattern or signature. Within such signature, specific biomarkers may be upregulated, others may be not be differentially expressed, and still others may be down regulated. The signatures of particular panels of biomarkers may themselves be grouped in order to diagnose or otherwise characterize a thyroid condition; such groupings may be referred to as "classification panels". Each classification panel may comprise at least 1, 2, 3, 4, 5, 6, 7, 8, 9, 10, 15, or more than 20 biomarker panels.

Classification panels may contain specified biomarkers (TCIDs) and use information saved during algorithm training to rule in, or rule out a given sample as "benign," "suspicious," or as comprising or not comprising one or more tissue types (e.g. NML, FA, NHP, LCT, HA, FC, PTC, FVPTC, MTC, HC, ATC, RCC, BCA, MMN, BCL, and PTA). Each classification panel may use simple decision rules to filter incoming samples, effectively removing any flagged samples from subsequent evaluation if the decision rules are met (e.g. a sample is characterized regarding the identity or status of one or more tissue types contained therein). The biomarker panels and classification panels provided herein are specifically useful for classifying, characterizing, identifying, and/or diagnosing thyroid cancer or other thyroid condition (including diagnosing the thyroid as normal). However, biomarker panels and classification panels similar to the present panels may be obtained using similar methods and can be used for other diseases or disorders, such as other diseases or disorder described herein.

Figure 3 provides an example of a set of classification panels that can be used to diagnose a thyroid condition. For example, as shown in Figure 3, one classification panel can contain a single biomarker panel such as the MTC biomarker panel (e.g., classification panel #1); another classification panel can contain a single biomarker panel such as the RCC biomarker panel (e.g., classification panel #2); yet another classification panel can contain a single biomarker panel such as the PTA biomarker panel (e.g., classification panel #3); yet another classification panel can contain a single biomarker panel such as the BCA biomarker panel (e.g., classification panel #4); yet another classification panel can contain a single biomarker panel such as the MMN biomarker panel (e.g., classification panel #5); yet another classification panel can contain a two biomarker panels such as the HA and HC biomarker panels (e.g., classification panel 6); and yet another classification panel can contain a combination of the FA, FC, NHP, PTC, FVPTC, HA, HC, and LCT panels (e.g., classification panel #7, which is also an example of a "main" classifier). One or more such classifiers may be used simultaneously or in sequence, and in any combination, to classify, characterize, identify, or diagnose a thyroid sample. In some embodiments, a sample is identified as containing or not containing tissue having an HA or HC tissue type.

Other potential classification panels that may be useful for characterizing, identifying, and/or diagnosing thyroid cancers may include: 1) biomarkers of metastasis to the thyroid from non-thyroid organs (e.g., one of or any combination of two or more of the following: RCC, MTC, MMN, BCL, and BCA panels); 2) 1) biomarkers correlated with thyroid tissue that originated from non-thyroid organs (e.g., any one of or any combination of two or more of the following: RCC, MTC, MMN, BCL, BCA, and PTA panels); 3) biomarkers with significant changes in alternative gene splicing, 4) KEGG Pathways, 5) gene ontology; 6) biomarker panels associated with thyroid cancer (e.g., one of or groups of two or more of the following panels: FC, PTC, FVPTC, MTC, HC, and ATC); 7) biomarker panels associated with benign thyroid conditions (e.g., one of or groups of two or more of the following: FA, NHP, LCT, or HA); 8) biomarker panels associated with benign thyroid conditions or normal thyroid tissue (e.g., one of or groups of two or more of the following: FA, NHP, LCT, HA or NML); 9) biomarkers related to signaling pathways such as adherens pathway, focal adhesion pathway, and tight junction pathway, or other pathway described in International Application No. PCT/US2009/006162, filed 11/17/2009. In addition, biomarkers that indicate metastasis to the thyroid from a non-thyroid organ may be used in the subject methods and compositions. Metastatic cancers that metastasize to thyroid that can be used for a classifier to diagnose a thyroid condition include but are not limited to: metastatic parathyroid cancer, metastatic melanoma, metastatic renal carcinoma, metastatic breast carcinoma, and metastatic B cell lymphoma.

In some cases, the method provides a number, or a range of numbers, of biomarkers (including gene expression products) that are used to diagnose or otherwise characterize a biological sample. As described herein, such biomarkers can be identified using the methods provided herein, particularly the methods of correlating gene expression signatures with specific types of tissue, such as the types listed in Figure 2. The sets of biomarkers indicated in Figure 4, can be obtained using the methods described herein. Said biomarkers can also be used, in turn, to classify tissue. In some cases, all of the biomarkers in Figure 4 are used to diagnose or otherwise characterize thyroid tissue. In some cases, a subset of the biomarkers in Figure 4 are used to diagnose or otherwise characterize thyroid tissue. In some cases, all, or a subset, of the biomarkers in Figure 4, along with additional biomarkers, are used to diagnose or otherwise characterize thyroid tissue. In some embodiments, at least 1, 2, 3, 4, 5, 6, 7, 8, 9, 10, 15, 20, 25, 30, 33, 35, 38, 40, 43, 45, 48, 50, 53, 58, 63, 65, 68, 100, 120, 140, 142, 145, 147, 150, 152, 157, 160, 162, 167, 175, 180, 185, 190, 195, 200, or 300 total biomarkers are used to diagnose or otherwise characterize thyroid tissue. In other embodiments, at most 2, 3, 4, 5, 6, 7, 8, 9, 10, 15, 20, 25, 30, 33, 35, 38, 40, 43, 45, 48, 50, 53, 58, 63, 65, 68, 100, 120, 140, 142, 145, 147, 150, 152, 157, 160, 162, 167, 175, 180, 185, 190, 195, 200, or 300 total biomarkers are used to diagnose or otherwise characterize thyroid tissue. In still other embodiments, at least 1, 2, 3, 4, 5, 6, 7, 8, 9, 10, 15, 20, 25, 30, 33, 35, 38, 40, 43, 45, 48, 50, 53, 58, 63, 65, 68, 100, 120, 140, 142, 145, 147, 150, 152, 157, 160, 162, 167, 175, 180, 185, 190, or more of the biomarkers identified in Figure 4 are used to diagnose or otherwise characterize thyroid tissue.

Exemplary biomarkers and an example of their associated classification panel (and/or biomarker panel) are listed in Figure 4. The methods and compositions provided herein may use any or all of the biomarkers listed in Figure 4. In some embodiments, the biomarkers listed in Figure 4 are used as part of the corresponding classification panel indicated in Figure 4. In other cases, the biomarkers in Figure 4 may be used for a different classification panel than the one indicated in Figure 4.

Optimized classification panels may be assigned specific numbers of biomarkers per classification panel. For example, an optimized classification panel may be assigned at least 1, 2, 3, 4, 5, 6, 7, 8, 9, 10, 15, 20, 25, 30, 35, 40, 45 or 50, 100, 120, 140, 142, 145, 160, 180, or over 200 biomarkers. For example, as shown in Figure 3, a classification panel may contain 5, 33, or 142 biomarkers. Methods of the invention can use biomarkers selected from 1, 2, 3, 4, 5, 6, 7, 8, 9, 10, 11, 12, 13, 14, 15 or 16 or more biomarker panels and each of these biomarker panels may have more than 1, 2, 3, 4, 5, 6, 7, 8, 9, 10, 15, 20, 25, 30, 35, 40, 45, 50 or more biomarkers, in any combination. In some embodiments, the set of markers combined give a specificity or sensitivity of greater than 60%, 70%, 75%, 80%, 85%, 86%, 87%, 88%, 89%, 90%, 91%, 92%, 93%, 94%, 95%, 96%, 97%, 98%, 99%, or 99.5%, or a positive predictive value or negative predictive value of at least 90%, 95%, 95.5%, 96%, 96.5%, 97%, 97.5%, 98%, 98.5%, 99%, 99.5% or more.

Analysis of the gene expression levels may involve sequential application of different classifiers described herein to the gene expression data. Such sequential analysis may involve applying a classifier obtained from gene expression analysis of cohorts of diseased thyroid tissue, followed by applying a classifier obtained from analysis of a mixture of different samples of thyroid tissue, with some of the samples containing diseased thyroid tissues and others containing benign thyroid tissue. In preferred embodiments, the diseased tissue is malignant or cancerous tissue (including tissue that has metastasized from a non-thyroid organ). In more preferred embodiments, the diseased tissue is thyroid cancer or a non-thyroid cancer that has metastasized to the thyroid. In some embodiments, the classifier is obtained from analysis of gene expression patterns in benign tissue, normal tissue, and/or non-thyroid tissue (e.g., parathyroid tissue). In some embodiments, the diseased tissue is HA and/or HC tissue.

In some embodiments, the classification process begins when each classification panel receives as input biomarker expression levels (e.g., summarized microarray intensity values, qPCR, or sequencing data) from a patient sample. The biomarkers and expression levels specified in a classification panel are then evaluated. If the data from a given sample matches the rules specified within the classification panel (or otherwise correlate with the signature of the classification panel), then its data output flags the sample and prevents it from further evaluation and scoring by the main (downstream) classifier. When a classification panel flags a sample, the system automatically returns a "suspicious" call for that sample. When a classification panel does not flag a sample, the evaluation continues downstream to the next classification panel and it may be flagged or not flagged. In some situations, the classification panels are applied in a specific order; in other cases, the order of the applications can be any order. In some embodiments, classification panels 1-5 from Figure 3 in the optimized list of thyroid gene signature panels are executed in any particular order, but then are followed by classification panel 6, which then precedes application of the main classifier (e.g. classification panel 7).

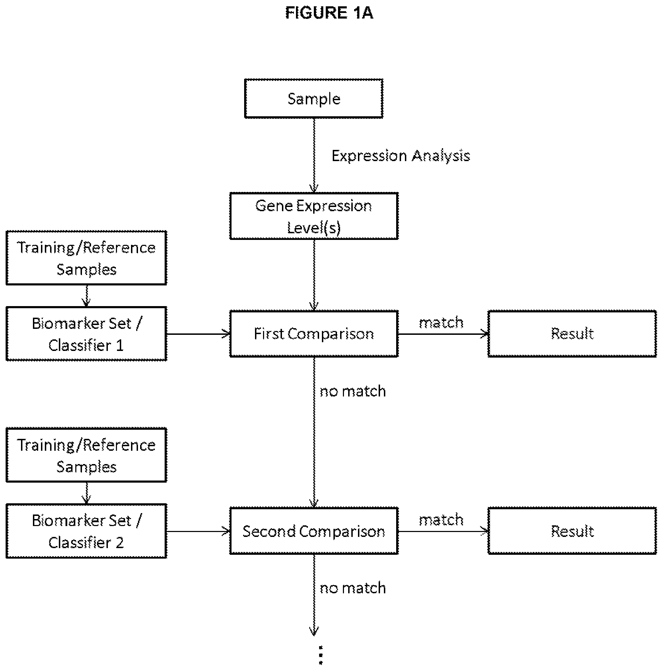

An example illustration of a classification process in accordance with the methods of the invention is provided in Figure 1A. The process begins with determining, such as by gene expression analysis, expression level(s) for one or more gene expression products from a sample (e.g. a thyroid tissue sample) from a subject. Separately, one or more sets of reference or training samples may be analyzed to determine gene expression data for at least two different sets of biomarkers, the gene expression data for each biomarker set comprising one or more gene expression levels correlated with the presence of one or more tissue types. The gene expression data for a first set of biomarkers may be used to train a first classifier; gene expression data for a second set may be used to train a second classifier; and so on for 2, 3, 4, 5, 6, 7, 8, 9, 10, 11, 12, 13, 14, 15, 16, or more sets of biomarkers and optionally corresponding classifiers. The sets of reference or training samples used in the analysis of each of the sets of biomarkers may be overlapping or non-overlapping. In some embodiments, the reference or training samples comprise HA and/or HC tissue. In the next step of the example classification process, a first comparison is made between the gene expression level(s) of the sample and the first set of biomarkers or first classifier. If the result of this first comparison is a match, the classification process ends with a result, such as designating the sample as suspicious, cancerous, or containing a particular tissue type (e.g. HA or HC). If the result of the comparison is not a match, the gene expression level(s) of the sample are compared in a second round of comparison to a second set of biomarkers or second classifier. If the result of this second comparison is a match, the classification process ends with a result, such as designating the sample as suspicious, cancerous, or containing a particular tissue type (e.g. HA or HC). If the result of the comparison is not a match, the process continues in a similar stepwise process of comparisons until a match is found, or until all sets of biomarkers or classifiers included in the classification process are used as a basis of comparison. If no match is found between the gene expression level(s) of the sample and any set of biomarkers or classifiers utilized in the classification process, the sample may be designated as "benign." In some embodiments, the final comparison in the classification process is between the gene expression level(s) of the sample and a main classifier, as described herein.

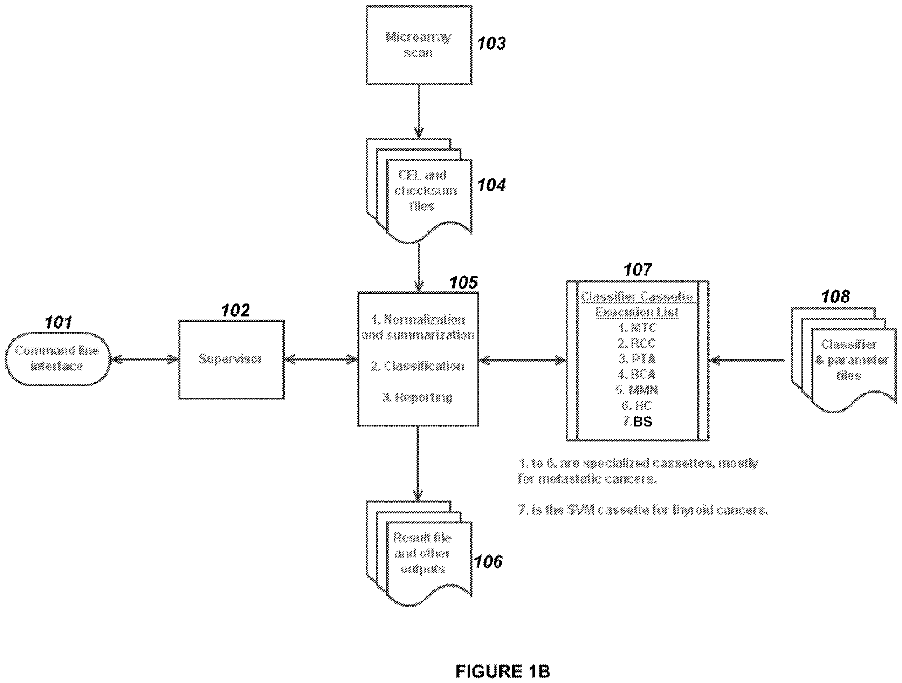

A further example of a classification process in accordance with the methods of the invention is illustrated in Figure 1B. Gene expression analysis is performed by microarray hybridization. Scanning of the microarray **103** produces gene expression data **104** in the form of CEL files (the data) and checksum files (for verification of data integrity). Separately, gene expression data for training samples are analyzed to produce classifier and parameter files **108** comprising gene expression data correlated with the presence of one or more tissue types. Classifier cassettes are compiled into an ordered execution list **107**. Analysis of sample data using the classifier cassettes is initiated with input of commands using a command line interface **101**, the execution of which commands are coordinated by a supervisor **102**. The classification analysis in this example process is further detailed at **105** and **107**. Gene expression data **104** is normalized and summarized, and subsequently analyzed with each classifier cassette in sequence for the cassettes in the execution list **105**. In this example, gene expression data is classified using classification cassettes comprising biomarker expression data correlated with medullary thyroid carcinoma (MTC), followed in sequence by comparison using classifier cassettes for renal carcinoma metastasis to the thyroid (RCC), parathyroid (PTA), breast carcinoma metastasis to the thyroid (BCA), melanoma metastasis to the thyroid (MMN), Hurthle cell carcinoma and/or Hurthle cell adenoma (HC), and concluding with a main classifier to distinguish benign from suspicious tissue samples (BS). The result of sequentially analyzing the gene expression data with each classifier cassette is then reported in a result file and any other report information or output **106**.

In some embodiments, the classification process uses a main classifier (e.g., classification panel 7) to designate a sample as "benign" or "suspicious," or as containing or not containing one or more tissues of a particular type (e.g. HA or HC). In some embodiments, gene expression data obtained from a sample undergoes a series of "filtering" steps, where the data is sequentially run through different classification panels or biomarker panels. For example, the sample may be analyzed with the MMN biomarker panel followed by the MTC biomarker panel. In some cases, the sequence of classification panels is classification panels 1 through 5 in any order, followed by classification panel 6, followed by the main classifier (as shown in Figure 3). In some cases, one classification panel is used followed by the main classifier. In some cases, 1, 2, 3, 4, 5, 6, 7, 8, 9, or 10 classifier panels are used followed by the main classifier. In some cases, classifier 6 (HA and HC combined) is used directly before the main classifier. In some cases, one or more of the classifiers 1 through 5 are applied, in any combination, followed by classifier 7. In some cases, one or more of the classifiers 1 through 5 are applied, in any combination or sequence, followed by application of classifier 6, followed by application of classifier 7. In some cases, one or more of the classifiers 1 through 6 are applied, in any combination or sequence, followed by application of classifier 7 (or other main classifier).

In some embodiments, the biomarkers within each panel are interchangeable (modular). The plurality of biomarkers in all panels can be substituted, increased, reduced, or improved to accommodate the definition of new pathologic subtypes (e.g. new case reports of metastasis to the thyroid from other organs). The current disclosure describes a plurality of biomarkers that define each of sixteen heterogeneous, semi-redundant, and distinct pathologies found in the thyroid. Such biomarkers may allow separation between malignant and benign representatives of the sixteen heterogeneous thyroid pathologies. In some cases, all sixteen panels are required to arrive at an accurate diagnosis, and any given panel alone does not have sufficient power to make a true characterization, classification, identification, or diagnostic determination. In other cases, only a subset of the panels is required to arrive at an accurate characterization, classification, identification, or diagnostic determination, such as less than 2, 3, 4, 5, 6, 7, 8, 9, 10, 11, 12, 13, 14, 15, or 16 of the biomarker panels. In some embodiments, the biomarkers in each panel are interchanged with a suitable combination of biomarkers, such that the plurality of biomarkers in each panel still defines a given pathology subtype within the context of examining the plurality of biomarkers that define all other pathology subtypes.

Classifiers used early in a sequential analysis may be used to either rule-in or rule-out a sample as benign or

suspicious, or as containing or not containing one or more tissues of a particular type (e.g. HA or HC). In some embodiments, such sequential analysis ends with the application of a "main" classifier to data from samples that have not been ruled out by the preceding classifiers, wherein the main classifier is obtained from data analysis of gene expression levels in multiple types of tissue and wherein the main classifier is capable of designating the sample as benign or suspicious (or malignant), or as containing or not containing one or more tissues of a particular type (e.g. HA or HC).

Provided herein are sixteen thyroid biomarker panels. In some embodiments, two or more biomarker panels associated with tissue types selected from NML, FA, NHP, LCT, HA, FC, PTC, FVPTC, MTC, HC, ATC, RCC, BCA, MMN, BCL, and PTA tissue types are used to distinguish i) benign FNA thyroid samples from malignant (or suspicious) FNA thyroid samples, ii) the presence of from the absence of one or more of NML, FA, NHP, LCT, HA, FC, PTC, FVPTC, MTC, HC, ATC, RCC, BCA, MMN, BCL, and PTA tissue types in a sample, and/or iii) the presence of HA and/or HC tissue from the absence of HA and/or HC tissue in a sample. The benign versus malignant characterization may be more accurate after examination and analysis of the differential gene expression that defines each pathology subtype in the context of all other subtypes. In one embodiments, the current disclosure describes a plurality of markers that are useful in accurate classification of thyroid FNA.

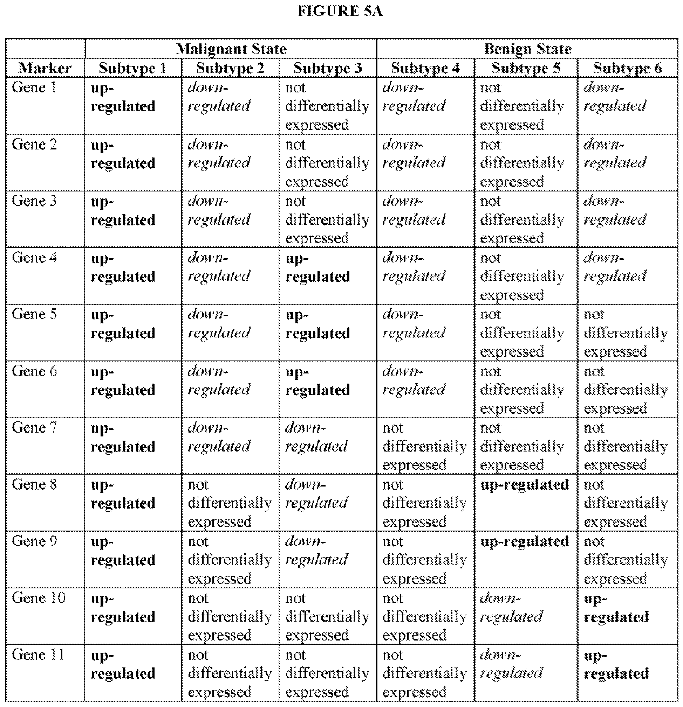

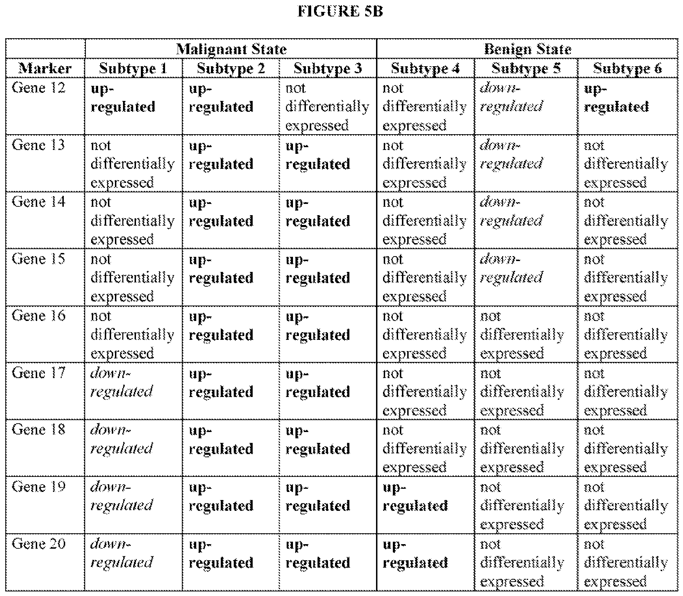

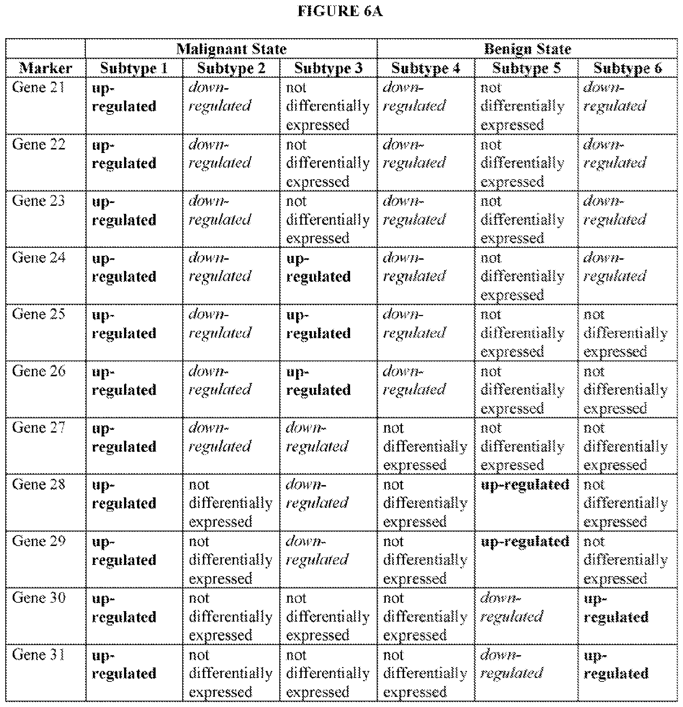

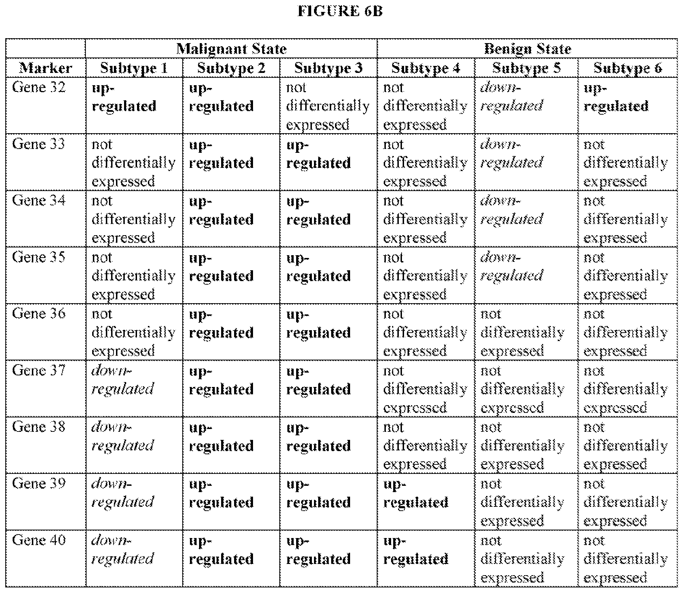

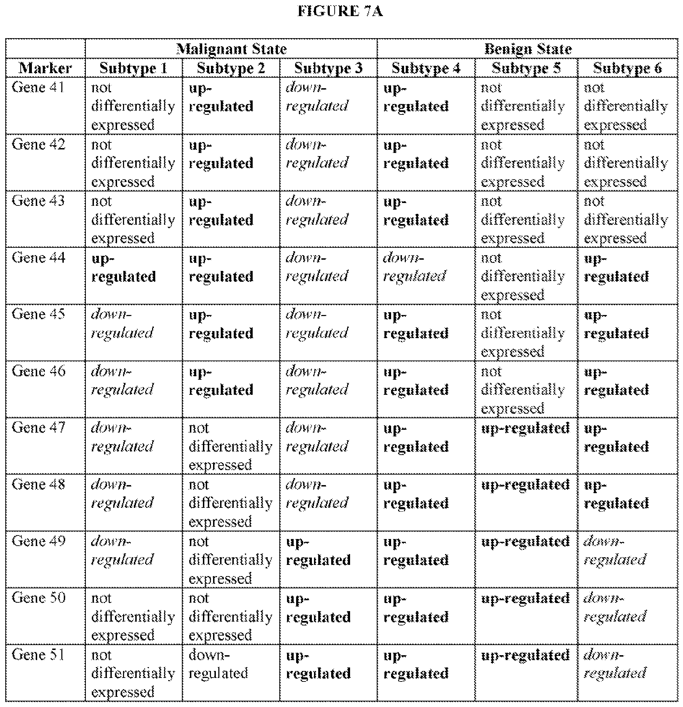

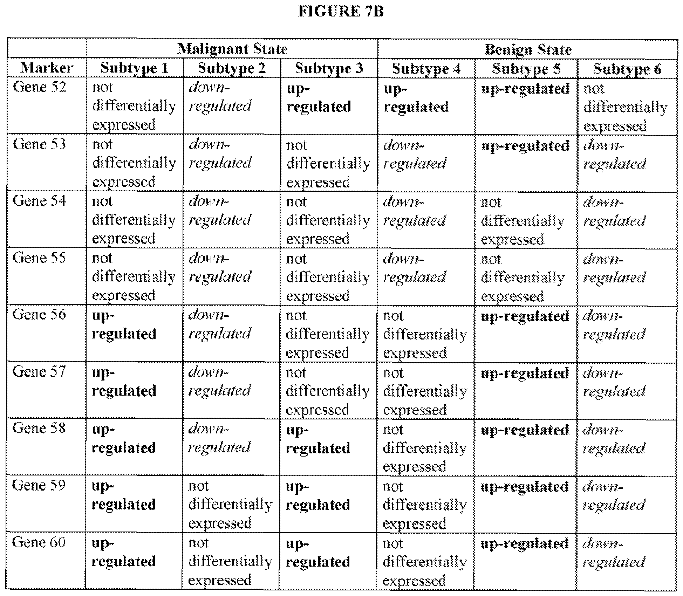

Classification optimization and simultaneous and/or sequential examination of the initial sixteen biomarker panels described in Figure 2 can be used to select a set of 2, 3, 4, 5, 6, 7, 8, 9, 10 or more (e.g. seven classification panels in Figure 3), which optimization may include a specified order of sequential comparison using such classification panels. A person skilled in the art may study a cohort of thyroid surgical tissue and/or FNA samples and use the novel methods described herein to generate similar biomarker panels, that are completely or partially distinct from those described herein. Hence, it is the subtype panels themselves that have utility and not necessarily the actual genes found within these. A person skilled in the art may also use the methods described herein to design multiple, mutually exclusive panels per subtype (e.g. Figure 6), where each of the genes in a panel is substituted with genes whose expression have a similar trend to those in Figure 3. Similarly, a person skilled in the art may design multiple, novel panels per subtype (also a distinct modular series, e.g. Figure 7), where each of the genes in a panel has distinct gene expression signatures than the genes shown in Figure 5). Each modular series of subtype panels may be mutually exclusive and sufficient to arrive at accurate thyroid FNA classification.

Examples of biomarkers with diagnostic utility for the evaluation of thyroid FNA are shown in Figure 4. Unlike differential gene expression analysis (e.g. malignant vs benign), it may not be necessary for biomarkers to reach statistical significance in the benign versus malignant comparison in order to be useful in a panel for accurate classification. In some embodiments, the benign versus malignant (or benign versus suspicious) comparison is not statistically significant. In some embodiments, the benign versus malignant (or benign versus suspicious) comparison is statistically significant. In some embodiments, a comparison or correlation of a specific subtype is not statistically significant. In some embodiments, a comparison or correlation of a specific subtype is statistically significant.

The sixteen panels described in Figure 2 represent distinct pathologies found in the thyroid (whether of thyroid origin or not). However, subtype prevalence in a given population is not necessarily uniform. For example, NHP and PTC are far more common than rare subtypes such as FC or ATC. In some embodiments, the relative frequency of biomarkers in each subtype panel is subsequently adjusted to give the molecular test sufficient sensitivity and specificity.

The biomarker groupings provided herein are examples of biomarker groupings that can be used for thyroid conditions. However, biomarker groupings can be used for other diseases or disorders as well, e.g., any disease or disorder described herein.

### (viii) Classification Error Rates

In some embodiments, top thyroid biomarkers are subdivided into bins (50 TCIDs per bin) to demonstrate the minimum number of genes required to achieve an overall classification error rate of less than 4%. The original TCIDs used for classification correspond to the Affymetrix Human Exon 1.OST microarray chip and each may map to more than one gene or no genes at all (Affymetrix annotation file: HuEx-1_0-st-v2.na29.hg18.transcript.csv). When no genes map to a TCID the biomarker is denoted as TCID-######.

### IX. Compositions

### (i) Gene Expression Products and splice variants of the present invention

Molecular profiling may also include but is not limited to assays of the present disclosure including assays for one or more of the following: proteins, protein expression products, DNA, DNA polymorphisms, RNA, RNA expression products, RNA expression product levels, or RNA expression product splice variants of the genes or markers provided in Figure 4. In some cases, the methods of the present invention provide for improved cancer diagnostics by molecular profiling of at least about 1, 2, 3, 4, 5, 6, 7, 8, 9, 10, 15, 20, 25, 30, 35, 40, 45, 50, 60, 70, 80, 90, 100, 120, 140, 160, 180, 200, 240, 280, 300, 350, 400, 450, 500, 600, 700, 800, 1000, 1500, 2000, 2500, 3000, 3500, 4000, 5000 or more DNA polymorphisms, expression product markers, and/or alternative splice variant markers.

In one embodiment, molecular profiling involves microarray hybridization that is performed to determine gene expression product levels for one or more genes selected from Figure 4. In some cases, gene expression product levels of one or more genes from one group are compared to gene expression product levels of one or more genes in another group or groups. As an example only and without limitation, the expression level of gene TPO may be compared to the expression level of gene GAPDH. In another embodiment, gene expression levels are determined for one or more genes involved in one or more of the following metabolic or signaling pathways: thyroid hormone production and/or release, protein kinase signaling pathways, lipid kinase signaling pathways, and cyclins. In some cases, the methods of the present invention provide for analysis of gene expression product levels and or alternative exon usage of at least one gene of 1, 2, 3, 4, 5, 6, 7, 9, 10, 11, 12, 13, 14, or 15 or more different metabolic or signaling pathways.

### (ii) Compositions of the present disclosure

Compositions of the present disclosure are also provided which composition comprises one or more of the following: nucleotides (e.g. DNA or RNA) corresponding to the genes or a portion of the genes provided in Figure 4, and nucleotides (e.g. DNA or RNA) corresponding to the complement of the genes or a portion of the complement of the genes provided in Figure 4. In some embodiments, this disclosure provides for collections of probes, such as sets of probes that can bind to at least1, 2, 3, 4, 5, 6, 7, 8, 9, 10, 15, 20, 25, 30, 35, 40, 45 or 50, 100, 120, 140, or 160 of the biomarkers identified in Figure 4.

The nucleotides (including probes) of the present invention can be at least about 10, 15, 20, 25, 30, 35, 40, 45, 50, 55, 60, 65, 70, 75, 100, 150, 200, 250, 300, 350, or about 400 or 500 nucleotides in length. In some embodiments of the present invention, the nucleotides can be natural or man-made derivatives of ribonucleic acid or deoxyribonucleic acid including but not limited to peptide nucleic acids, pyranosyl RNA, nucleosides, methylated nucleic acid, pegylated nucleic acid, cyclic nucleotides, and chemically modified nucleotides. In some of the compositions of the present disclosure, nucleotides of the present disclosure have been chemically modified to include a detectable label. In some embodiments of the present invention the biological sample has been chemically modified to include a label.

A further composition of the present disclosure comprises oligonucleotides for detecting (i.e. measuring) the expression products of the genes provided in Figure 4 and/or their complement. A further composition of the present disclosure comprises oligonucleotides for detecting (i.e. measuring) the expression products of polymorphic alleles of the genes provided in Figures 5-8 and their complement. Such polymorphic alleles include but are not limited to splice site variants, single nucleotide polymorphisms, variable number repeat polymorphisms, insertions, deletions, and homologues. In some cases, the variant alleles are between about 99.9% and about 70% identical to the genes listed in Figure 4, including about, less than about, or more than about 99.75%, 99.5%, 99.25%, 99%, 97.5%, 95%, 92.5%, 90%, 85%, 80%, 75%, and about 70% identical. In some cases, the variant alleles differ by between about 1 nucleotide and about 500 nucleotides from the genes provided in Figure 4, including about, less than about, or more than about 1, 2, 3, 5, 7, 10, 15, 20, 25, 30, 35, 50, 75, 100, 150, 200, 250, 300, and about 400 nucleotides.

In some embodiments, the composition of the present invention may be specifically selected from the top differentially expressed gene products between benign and malignant samples (or between presence and absence of one or more particular tissue types, such as HA and/or HC), or the top differentially spliced gene products between benign and malignant samples(or between presence and absence of one or more particular tissue types, such as HA and/or HC), or the top differentially expressed gene products between normal and benign or malignant samples(or between presence and absence of one or more particular tissue types, such as HA and/or HC), or the top differentially spliced gene products between normal and benign or malignant samples(or between presence and absence of one or more particular tissue types, such as HA and/or HC). In some cases the top differentially expressed gene products may be selected from Figure 4.

### Diseases and Disorders

In some embodiments, the subject methods and algorithm are used to diagnose, characterize, detect, exclude and/or monitor thyroid cancer. Thyroid cancer includes any type of thyroid cancer, including but not limited to, any malignancy of the thyroid gland, e.g., papillary thyroid cancer, follicular thyroid cancer, medullary thyroid cancer and/or anaplastic thyroid cancer. In some cases, the thyroid cancer is differentiated. In some cases, the thyroid cancer is undifferentiated. In some cases, the instant methods are used to diagnose, characterize, detect, exclude and/or monitor one or more of the following types of thyroid cancer: papillary thyroid carcinoma (PTC), follicular variant of papillary thyroid carcinoma (FVPTC), follicular carcinoma (FC), Hurthle cell carcinoma (HC) or medullary thyroid carcinoma (MTC).

Other types of cancer that can be diagnosed, characterized and/or monitored using the algorithms and methods of the present disclosure include but are not limited to adrenal cortical cancer, anal cancer, aplastic anemia, bile duct cancer, bladder cancer, bone cancer, bone metastasis, central nervous system (CNS) cancers, peripheral nervous system (PNS) cancers, breast cancer, Castleman's disease, cervical cancer, childhood Non-Hodgkin's lymphoma, lymphoma, colon and rectum cancer, endometrial cancer, esophagus cancer, Ewing's family of tumors (e.g. Ewing's sarcoma), eye cancer, gallbladder cancer, gastrointestinal carcinoid tumors, gastrointestinal stromal tumors, gestational trophoblastic disease, hairy cell leukemia, Hodgkin's disease, Kaposi's sarcoma, kidney cancer, laryngeal and hypopharyngeal cancer, acute lymphocytic leukemia, acute myeloid leukemia, children's leukemia, chronic lymphocytic leukemia, chronic myeloid leukemia, liver cancer, lung cancer, lung carcinoid tumors, Non-Hodgkin's lymphoma, male breast cancer, malignant mesothelioma, multiple myeloma, myelodysplastic syndrome, myeloproliferative disorders, nasal cavity and paranasal cancer, nasopharyngeal cancer, neuroblastoma, oral cavity and oropharyngeal cancer, osteosarcoma, ovarian cancer, pancreatic cancer, penile cancer, pituitary tumor, prostate cancer, retinoblastoma, rhabdomyosarcoma, salivary gland cancer, sarcoma (adult soft tissue cancer), melanoma skin cancer, non-melanoma skin cancer, stomach cancer, testicular cancer, thymus cancer, uterine cancer (e.g. uterine sarcoma), vaginal cancer, vulvar cancer, and Waldenstrom's macroglobulinemia.

Expression profiling using panels of biomarkers can be used to characterize thyroid tissue as benign, suspicious, and/or malignant. Panels may be derived from analysis of gene expression levels of cohorts containing benign (non-cancerous) thyroid subtypes including follicular adenoma (FA), nodular hyperplasia (NHP), lymphocytic thyroiditis (LCT), and Hurthle cell adenoma (HA); malignant subtypes including follicular carcinoma (FC), papillary thyroid carcinoma (PTC), follicular variant of papillary carcinoma (FVPTC), medullary thyroid carcinoma (MTC), Hürthle cell carcinoma (HC), and anaplastic thyroid carcinoma (ATC). Such panels may also be derived from non-thyroid subtypes including renal carcinoma (RCC), breast carcinoma (BCA), melanoma (MMN), B cell lymphoma (BCL), and parathyroid (PTA). Biomarker panels associated with normal thyroid tissue (NML) may also be used in the methods and compositions provided herein. Exemplary panels of biomarkers are provided in Figure 2, and will be described further herein. Of note, each panel listed in Figure 2, relates to a signature, or pattern of biomarker expression (e.g., gene expression), that correlates with samples of that particular pathology or description.

The present disclosure also provides novel methods and compositions for identification of types of aberrant cellular proliferation through an iterative process (e.g., differential diagnosis) such as carcinomas including follicular carcinomas (FC), follicular variant of papillary thyroid carcinomas (FVPTC), Hurthle cell carcinomas (HC), Hurthle cell adenomas (HA); papillary thyroid carcinomas (PTC), medullary thyroid carcinomas (MTC), and anaplastic carcinomas (ATC); adenomas including follicular adenomas (FA); nodule hyperplasias (NHP); colloid nodules (CN); benign nodules (BN); follicular neoplasms (FN); lymphocytic thyroiditis (LCT), including lymphocytic autoimmune thyroiditis; parathyroid tissue; renal carcinoma metastasis to the thyroid; melanoma metastasis to the thyroid; B-cell lymphoma metastasis to the thyroid; breast carcinoma to the thyroid; benign (B) tumors, malignant (M) tumors, and normal (N) tissues. The present disclosure further provides novel gene expression markers and novel groups of genes and markers useful for the characterization, diagnosis, and/or treatment of cellular proliferation. Additionally the present disclosure provides business methods for providing enhanced diagnosis, differential diagnosis, monitoring, and treatment of cellular proliferation.

In some embodiments, the diseases or conditions classified, characterized, or diagnosed by the methods of the present invention include benign and malignant hyperproliferative disorders including but not limited to cancers, hyperplasias, or neoplasias. In some cases, the hyperproliferative disorders classified, characterized, or diagnosed by the methods of the present disclosure include but are not limited to breast cancer such as a ductal carcinoma in duct tissue in a mammary gland, medullary carcinomas, colloid carcinomas, tubular carcinomas, and inflammatory breast cancer; ovarian cancer, including epithelial ovarian tumors such as adenocarcinoma in the ovary and an adenocarcinoma that has migrated from the ovary into the abdominal cavity; uterine cancer; cervical cancer such as adenocarcinoma in the cervix epithelial including squamous cell carcinoma and adenocarcinomas; prostate cancer, such as a prostate cancer selected from the following: an adenocarcinoma or an adenocarinoma that has migrated to the bone; pancreatic cancer such as epitheliod carcinoma in the pancreatic duct tissue and an adenocarcinoma in a pancreatic duct; bladder cancer such as a transitional cell carcinoma in urinary bladder, urothelial carcinomas (transitional cell carcinomas), tumors in the urothelial cells that line the bladder, squamous cell carcinomas, adenocarcinomas, and small cell cancers; leukemia such as acute myeloid leukemia (AML), acute lymphocytic leukemia, chronic lymphocytic leukemia, chronic myeloid leukemia, hairy cell leukemia, myelodysplasia, myeloproliferative disorders, acute myelogenous leukemia (AML), chronic myelogenous leukemia (CML), mastocytosis, chronic lymphocytic leukemia (CLL), multiple myeloma (MM), and myelodysplastic syndrome (MDS); bone cancer; lung cancer such as non-small cell lung cancer (NSCLC), which is divided into squamous cell carcinomas, adenocarcinomas, and large cell undifferentiated carcinomas, and small cell lung cancer; skin cancer such as basal cell carcinoma, melanoma, squamous cell carcinoma and actinic keratosis, which is a skin condition that sometimes develops into squamous cell carcinoma; eye retinoblastoma; cutaneous or intraocular (eye) melanoma; primary liver cancer (cancer that begins in the liver); kidney cancer; AIDS-related lymphoma such as diffuse large B-cell lymphoma, B-cell immunoblastic lymphoma and small non-cleaved cell lymphoma; Kaposi's Sarcoma; viral-induced cancers including hepatitis B virus (HBV), hepatitis C virus (HCV), and hepatocellular carcinoma; human lymphotropic virus-type 1 (HTLV-1) and adult T-cell leukemia/lymphoma; and human papilloma virus (HPV) and cervical cancer; central nervous system cancers (CNS) such as primary brain tumor, which includes gliomas (astrocytoma, anaplastic astrocytoma, or glioblastoma multiforme), Oligodendroglioma, Ependymoma, Meningioma, Lymphoma, Schwannoma, and Medulloblastoma; peripheral nervous system (PNS) cancers such as acoustic neuromas and malignant peripheral nerve sheath tumor (MPNST) including neurofibromas and schwannomas, malignant fibrous cytoma, malignant fibrous histiocytoma, malignant meningioma, malignant mesothelioma, and malignant mixed Mullerian tumor; oral cavity and oropharyngeal cancer such as, hypopharyngeal cancer, laryngeal cancer, nasopharyngeal cancer, and oropharyngeal cancer; stomach cancer such as lymphomas, gastric stromal tumors, and carcinoid tumors; testicular cancer such as germ cell tumors (GCTs), which include seminomas and nonseminomas, and gonadal stromal tumors, which include Leydig cell tumors and Sertoli cell tumors; thymus cancer such as to thymomas, thymic carcinomas, Hodgkin disease, non-Hodgkin lymphomas carcinoids or carcinoid tumors; rectal cancer; and colon cancer. In some cases, the diseases or conditions classified, characterized, or diagnosed by the methods of the present invention include but are not limited to thyroid disorders such as for example benign thyroid disorders including but not limited to follicular adenomas, Hurthle cell adenomas, lymphocytic thyroiditis, and thyroid hyperplasia. In some cases, the diseases or conditions classified, characterized, or diagnosed by the methods of the present invention include but are not limited to malignant thyroid disorders such as for example follicular carcinomas, follicular variant of papillary thyroid carcinomas, medullary carcinomas, and papillary carcinomas. In some cases, the methods of the present invention provide for a classification, characterization, or diagnosis of a tissue as diseased or normal. In other cases, the methods of the present invention provide for a classification, characterization, or diagnosis of normal, benign, or malignant. In some cases, the methods of the present invention provide for a classification, characterization, or diagnosis of benign/normal, or malignant. In some cases, the methods of the present invention provide for a classification, characterization, or diagnosis of one or more of the specific diseases or conditions provided herein.

In one aspect, the present invention provides algorithms and methods that can be used for classification, characterization, or diagnosis and monitoring of a genetic disorder. A genetic disorder is an illness caused by abnormalities in genes or chromosomes. While some diseases, such as cancer, are due in part to genetic disorders, they can also be caused by environmental factors. In some embodiments, the algorithms and the methods disclosed herein are used for classification, characterization, or diagnosis and monitoring of a cancer such as thyroid cancer.

Genetic disorders can be typically grouped into two categories: single gene disorders and multifactorial and polygenic (complex) disorders. A single gene disorder is the result of a single mutated gene. There are estimated to be over 4000 human diseases caused by single gene defects. Single gene disorders can be passed on to subsequent generations in several ways. There are several types of inheriting a single gene disorder including but not limited to autosomal dominant, autosomal recessive, X-linked dominant, X-linked recessive, Y-linked and mitochondrial inheritance. Only one mutated copy of the gene will be necessary for a person to be affected by an autosomal dominant disorder. Examples of autosomal dominant type of disorder include but are not limited to Huntington's disease, Neurofibromatosis 1, Marfan Syndrome, Hereditary nonpolyposis colorectal cancer, and Hereditary multiple exostoses. In autosomal recessive disorder, two copies of the gene must be mutated for a person to be affected by an autosomal recessive disorder. Examples of this type of disorder include but are not limited to cystic fibrosis, sickle-cell disease (also partial sickle-cell disease), Tay-Sachs disease, Niemann-Pick disease, spinal muscular atrophy, and dry earwax. X-linked dominant disorders are caused by mutations in genes on the X chromosome. Only a few disorders have this inheritance pattern, with a prime example being X-linked hypophosphatemic rickets. Males and females are both affected in these disorders, with males typically being more severely affected than females. Some X-linked dominant conditions such as Rett syndrome, Incontinentia Pigmenti type 2 and Aicardi Syndrome are usually fatal in males either in utero or shortly after birth, and are therefore predominantly seen in females. X-linked recessive disorders are also caused by mutations in genes on the X chromosome. Examples of this type of disorder include but are not limited to Hemophilia A, Duchenne muscular dystrophy, red-green color blindness, muscular dystrophy and Androgenetic alopecia. Y-linked disorders are caused by mutations on the Y chromosome. Examples include but are not limited to Male Infertility and hypertrichosis pinnae. Mitochondrial inheritance, also known as maternal inheritance, applies to genes in mitochondrial DNA. An example of this type of disorder is Leber's Hereditary Optic Neuropathy.

Genetic disorders may also be complex, multifactorial or polygenic. Polygenic genetic disorders are likely associated with the effects of multiple genes in combination with lifestyle and environmental factors. Although complex disorders often cluster in families, they do not have a clear-cut pattern of inheritance. This makes it difficult to determine a person's risk of inheriting or passing on these disorders. Complex disorders are also difficult to study and treat because the specific factors that cause most of these disorders have not yet been identified. Multifactoral or polygenic disorders that can be diagnosed, characterized and/or monitored using the algorithms and methods of the present disclosure include but are not limited to heart disease, diabetes, asthma, autism, autoimmune diseases such as multiple sclerosis, cancers, ciliopathies, cleft palate, hypertension, inflammatory bowel disease, mental retardation and obesity.

Other genetic disorders that can be diagnosed, characterized and/or monitored using the algorithms and methods of the present disclosure include but are not limited to 1p36 deletion syndrome, 21-hydroxylase deficiency, 22q11.2 deletion syndrome, 47,XYY syndrome, 48, XXXX, 49, XXXXX, aceruloplasminemia, achondrogenesis, type II, achondroplasia, acute intermittent porphyria, adenylosuccinate lyase deficiency, Adrenoleukodystrophy, ALA deficiency porphyria, ALA dehydratase deficiency, Alexander disease, alkaptonuria, alpha-1 antitrypsin deficiency, Alstrom syndrome, Alzheimer's disease (type 1, 2, 3, and 4), Amelogenesis Imperfecta, amyotrophic lateral sclerosis, Amyotrophic lateral sclerosis type 2, Amyotrophic lateral sclerosis type 4, amyotrophic lateral sclerosis type 4, androgen insensitivity syndrome, Anemia, Angelman syndrome, Apert syndrome, ataxia-telangiectasia, Beare-Stevenson cutis gyrata syndrome, Benjamin syndrome, beta thalassemia, biotinidase deficiency, Birt-Hogg-Dubé syndrome, bladder cancer, Bloom syndrome, Bone diseases, breast cancer, CADASIL, Camptomelic dysplasia, Canavan disease, Cancer, Celiac Disease, CGD Chronic Granulomatous Disorder, Charcot-Marie-Tooth disease, Charcot-Marie-Tooth disease Type 1, Charcot-Marie-Tooth disease Type 4, Charcot-Marie-Tooth disease, type 2, Charcot-Marie-Tooth disease, type 4, Cockayne syndrome, Coffin-Lowry syndrome, collagenopathy, types II and XI, Colorectal Cancer, Congenital absence of the vas deferens, congenital bilateral absence of vas deferens, congenital diabetes, congenital erythropoietic porphyria, Congenital heart disease, congenital hypothyroidism, Connective tissue disease, Cowden syndrome, Cri du chat, Crohn's disease, fibrostenosing, Crouzon syndrome, Crouzonodermoskeletal syndrome, cystic fibrosis, De Grouchy Syndrome, Degenerative nerve diseases, Dent's disease, developmental disabilities, DiGeorge syndrome, Distal spinal muscular atrophy type V, Down syndrome, Dwarfism, Ehlers-Danlos syndrome, Ehlers-Danlos syndrome arthrochalasia type, Ehlers-Danlos syndrome classical type, Ehlers-Danlos syndrome dermatosparaxis type, Ehlers-Danlos syndrome kyphoscoliosis type, vascular type, erythropoietic protoporphyria, Fabry's disease, Facial injuries and disorders, factor V Leiden thrombophilia, familial adenomatous polyposis, familial dysautonomia, fanconi anemia, FG syndrome, fragile X syndrome, Friedreich ataxia, Friedreich's ataxia, G6PD deficiency, galactosemia, Gaucher's disease (type 1, 2, and 3), Genetic brain disorders, Glycine encephalopathy, Haemochromatosis type 2, Haemochromatosis type 4, Harlequin Ichthyosis, Head and brain malformations, Hearing disorders and deafness, Hearing problems in children, hemochromatosis (neonatal, type 2 and type 3), hemophilia, hepatoerythropoietic porphyria, hereditary coproporphyria, Hereditary Multiple Exostoses, hereditary neuropathy with liability to pressure palsies, hereditary nonpolyposis colorectal cancer, homocystinuria, Huntington's disease, Hutchinson Gilford Progeria Syndrome, hyperoxaluria, primary, hyperphenylalaninemia, hypochondrogenesis, hypochondroplasia, idic15, incontinentia pigmenti, Infantile Gaucher disease, infantile-onset ascending hereditary spastic paralysis, Infertility, Jackson-Weiss syndrome, Joubert syndrome, Juvenile Primary Lateral Sclerosis, Kennedy disease, Klinefelter syndrome, Kniest dysplasia, Krabbe disease, Learning disability, Lesch-Nyhan syndrome, Leukodystrophies, Li-Fraumeni syndrome, lipoprotein lipase deficiency, familial, Male genital disorders, Marfan syndrome, McCune-Albright syndrome, McLeod syndrome, Mediterranean fever, familial, MEDNIK, Menkes disease, Menkes syndrome, Metabolic disorders, methemoglobinemia beta-globin type, Methemoglobinemia congenital methaemoglobinaemia, methylmalonic acidemia, Micro syndrome, Microcephaly, Movement disorders, Mowat-Wilson syndrome, Mucopolysaccharidosis (MPS I), Muenke syndrome, Muscular dystrophy, Muscular dystrophy, Duchenne and Becker type, muscular dystrophy, Duchenne and Becker types, myotonic dystrophy, Myotonic dystrophy type 1 and type 2, Neonatal hemochromatosis, neurofibromatosis, neurofibromatosis 1, neurofibromatosis 2, Neurofibromatosis type I, neurofibromatosis type II, Neurologic diseases, Neuromuscular disorders, Niemann-Pick disease, Nonketotic hyperglycinemia, nonsyndromic deafness, Nonsyndromic deafness autosomal recessive, Noonan syndrome, osteogenesis imperfecta (type I and type III), otospondylomegaepiphyseal dysplasia, pantothenate kinase-associated neurodegeneration, Patau Syndrome (Trisomy 13), Pendred syndrome, Peutz-Jeghers syndrome, Pfeiffer syndrome, phenylketonuria, porphyria, porphyria cutanea tarda, Prader-Willi syndrome, primary pulmonary hypertension, prion disease, Progeria, propionic acidemia, protein C deficiency, protein S deficiency, pseudo-Gaucher disease, pseudoxanthoma elasticum, Retinal disorders, retinoblastoma, retinoblastoma FA - Friedreich ataxia, Rett syndrome, Rubinstein-Taybi syndrome, SADDAN, Sandhoff disease, sensory and autonomic neuropathy type III, sickle cell anemia, skeletal muscle regeneration, Skin pigmentation disorders, Smith Lemli Opitz Syndrome, Speech and communication disorders, spinal muscular atrophy, spinal-bulbar muscular atrophy, spinocerebellar ataxia, spondyloepimetaphyseal dysplasia, Strudwick type, spondyloepiphyseal dysplasia congenita, Stickler syndrome, Stickler syndrome COL2A1, Tay-Sachs disease, tetrahydrobiopterin deficiency, thanatophoric dysplasia, thiamine-responsive megaloblastic anemia with diabetes mellitus and sensorineural deafness, Thyroid disease, Tourette's Syndrome, Treacher Collins syndrome, triple X syndrome, tuberous sclerosis, Turner syndrome, Usher syndrome, variegate porphyria, von Hippel-Lindau disease, Waardenburg syndrome, Weissenbacher-Zweymüller syndrome, Wilson disease, Wolf-Hirschhorn syndrome, Xeroderma Pigmentosum, X-linked severe combined immunodeficiency, X-linked sideroblastic anemia, and X-linked spinal-bulbar muscle atrophy.

## IX. Business Methods

As described herein, the term customer or potential customer refers to individuals or entities that may utilize methods or services of a molecular profiling business (e.g. a business carrying out the methods of the present invention). Potential customers for the molecular profiling methods and services described herein include for example, patients, subjects, physicians, cytological labs, health care providers, researchers, insurance companies, government entities such as Medicaid, employers, or any other entity interested in achieving more economical or effective system for diagnosing, monitoring and treating cancer.

Such parties can utilize the molecular profiling results, for example, to selectively indicate expensive drugs or therapeutic interventions to patients likely to benefit the most from said drugs or interventions, or to identify individuals who would not benefit or may be harmed by the unnecessary use of drugs or other therapeutic interventions.

### (i) Methods of Marketing

The services of the molecular profiling business of the present disclosure may be marketed to individuals concerned about their health, physicians or other medical professionals, for example as a method of enhancing diagnosis and care; cytological labs, for example as a service for providing enhanced diagnosis to a client; health care providers, insurance companies, and government entities, for example as a method for reducing costs by eliminating unwarranted therapeutic interventions. Methods of marketing to potential clients, further includes marketing of database access for researchers and physicians seeking to find new correlations between gene expression products and diseases or conditions.

The methods of marketing may include the use of print, radio, television, or internet based advertisement to potential customers. Potential customers may be marketed to through specific media, for example, endocrinologists may be marketed to by placing advertisements in trade magazines and medical journals including but not limited to *The Journal of the American Medical Association, Physicians Practice, American Medical News, Consultant, Medical Economics, Physician's Money Digest, American Family Physician, Monthly Prescribing Reference, Physicians' Travel and Meeting Guide, Patient Care, Cortlandt Forum, Internal Medicine News, Hospital Physician, Family Practice Management, Internal Medicine World Report, Women's Health in Primary Care, Family Practice News, Physician's Weekly, Health Monitor, The Endocrinologist, Journal of Endocrinology, The Open Endocrinology Journal*, and *The Journal of Molecular Endocrinology.* Marketing may also take the form of collaborating with a medical professional to perform experiments using the methods and services of the present disclosure and in some cases publish the results or seek funding for further research. In some cases, methods of marketing may include the use of physician or medical professional databases such as, for example, the American Medical Association (AMA) database, to determine contact information.

In one embodiment methods of marketing comprises collaborating with cytological testing laboratories to offer a molecular profiling service to customers whose samples cannot be unambiguously diagnosed using routine methods.

### (ii) Methods utilizing a computer

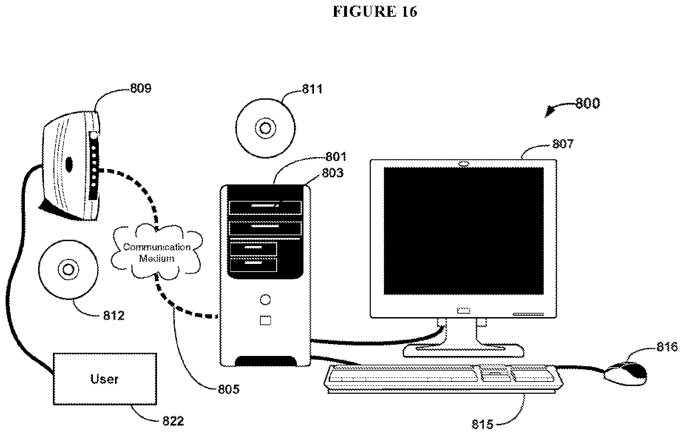

A molecular profiling business may utilize one or more computers in the methods of the present invention such as a computer **800** as illustrated in Figure16. The computer **800** may be used for managing customer and sample information such as sample or customer tracking, database management, analyzing molecular profiling data, analyzing cytological data, storing data, billing, marketing, reporting results, or storing results. The computer may include a monitor **807** or other graphical interface for displaying data, results, billing information, marketing information (e.g. demographics), customer information, or sample information. The computer may also include means for data or information input **815, 816.** The computer may include a processing unit **801** and fixed **803** or removable **811** media or a combination thereof. The computer may be accessed by a user in physical proximity to the computer, for example via a keyboard and/or mouse, or by a user **822** that does not necessarily have access to the physical computer through a communication medium **805** such as a modem, an internet connection, a telephone connection, or a wired or wireless communication signal carrier wave. In some cases, the computer may be connected to a server **809** or other communication device for relaying information from a user to the computer or from the computer to a user. In some cases, the user may store data or information obtained from the computer through a communication medium **805** on media, such as removable media **812.** It is envisioned that data relating to the present invention can be transmitted over such networks or connections for reception and/or review by a party. The receiving party can be but is not limited to an individual, a health care provider or a health care manager. In one embodiment, a computer-readable medium includes a medium suitable for transmission of a result of an analysis of a biological sample, such as a gene expression profile or other bio-signature. The medium can include a result regarding a gene expression profile or other bio-signature of a subject, wherein such a result is derived using the methods described herein.

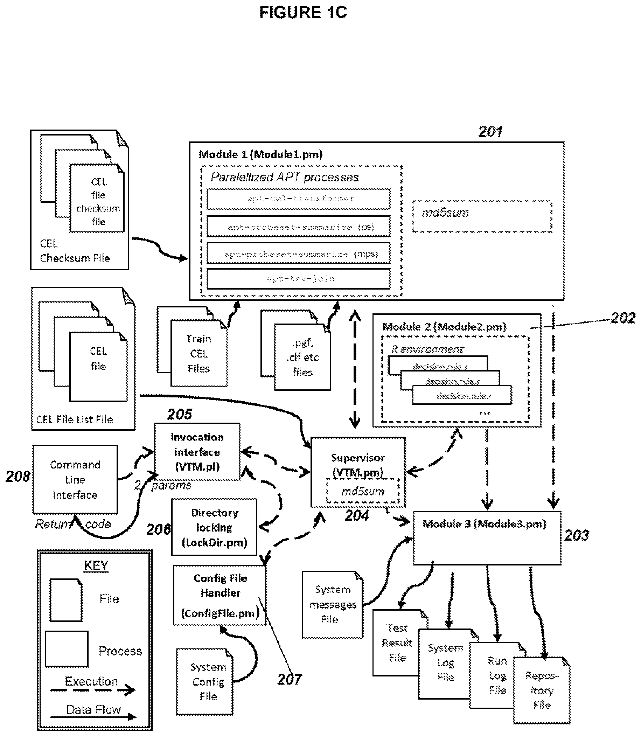

An example architecture of a system for conducting analysis according to the methods of the invention is provided in Figure 1C. This system comprises a number of components for processing, generating, storing, and outputting various files and information. In this example, the process is initiated using a command line interface **208,** commands from which are transmitted via an invocation interface **205** to a supervisor **204.** The supervisor **204** coordinates the functions of the system to carry out the analysis and comparison steps of the process. The first step in the analysis, illustrated at Module 1 **201,** includes a quality control check for the data to be analyzed by comparing the gene expression data file ("CEL" file) for a thyroid tissue sample to a corresponding checksum file. If data integrity is confirmed, Module 1 **201** progresses to normalization and summarization of the gene expression data, such as by utilizing the Affymetrix Power Tools (APT) suite of programs according to methods known in the art. The system may further comprise files needed for APT processes (e.g..pgf files, .clf files, and others). Module 1 **201** is also applied to gene expression data for training sample sets ("Train CEL Files"), which are grouped to produce classifiers comprising sets of biomarkers, with gene expression data for each set of biomarkers comprising one or more reference gene expression levels correlated with the presence of one or more tissue types. Gene expression data from Module 1 **201** is next processed by Module 2 **202,** which uses the statistical software environment "R" to compare classifiers to gene expression data for the thyroid tissue sample. Each classifier is used to establish a rule for scoring the sample gene expression data as a match or non-match. Each classifier in a set of classifiers for comparison is applied to the gene expression data one after the other. The result of the comparisons performed by Module 2 **202** are processed by Module 3 **203** to report the result by generating a "test result file," which may contain for each CEL file analyzed the name of the CEL file, a test result (e.g. benign, suspicious, or a specific tissue type), and/or a comment (e.g. classifiers used, matches found, errors encountered, or other details about the comparison process). In some embodiments, a result of "suspicious" is reported if a sample is scored as a match to any of the classifiers at any point in a sequence of comparisons. In some embodiments, a result of "benign" is reported if no match between the sample gene expression data and any of the classifiers is found. Module 3 **203** also generates system log, run log, and repository files that catalogue what happened at each step of the data handling and analysis, the output from all stages of the analysis (e.g. data integrity check and any error messages), and a table of results from each step, respectively. The log and repository files can be used for diagnosing errors in the comparison process, such as if a data analysis process fails to run through to completion and generation of a result. Module 3 **203** may reference a system messages file that contains a list of error messages. The system of this example architecture may also comprise a directory locking component **205** to prevent multiple analyses of the same CEL file at the same time, and a config file handler **207** to contain information regarding file location (e.g. executable files and CEL files) to help manage execution of the work flow of the system processes.

The molecular profiling business may enter sample information into a database for the purpose of one or more of the following: inventory tracking, assay result tracking, order tracking, customer management, customer service, billing, and sales. Sample information may include, but is not limited to: customer name, unique customer identification, customer associated medical professional, indicated assay or assays, assay results, adequacy status, indicated adequacy tests, medical history of the individual, preliminary diagnosis, suspected diagnosis, sample history, insurance provider, medical provider, third party testing center or any information suitable for storage in a database. Sample history may include but is not limited to: age of the sample, type of sample, method of acquisition, method of storage, or method of transport.

The database may be accessible by a customer, medical professional, insurance provider, third party, or any individual or entity which the molecular profiling business grants access. Database access may take the form of electronic communication such as a computer or telephone. The database may be accessed through an intermediary such as a customer service representative, business representative, consultant, independent testing center, or medical professional. The availability or degree of database access or sample information, such as assay results, may change upon payment of a fee for products and services rendered or to be rendered. The degree of database access or sample information may be restricted to comply with generally accepted or legal requirements for patient or customer confidentiality. The molecular profiling company may bill the individual, insurance provider, medical provider, or government entity for one or more of the following: sample receipt, sample storage, sample preparation, cytological testing, molecular profiling, input and update of sample information into the database, or database access.

### (iii) Business Flow

In some embodiments, samples of thyroid cells, for example, may be obtained by an endocrinologist perhaps via fine needle aspiration. Samples are subjected to routine cytological staining procedures. Said routine cytological staining provides four different possible preliminary diagnoses non-diagnostic, benign, ambiguous or suspicious, or malignant. The molecular profiling business may then analyze gene expression product levels as described herein. Said analysis of gene expression product levels, molecular profiling, may lead to a definitive diagnosis of malignant or benign. In some cases only a subset of samples are analyzed by molecular profiling such as those that provide ambiguous and non-diagnostic results during routine cytological examination.

In some cases the molecular profiling results confirms the routine cytological test results. In other cases, the molecular profiling results differ. In such cases where the results differ, samples may be further tested, data may be reexamined, or the molecular profiling results or cytological assay results may be taken as the correct classification, characterization, or diagnosis. Classification, characterization, or diagnosis as benign may also include diseases or conditions that, while not malignant cancer, may indicate further monitoring or treatment (e.g. HA). Similarly, classification, characterization, or diagnosis as malignant may further include classification, characterization, or diagnosis of the specific type of cancer (e.g. HC) or a specific metabolic or signaling pathway involved in the disease or condition. A classification, characterization, or diagnosis may indicate a treatment or therapeutic intervention such as radioactive iodine ablation, surgery, thyroidectomy, administering one or more therapeutic agents; or further monitoring.

In some embodiments, administering one or more therapeutic agent comprises administering one or more chemotherapeutic agents. In general, a "chemotherapeutic agent" refers to any agent useful in the treatment of a neoplastic condition. "Chemotherapy" means the administration of one or more chemotherapeutic drugs and/or other agents to a cancer patient by various methods, including intravenous, oral, intramuscular, intraperitoneal, intravesical, subcutaneous, transdermal, buccal, or inhalation or in the form of a suppository. In some embodiments, the chemotherapeutic is selected from the group consisting of mitotic inhibitors, alkylating agents, anti-metabolites, intercalating antibiotics, growth factor inhibitors, cell cycle inhibitors, enzymes, topoisomerase inhibitors, biological response modifiers, antihormones, angiogenesis inhibitors, and anti-androgens. Non-limiting examples are chemotherapeutic agents, cytotoxic agents, and non-peptide small molecules such as Gleevec (Imatinib Mesylate), Velcade (bortezomib), Casodex (bicalutamide), Iressa (gefitinib), and Adriamycin as well as a host of chemotherapeutic agents. Non-limiting examples of chemotherapeutic agents include alkylating agents such as thiotepa and cyclosphosphamide (CYTOXAN™); alkyl sulfonates such as busulfan, improsulfan and piposulfan; aziridines such as benzodopa, carboquone, meturedopa, and uredopa; ethylenimines and methylamelamines including altretamine, triethylenemelamine, trietylenephosphoramide, triethylenethiophosphaoramide and trimethylolomelamine; nitrogen mustards such as chlorambucil, chlomaphazine, cholophosphamide, estramustine, ifosfamide, mechlorethamine, mechlorethamine oxide hydrochloride, melphalan, novembichin, phenesterine, prednimustine, trofosfamide, uracil mustard; nitrosureas such as carmustine, chlorozotocin, fotemustine, lomustine, nimustine, ranimustine; antibiotics such as aclacinomysins, actinomycin, authramycin, azaserine, bleomycins, cactinomycin, calicheamicin, carabicin, carminomycin, carzinophilin, Casodex™, chromomycins, dactinomycin, daunorubicin, detorubicin, 6-diazo-5-oxo-L-norleucine, doxorubicin, epirubicin, esorubicin, idarubicin, marcellomycin, mitomycins, mycophenolic acid, nogalamycin, olivomycins, peplomycin, potfiromycin, puromycin, quelamycin, rodorubicin, streptonigrin, streptozocin, tubercidin, ubenimex, zinostatin, zorubicin; anti-metabolites such as methotrexate and 5-fluorouracil (5-FU); folic acid analogues such as denopterin, methotrexate, pteropterin, trimetrexate; purine analogs such as fludarabine, 6-mercaptopurine, thiamiprine, thioguanine; pyrimidine analogs such as ancitabine, azacitidine, 6-azauridine, carmofur, cytarabine, dideoxyuridine, doxifluridine, enocitabine, floxuridine, androgens such as calusterone, dromostanolone propionate, epitiostanol, mepitiostane, testolactone; anti-adrenals such as aminoglutethimide, mitotane, trilostane; folic acid replenisher such as frolinic acid; aceglatone; aldophosphamide glycoside; aminolevulinic acid; amsacrine; bestrabucil; bisantrene; edatraxate; defofamine; demecolcine; diaziquone; elfomithine; elliptinium acetate; etoglucid; gallium nitrate; hydroxyurea; lentinan; lonidamine; mitoguazone; mitoxantrone; mopidamol; nitracrine; pentostatin; phenamet; pirarubicin; podophyllinic acid; 2-ethylhydrazide; procarbazine; PSK.R™; razoxane; sizofiran; spirogermanium; tenuazonic acid; triaziquone; 2,2',2"-trichlorotriethyla-mine; urethan; vindesine; dacarbazine; mannomustine; mitobronitol; mitolactol; pipobroman; gacytosine; arabinoside ("Ara-C"); cyclophosphamide; thiotepa; taxanes, e.g. paclitaxel (TAXOL™, Bristol-Myers Squibb Oncology, Princeton, N.J.) and docetaxel (TAXOTERE™, Rhone-Poulenc Rorer, Antony, France); retinoic acid; esperamicins; capecitabine; and pharmaceutically acceptable salts, acids or derivatives of any of the above. Also included as suitable chemotherapeutic cell conditioners are anti-hormonal agents that act to regulate or inhibit hormone action on tumors such as anti-estrogens including for example tamoxifen (Nolvadex™), raloxifene, aromatase inhibiting 4(5)-imidazoles, 4-hydroxytamoxifen, trioxifene, keoxifene, LY 117018, onapristone, and toremifene (Fareston); and anti-androgens such as flutamide, nilutamide, bicalutamide, leuprolide, and goserelin; chlorambucil; gemcitabine; 6-thioguanine; mercaptopurine; methotrexate; platinum analogs such as cisplatin and carboplatin; vinblastine; platinum; etoposide (VP-16); ifosfamide; mitomycin C; mitoxantrone; vincristine; vinorelbine; navelbine; novantrone; teniposide; daunomycin; aminopterin; xeloda; ibandronate; camptothecin-11 (CPT-11); topoisomerase inhibitor RFS 2000; difluoromethylomithine (DMFO). Where desired, the compounds or pharmaceutical composition of the present disclosure can be used in combination with commonly prescribed anti-cancer drugs such as Herceptin®, Avastin®, Erbitux®, Rituxan®, Taxol®, Arimidex®, Taxotere®, and Velcade®.

## XI. Kits

The molecular profiling business may provide a kit for obtaining a suitable sample. In some embodiments, the kit comprises a container, a means for obtaining a sample, reagents for storing the sample, and instructions for use of the kit. Figure 19 depicts one embodiment of a kit **203**, comprising a container **202**, a means **200** for obtaining a sample, reagents **205** for storing the sample, and instructions **201** for use of the kit. In another embodiment, the kit further comprises reagents and materials for performing the molecular profiling analysis. In some cases, the reagents and materials include a computer program for analyzing the data generated by the molecular profiling methods. In still other cases, the kit contains a means by which the biological sample is stored and transported to a testing facility such as a molecular profiling business or a third party testing center.

The molecular profiling business may also provide a kit for performing molecular profiling. Said kit may comprise a means for extracting protein or nucleic acids including any or all necessary buffers and reagents; and, a means for analyzing levels of protein or nucleic acids including controls, and reagents. The kit may further comprise software or a license to obtain and use software for analysis of the data provided using the methods of the present invention.

## Examples

### Example 1: Classification Panels from Analysis of Clinical thyroid samples

Prospective clinical thyroid FNA samples (n=248) and post-surgical thyroid tissues (n=220) were examined with the Affymetrix Human Exon 1.0 ST microarray in order to identify genes that differ significantly in mRNA expression between benign and malignant samples.

Affymetrix software was used to extract, normalize, and summarize intensity data from roughly 6.5 million probes. Approximately 280,000 core probe sets were subsequently used in feature selection and classification. Models used included LIMMA (for feature selection), and SVM (used for classification) (Smyth 2004;). Top genes used in each classification panel were identified in several separate analyses using a combination of LIMMA and algorithms.

While the annotation and mapping of genes to transcript cluster identifiers (TCID) is constantly evolving, the nucleotide sequences in the probes and probe sets that make up a TCID do not change. Furthermore, a number of significant TCIDs do not map any known genes, yet these are equally important biomarkers in the classification of thyroid malignancy. Results are described using both the TCID and the genes currently mapped to each (Affymetrix annotation file: HuEx-1_0-st-v2.na30.hg19.transcript.csv).

**Sample cohorts used to train classifier:**

| Subtype | Simplified Classification | Post-Surgical Thyroid Tissue | Thyroid FNA |
|---|---|---|---|
| FA | Benign | 26 | 28 |
| HA | Benign | 0 | 5 |
| LCT | Benign | 40 | 27 |
| NHP | Benign | 23 | 111 |
| PTA | Benign | 5 | 0 |
| OM[1] | Malignant | 0 | 3 |
| FC | Malignant | 19 | 5 |
| HC | Malignant | 23 | 0 |
| FVPTC | Malignant | 21 | 11 |
| PTC | Malignant | 26 | 58 |
| MTC | Malignant | 23 | 0 |
| BCA | Malignant | 5 | 0 |
| MMN | Malignant | 4 | 0 |
| RCC | Malignant | 5 | 0 |
| **Total** | | **220** | **248** |

[1]OM-denotes "other malignant", and consists of extremely rare subtypes of thyroid origin (e.g., metastasized tissue to the lymph node) that were grouped together.

Classification panels for MTC, BCA, MMN, PTA, and RCC were derived using only samples from the post-surgical thyroid tissue cohort. Each subtype was compared against all other subtypes combined, for example the 23 MTC samples were compared to the remaining 197 samples in the cohort.

The HA/HC classification panel was derived by combining samples of these two subtypes from both the tissue and FNA cohorts. The combined HA/HC samples were then compared against all other subtypes combined. The "Benign/Suspicious" classification panel was derived by combining several sub-analyses in which subsets of "benign" and "malignant" samples were compared. The genes in each classification panel (Figures 3, 4) may be used to accurately classify clinical thyroid FNAs, such as by methods known in the art.

### Example 2: Molecular Profiling of Thyroid Nodule

An individual notices a lump on his thyroid. The individual consults his family physician. The family physician decides to obtain a sample from the lump and subject it to molecular profiling analysis. Said physician uses a kit to obtain the sample via fine needle aspiration, perform an adequacy test, store the sample in a liquid based cytology solution, and sends it to a molecular profiling business. Optionally, the physician may have the cytology examination performed by another party or laboratory. If the cytology examination results in an indeterminate diagnosis, the remaining portion of the sample is sent to the molecular profiling business, or to a third party. The molecular profiling business divides the sample for cytological analysis of one part and for the remainder of the sample extracts mRNA from the sample, analyzes the quality and suitability of the mRNA sample extracted, and analyzes the expression levels and alternative exon usage of a subset of the genes listed in Figure 4. Optionally, a third party not associated with the molecular profiling business may extract the mRNA and /or identify the expression levels of particular biomarkers. The particular gene expression products profile is determined by the sample type, by the preliminary diagnosis of the physician, and by the molecular profiling company.

The molecular profiling business analyzes the data using the classification system obtained by the methods described in Example 1 and provides a resulting diagnosis to the individual's physician. The results provide 1) a list of gene expression products profiled, 2) the results of the profiling (e.g. the expression level normalized to an internal standard such as total mRNA or the expression of a well characterized gene product such as tubulin, 3) the gene product expression level expected for normal tissue of matching type, and 4) a diagnosis and recommended treatment for individual based on the gene product expression levels. The molecular profiling business bills the individual's insurance provider for products and services rendered.

**Example 3: Identification of Hurthle Cell Adenoma and Carcinoma in Thyroid Tissue**

Post-surgical thyroid tissue samples and clinical thyroid FNA biopsies were examined with the Affymetrix Human Exon 1.0 ST microarray in order to identify biomarkers that differ significantly in mRNA expression between benign and malignant samples. These biomarkers were then used to train a molecular classifier using the same post-surgical tissue sample cohort. The information learned during algorithm training using tissue samples, including but not limited to biomarker selection for each thyroid subtype, was combined with a further step of algorithm training using clinical FNA samples, such that the high-dimensionality nature of biomarker expression in FNA can be preserved and used to train an optimized or next-generation molecular classifier. By combining the information learned from tissue and clinical FNAs, the molecular classifier proved to be an accurate molecular diagnostic of Hurthle cell adenoma and Hurthle cell carcinoma. The cohort of samples used to train the tissue-classifier did not contain any Hurthle cell adenoma samples, and the cohort of samples used to train the FNA classifier did not contain any Hurthle cell carcinoma samples. Thus, each molecular classifier training set was deficient in (and unable to learn) how to classify one subtype or the other, but the classifier trained using both sets was able to properly classify both, overcoming the individual limitations of the tissue and FNA training sample sets. Independent validation of the optimized FNA classifier, using a small cohort of HA (n=2) and HC (n=2), resulted in 100% classification accuracy. This demonstrated that a classifier can be trained to accurately classify a sample of thyroid tissue when a member of the class is not represented in a sample set used to train the classifier.

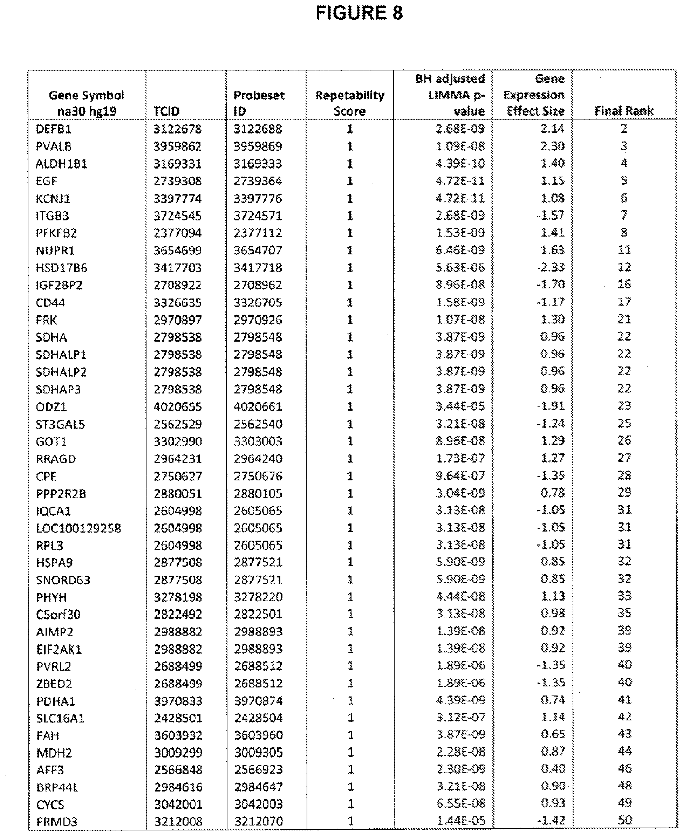

Affymetrix software was used to extract, normalize, and summarize intensity data from roughly 6.5 million probes on the Affymetrix Human Exon 1.0 ST microarray. Approximately 280,000 core probe sets were subsequently used in feature selection and classification. Feature/biomarker selection was carried out using LIMMA models, while random forest and SVM were used for classification (see e.g. Smyth 2004, Statistical applications in genetics and molecular biology 3: Article 3; and Diaz-Uriarte and Alvarez de Andres 2006, BMC Bioinformatics, 7(3)). Iterative rounds of training, classification, and cross-validation were performed using random subsets of data. Top features were identified in at least three separate analyses using the classification scheme described in this example. Features/biomarkers in this example are referred to by a transcript cluster identifier (TCID), as well as by gene name, where available. Some TCIDs may not correspond to a known gene, which depends in part on the progress of gene mapping and identification. Biomarkers identified in this example are listed in a table in Figure 8.

**Example 4: Molecular Classification Using High-Dimensionality Genomic Data**

This examples describes mRNA expression analysis of more than 247,186 transcripts in 363 thyroid nodules comprising multiple subtypes. Starting with surgical tissue from resected thyroid nodules, differentially-expressed transcripts that distinguish benign and malignant nodules are identified. A classifier trained on 178 tissue samples was used to test an independent set of fine needle aspirates (FNAs). Retraining of the algorithm on a set of 137 prospectively collected thyroid FNAs resulted in increased performance, estimated using both 30-fold cross-validation as well as testing on an independent set of FNAs, which included 50% with indeterminate cytopathology. The FNA-trained algorithm was able to classify RNAs in which substantial RNA degradation had occurred and in the presence of blood. Preliminary performance characteristics of the test showed a negative predictive value (NPV) of 96% (95% C.I. 82-99%) and specificity of 84% (95% C.I. 82-99%). The majority of malignant FNAs tolerated a dilution down to 20%.

## Specimens and RNA isolation, amplification, and microarray hybridization

Prospective FNA samples used in this example were either 1) aspirated in vivo at outpatient clinical sites, 2) aspirated pre-operatively, after administering general anesthesia, but prior to surgical incision, or 3) aspirated ex vivo immediately after surgical excision, then directly placed into RNAprotect preservative solution (Qiagen) and stored frozen at -80 C. Prospectively collected FNAs were scored for bloodiness by visual inspection on a 4 point scale. This scale was developed based on an assessment of red/brown coloration and transparency within the preservative solution as compared to assigned reference samples. A score of zero indicates no coloration and complete transparency; a score of 3 indicates dark red/brown coloration and no transparency. Post surgical thyroid tissue was snap-frozen immediately after excision, and stored at -80° C. Cytology and post-surgical histopathology data (when available) was obtained from the collecting site. In order to validate post-surgical pathology findings, slides were re-examined by an expert pathologist who then adjudicated a gold-standard subtype label used for classification training. The specimens in the tissue training set included a 1:1 proportion of benign and malignant samples consisting of 23 nodular hyperplasia (NHP), 40 lymphocytic thyroiditis (Hashimoto's thyroiditis) (LCT), 26 follicular adenoma (FA), 23 Hurthle cell carcinoma (HC), 19 follicular carcinoma (FC), 21 follicular variant of papillary thyroid carcinoma (FVPTC), and 26 papillary thyroid carcinoma (PTC). The specimens in the FNA training set included 96 (70%) benign and 41 (30%) malignant nodules, consisting of 67 NHP, 18 LCT, 9 FA, 2 HA, 3 FC, 4 FVPTC, and 34 PTC. The independent FNA test set (n=48) was prospectively collected subsequent to the training set and included a 50% proportion of indeterminate samples, as determined by FNA cytopathology.

RNA from clinical FNAs was extracted using the AllPrep micro kit (Qiagen). RNA from surgical thyroid tissue was purified using a standard phenol-chloroform extraction and ethanol precipitation method. The quantity and integrity of RNA was determined using a Nanodrop ND-8000 spectrophotometer (Thermo Scientific), Bioanalyzer Picochip system (Agilent Technologies) and Quant-IT RNA kit (Invitrogen). Fifty or twenty-five nanograms of total RNA were then amplified using the NuGEN WT Ovation amplification system, and hybridized to Affymetrix Human Exon 1.0 ST arrays, followed by washing, staining and scanning following manufacturer's protocols (Affymetrix).

The 1.10.2 version of APT (Affymetrix Power Tools) was used to process, normalize, and summarize the .CEL files. Post-hybridization quality control included percent detection above background (DABG), and exon-intron signal separation for control probesets (AUC). Each .CEL file from the independent test set was normalized individually with APT using a quantile normalization sketch and RMA feature effects derived from the training set.

## Training models, classification, and biomarker selection

Classification of samples into benign and malignant categories was done using transcript cluster intensity summaries from the Exon array as features in the model. Selection of markers differentiating benign and malignant categories was done using a LIMMA linear model approach (see e.g. Smyth 2004), as an inner loop of the 30-fold cross-validation process (see e.g. Smyth 2004; and Varma and Simon 2006, BMC Bioinformatics 7(91)). Given a set of informative markers, a linear support vector machine (SVM) model was trained to perform binary classification using R package e1071 (see e.g. Dimitriadou et al. 2009, Misc Functions of the Department of Statistics (e1071); and Cortes and Vapnik 2005, Machine Learning 20:273-297). To estimate performance of the model, both marker selection and model estimation were cross-validated to avoid biases in error estimates. To select optimal number of features in the model, classification performance was estimated as a function of the number of markers in the model. Performance was defined as false positive rate given a fixed false-negative error rate of 5%. Biomarkers of medullary thyroid carcinoma (MTC) were developed separately. A simple linear algorithm applied at the beginning of the analysis, triggered classification of MTC samples, bypassing the molecular classifier described above. The FNA training model was created strictly on FNA samples as described above, except it used the overlap of biomarkers selected from three previous independent analyses using both tissue and FNA samples. When training the classifiers, mapping of SVM scores to a probability space was estimated using a sigmoidal transformation.

In order to determine a classification prediction cut-off value, the cross-validated prediction scores were re-sampled to represent the distribution of subtypes seen in the prospective FNA collection. The target distribution contains approximately 30% malignant samples, in agreement with the reported frequency of indeterminate FNA observed by cytopathology (3-8, 23). The composition of the re-sampled dataset contains the following subtypes: 27.6% NHP, 29.0% FA, 9.5% LCT, 5.4% HA, 1.8% FC, 9% FVPTC, 3.2% HC, 0.5% MTC, and 14% PTC. Since no HC's were accrued in the FNA training set, errors made on the HC subtype were sampled from the FC pool. This represents a conservative estimate of our ability to distinguish HCs since prior analysis based on thyroid tissue has shown comparable error rates between the FC and HC subtypes. Following the re-sampling step, placement of a cut-off value was examined from 0.1 to 0.2 at 0.01 increments. Sensitivity, Specificity, PPV and NPV were produced at each threshold. The threshold that achieved sensitivity above 93%, NPV above 95%, and specificity of at least 70% was chosen; currently the FNA prediction cut-off value is 0.15. Thus, samples with a score less than 0.15 were designated "benign" and those with a score greater than or equal to 0.15 were designated "suspicious."

## Cellular heterogeneity and mixture modeling

Markers of follicular content (FOL) were derived from the literature and are as follows: DIO1, DIO2, EGFR, KRT19, KRT7, MUC1, TG, and TPO (24). Lymphocyte markers were used to estimate lymphocytic content (LCT), these were CD4, FOXP3, IFNG, IGK@, IGL@, IL10, IL2, IL2RA, IL4, and KLRB1 (see e.g. Paul 2008, Fundamental Immunology, xviii:1603). The intensity of each marker in each sample was measured, then averaged across each marker set and mean follicular signal (FOL) was plotted as a function of mean lymphocyte signal (LCT) to generate a curve showing the trade-off between these two components within all tissue samples and all FNA samples used in training.

*In vitro* mixtures of pre-operatively collected PTC and NHP FNAs (each from a single patient) were created by combining total RNA using the following PTC:NHP proportions: 100:0, 40:60, 20:80, 0:100. All dilution ratios were processed in triplicate and carried out to completion including microarray hybridization as described above. *In silico* modeling from two sources was based on linear additive mixing of signals from individual samples in the original intensity space. Briefly, for any two samples A and B, represented by normalized and log-transformed intensity vectors $Y_A$ and $Y_B$, the expected signal in the mixture sample Yc was modeled as:

$$,,Y\text{-}c.=log\text{-}2.,\alpha*,2\text{-},Y\text{-}A..+,1\text{-}\alpha.*,2\text{-},Y\text{-}B...,$$

$$Y_c=log2(\alpha * 2^{Y_A} + (1\text{-}\alpha) * {}^{\wedge}Y_B)$$

where $\alpha$ and $(1\text{-}\alpha)$ represent the proportion of samples A and B in the mixture respectively. To validate the simulation, observed signals from pure NHP and PTC samples from the in vitro mixing experiment were used to generate predicted profiles at proportions of PTC varying from 0 to 1 at 0.01 increments.

*In silico* simulations were applied to estimate the tolerance of the classifier to the effects of LCT and NHP backgrounds. Using the equation above, we simulated intensity profiles for mixtures containing one of 39 PTC samples and one of 59 benign samples (7 LCT and 52 NHP samples). The LCT samples were selected among samples with high average intensity for lymphocyte markers as described above. In contrast, the NHP samples were selected among samples with low average intensity for these markers. This filtering step was performed to ensure good representation of LCT and NHP signals in each of the two pools. For each pair of benign and malignant samples, the *in silico* mixing was done at proportions of PTC varying from 0 to 1 at 0.01 increments, resulting in 100 simulated mixture profiles per pair. The *in silico* mixtures were then scored with a classifier, so that a prediction call of "suspicious" or "benign" could be recorded for all levels of mixing. For this purpose, the classifier was built excluding the pair of pure samples being mixed in order to estimate true "out-of-sample" tolerance to dilution. Given classifier predictions for 100 estimated mixtures per mixed pair, the mixing proportion of PTC signal at which the classifier call switched from "Suspicious" to "Benign" was estimated, effectively characterizing the tolerance of the classifier to the dilution.

## Gene enrichment analysis

A subset of top differentially-expressed genes (n=980), resulting from a LIMMA comparison of benign versus malignant FNAs, was filtered by FDR p-value (≤0.05) and absolute effect size (≥0.5), then subjected to over/under-representation analysis (ORA) using GeneTrail software (see e.g. Backes et al. 2007, Nucleic Acids Research 35:W186-192). Pathway analysis included test (n=306) and reference sets (n=5,048) with available annotation in the KEGG database (see e.g. Kanehisa et al. 2010, Nucleic Acids Research 38:D355-360). Gene ontology analysis used larger test (n=671), and reference sets (n=1 1,218), and was limited to manually curated annotations in the GO database (see e.g. Ashburner et al. 2000, Nature Genetics 25:25-29). Significance was examined using a Fisher's exact test with a threshold of p<0.05 after Benjamini and Hochberg (FDR) correction.

## Performance evaluation of tissue models on FNA samples

Microarray data was first generated from a set of 178 surgical thyroid tissue sample using the Affymetrix Human Exon 1.0 ST array, which measures all known and predicted human transcripts at both the gene and exon level, providing a comprehensive transcriptional profile of the samples. The sample set included the most common benign thyroid nodule subtypes: nodular hyperplasia (NHP), lymphocytic thyroiditis (LCT), follicular adenoma (FA), as well as malignant subtypes such as papillary thyroid carcinoma (PTC), follicular variant of papillary thyroid carcinoma (FVPTC), follicular carcinoma (FC) and Hurthle cell carcinoma (HC). Markers to accurately identify medullary thyroid carcinoma (MTC) were also developed, the identification consisting of applying a simple linear algorithm using a smaller set of markers at the beginning of the analysis, separate from the algorithm used to distinguish the more common thyroid FNA subtypes.

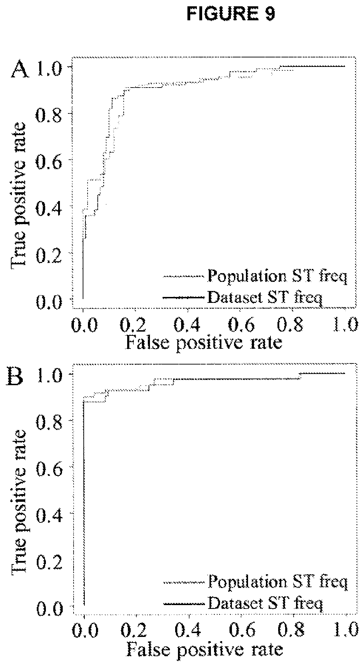

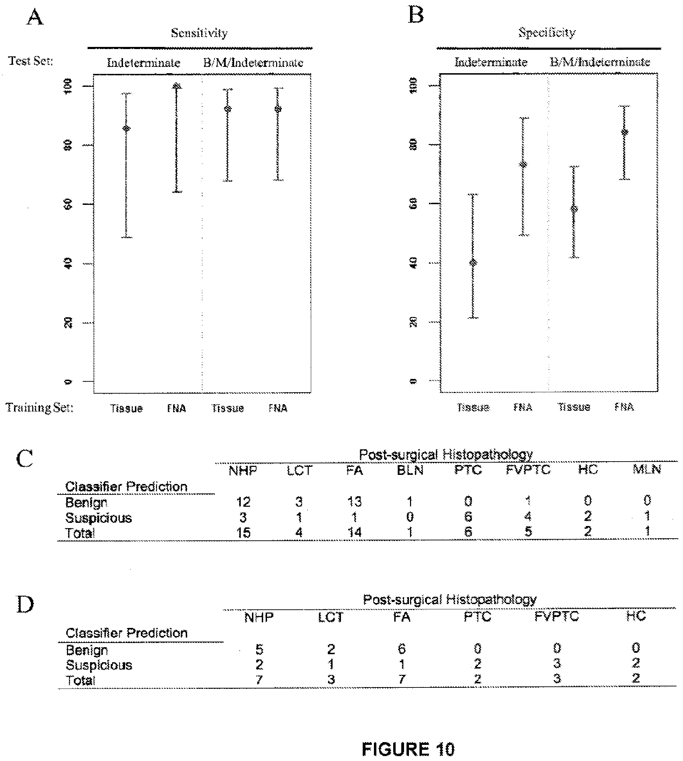

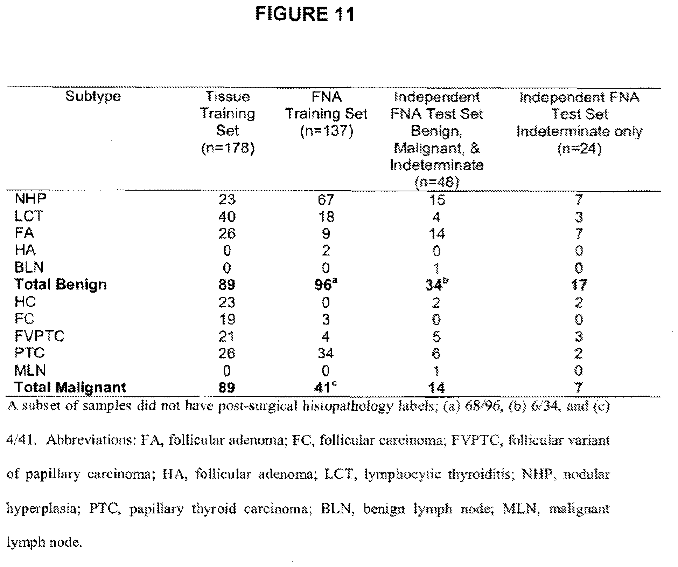

Machine-learning methods were implemented to train a molecular classifier on tissue samples, and following the evaluation of several analytical methods, the support-vector-machine (SVM) method for classification was chosen (see e.g. Cortes and Vapnik 2005). Using 30-fold cross-validation, false positive and false negative error rates were estimated. True positive rate (1-false negative rate) as a function of false positive rate generated a receiver-operator-characteristic (ROC) curve with an area-under-the-curve (AUC) of 0.90 (Figure 9A black line). To represent the true prevalence of malignant samples within the indeterminate group, re-sampling was performed to attain a target subtype distribution containing approximately 30% malignant samples The AUC of the re-sampled ROC curve is 0.89 (Figure 9A gray line). These parameters and models were then used to test an independent set of FNAs to determine whether this performance is generalizable to an unseen data set. A test set of 24 FNAs with indeterminate cytopathology and known surgical pathology diagnoses was combined with an additional 24 FNAs diagnosed as benign or malignant by cytopathology and known surgical pathology diagnoses, for an independent test set of 48 samples. The composition of the sample sets are described in the table in Figure 11. The performance of the tissue-trained classifier decreased when tested on the independent FNAs, with sensitivity of 92% (95% C.I. 68-99%) and specificity of 58% (95% C.I. 41-73%) on the larger set of 48 FNAs (Figure 10). Performance on the indeterminate-only subset of 24 FNAs is similar to the cross-validated performance (Figure 10). Without wishing to be bound by theory, the lower performance of the tissue-trained classifier on FNAs could be due to several reasons; algorithm overfitting, the small sample sizes used for independent testing, or a fundamental difference in the biological or technical properties of tissue samples and FNAs. We addressed the third possibility by first insuring that there were no RNA quality differences between the two sample types used in our analyses, and secondly, by examining cellular heterogeneity as a variable. The first two possibilities are addressed later in this example.

Figure 9 illustrates the performance of a classifier trained on post-surgical thyroid tissues or FNAs. In Figure 9A, ROC curves measure sensitivity (true positive rate) of the tissue classifier as a function of specificity (1-false positive rate) using 30-fold cross-validation. Two curves were generated, one showing performance on the training set without adjusting for subtype prevalence (black), and the second (gray) adjusting subtype error rates to reflect published subtype prevalence frequencies. The area under the curve (AUC) is 0.9 (black curve) or 0.89 (gray curve). In Figure 9B, performance of a classifier trained on FNAs is illustrated. Both training sets are described above and in the table in Figure 11. The AUC is 0.96 for both curves.

Figure 10 illustrates a comparison of tissue-trained and FNA-trained molecular classifiers and their performance on two independent test sets. Sensitivity (Figure 10A) and specificity (Figure 10B) of a tissue-trained classifier and an FNA-trained classifier, on two independent data sets are provided. Indeterminate denotes a set of 24 FNA samples with indeterminate cytopathology, and B/M/Indeterminate includes a set of 48 FNA samples with benign, malignant, or indeterminate cytopathology. Point estimates are shown, with 95% Wilson confidence intervals. Figure 10C provides subtype distribution of the two independent data sets and classifier prediction (either benign or suspicious) for each sample. Surgical pathology labels are abbreviated as follows: NHP, nodular hyperplasia; LCT, lymphocytic thyroiditis; FA, follicular adenoma; BLN, benign lymph node; PTC, papillary thyroid carcinoma; FVPTC, follicular variant of papillary thyroid carcinoma; HC, Hurthle cell carcinoma; and MLN, malignant lymph node.

Figure 11 provides a table illustrating the composition of samples used in algorithm training and testing, by subtype, as defined by expert post-surgical histopathology review. A subset of samples did not have post-surgical histopathology labels, as indicated by superscripts for values in the tables, which are as follows: (a) 68/96, (b) 6/34, and (c) 4/41. Surgical pathology labels are abbreviated in the table as follows: FA, follicular adenoma; FC, follicular carcinoma; FVPTC, follicular variant of papillary carcinoma; HA, Hurthle cell adenoma; LCT, lymphocytic thyroiditis; NHP, nodular hyperplasia; PTC, papillary thyroid carcinoma; BLN, benign lymp node; MLN, malignant lymph node.

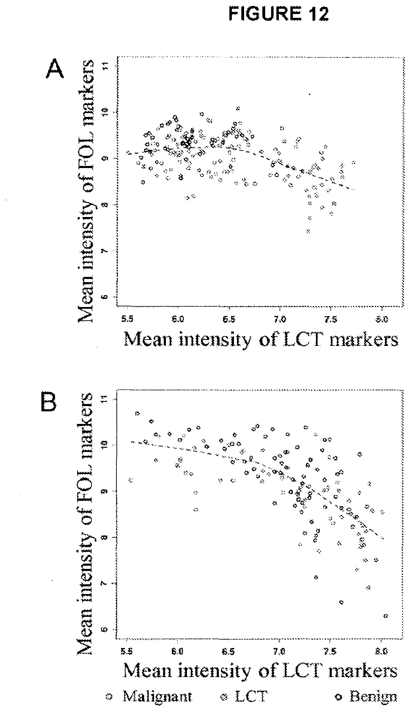

To evaluate cellular heterogeneity between tissues and FNAs, genes known to be present in thyroid follicular cells and lymphocytes were measured, and the measurements were used to create a composite measure of each sample based on the average signal of all follicular content markers as a function of average lymphocyte content markers. Markers were selected that were not differentially expressed in benign versus malignant nodules. This composite measure had significantly higher variability in FNA samples (Figure 12B) than in surgical tissue samples (Figure 12A). The data highlight the value of accounting for cellular heterogeneity in biomarker discovery. Specifically, Figure 12 provides a comparison of composite follicular (FOL) and lymphocytic (LCT) scores across surgical tissue (Figure 12A; n=178) and FNAs (Figure 12B; n=137). The mean signal intensity of follicular cell biomarkers decreases as the mean signal intensity of lymphocytic markers increases. This trade-off between follicular cell content and lymphocytic background is substantially greater in FNAs than in tissue.

Performance of FNA models on FNA samples

A cohort (n=960) of prospectively collected clinical thyroid FNAs from more than 20 clinics across the United States, 137 of which corresponding surgical pathology was available on FNAs encompassing both prevalent and rare thyroid subtypes. The composition of this training set is shown in Figure 11. Histopathology slides from all patients who underwent surgical resection were subjected to primary review by a surgical pathologist, and when available, subjected to secondary review by a panel of two experts in order to adjudicate gold-standard classification and subtype training labels. Genome-wide expression data from this cohort was used to develop a second-generation classifier, trained on FNAs, to achieve desired clinical performance. First, we estimated classifier performance using 30-fold cross-validation (similar to the process used with the tissue classifier, see Figure 9A). The cross-validated ROC curve (sensitivity of the classifier as a function of false positive rate) had an AUC of 0.96 for the training data "as is" and 0.97 when re-sampled to account for the prevalence of subtypes in the indeterminate population. When sensitivity is fixed at 95%, specificity remains very high, at 75% (Figure 9B) and is unaffected by varying quantities of blood in the FNA. This classifier was then tested on the same independent test sets of prospectively collected clinical FNAs used to test the tissue-trained classifier (Figure 10A and B). Data shown in Figure 10 indicates that sensitivity and specificity have increased significantly for both the n=24 and n=48 independent FNA test sets using FNA-trained classifiers. While these test sets are small in size, their performance is similar to that of the cross-validated training set, suggesting that the algorithm is not overfitted, and that the FNA-trained classifier is generalizable to unseen data sets. The composition of the test set is approximately 30% malignant subtypes, similar to that described for clinical FNA samples. A multi-center prospective clinical trial across over 40 U.S. academic and community-based sites can be used to validate the performance of this molecular test on a large set of indeterminate FNAs.

*In vitro* and *in silico* modeling of sample mixtures

In order to determine how sensitive the classifier is to decreasing proportions of malignant cells, a model for *in silico* simulation of the mixture signals was proposed, the model was validated with *in vitro* mixing experiments, and computational simulations were used to analyze the tolerance of the classifier to the dilution effects. In general, an *in silico* model can serve as a reasonable approximation to the mixing process if the deviation of simulated mixture profiles from the actual observed signals is within the noise typically observed for technical replicates. I this example, the distribution of the inter-quartile range of the difference in intensities between *in silico* predictions and *in vitro* observed signals for the marker set was similar to that observed for pairs of technical replicates.

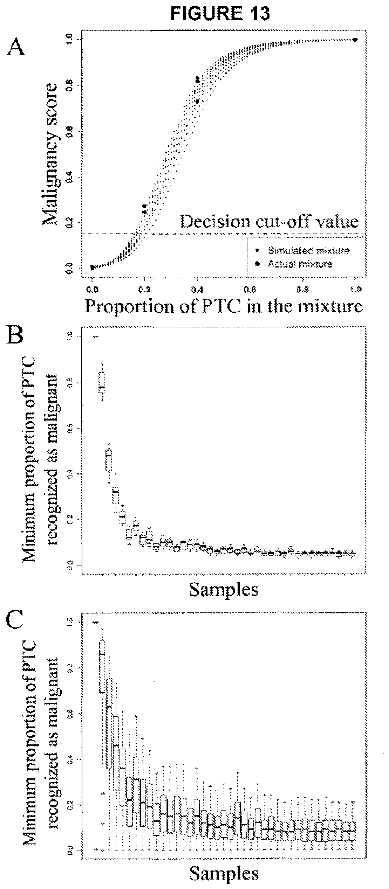

Figure 13A shows the effects of varying proportion of PTC signal in the mixture (x axis) on the classification scores (y axis), and that the classifier performance is highly tolerant to sample dilution and heterogeneity. The *in vitro* data is nearly superimposable on the *in silico* predictions made for mixtures with similar PTC content. In the case of this particular PTC sample, the classifier tolerates dilution of the PTC signal to less than 20% of the original level and reports a "suspicious" call for the "mixed" sample. However, a different clinical sample may contain a smaller proportion of malignant cells and may be characterized by smaller tolerance to dilution. Given the agreement established between *in silico* and *in vitro* simulations, we next used computational simulations to investigate dilution effects on a broader set of FNAs.

Each of 39 PTC FNA samples were mixed *in silico* with one of either LCT or NHP samples. Individual FNA samples did not represent pure expression of any single component of the possible cellular types. However, the variety of signal present in many LCT and NHP samples represents the spectrum of the possible composite background signals that could obscure malignant cell signals in clinical biopsies. To separately investigate the effects of LCT and NHP backgrounds, we restricted the pool of LCT samples to seven FNA samples with the highest average intensity of LCT markers derived from this data set. Similarly, the NHP samples were restricted to the 52 samples with the lowest estimated LCT content. This filtering step was performed to ensure good representation of LCT and NHP signals in each of the two sets. For each pair of benign and malignant samples, the mixing was done at proportions of PTC varying from 0 to 1 at 0.01 increments, resulting in 100 simulated mixture profiles per pair. The *in silico* mixture samples were then scored with a classifier, so that a "suspicious" or "benign" call could be recorded for all levels of mixing. For this purpose, the classifier was built excluding the pair of pure samples being mixed in order to estimate true "out-of-sample" tolerance to dilution. Given classifier predictions, we estimated the mixing proportion of PTC signal at which the classifier call switched from "suspicious" to "benign", effectively characterizing the tolerance of the classifier to the dilution.

The results of this simulation are summarized in Figure 13, showing the minimum proportion of the PTC signal that results in a "suspicious" call by the classifier. Prediction score tolerance results for mixing with LCT background are shown in Figure 13B and prediction score tolerance results for mixing with NHP background are shown in Figure 13C. Each of the PTC samples is represented by a boxplot, corresponding to mixes with all possible representatives of the benign subtype. The PTC samples are arranged on the x axis in the order of increasing classification scores for the original PTC sample. The values on the y axis are the minimum proportion of PTC that is still reported as "suspicious" by the classifier. Smaller values correspond to higher tolerance to dilution. Tolerance is higher for dilution with LCT signal. Over 80% of all PTC samples in this data set can be diluted to levels below 10% of the original signal with LCT background and still be correctly called by the classifier. Up to 50% of the samples can be diluted to less than 6% of the original sample. PTC samples appear more sensitive to dilutions with NHP signal, with highest scoring samples tolerating, on average, dilution down to 12% of the original signal, and approximately 80% of PTC samples tolerate dilutions down to 20% of the original signal. We also observe that the variances of tolerance for any given PTC sample are larger than those observed for LCT background.

Gene enrichment analysis

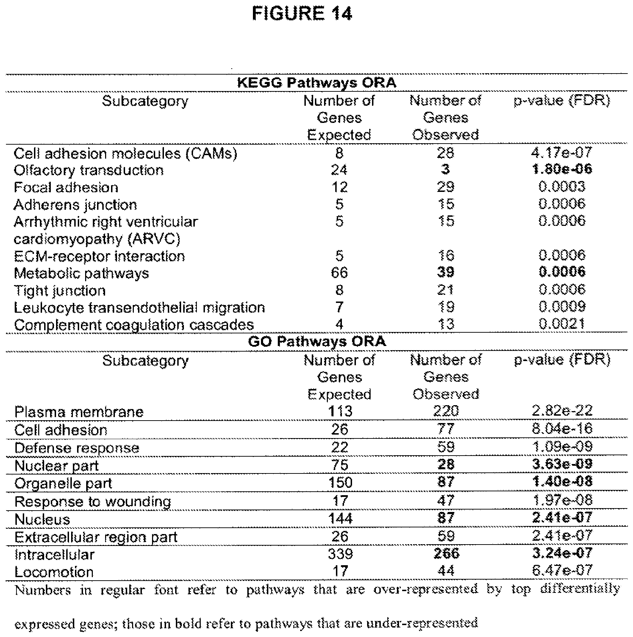

The classifier training process identified many genes well known for their involvement in thyroid malignancy, as well as those previously not associated with this disease. In order to characterize the biological signatures associated with these genes, we performed over representation analysis (ORA) using differentially expressed genes with high statistical support. The analysis tests the likelihood that an observed group of genes (i.e., genes in a pathway), share a non-random connection pointing to the underlying biology. The first analysis focused on the KEGG pathways database and revealed enrichment of cell membrane-mediated pathways (Figure 14). The extracellular membrane (ECM) receptor interaction, cell adhesion, tight junction, and focal adhesion pathways highlight the role of integrins among other membrane bound mediators in thyroid malignancy. Other top pathways point to TNF-, Rho-, and chemokine gene families long known for their involvement in carcinogenesis. These results are complemented by ORA using the gene ontology (GO) database. Again, endothelial, ECM, and cell membrane signatures represent five out of the top 10 results. Another, top ranked biological signature detected in the GO ORA points to wound healing. This gene expression signature has been associated with diminished survival in breast cancer patients.

Figure 14 summarizes the ORA of top differentially expressed genes (n=980), with 657 genes being up-regulated and 323 genes being down-regulated. Numbers in regular font refer to pathways that are over-represented by top differentially expressed genes, while numbers in bold refer to pathways that are under-represented.

Sample Biomarkers

The fibronectin gene FN1 was among the known genes identified in the gene selection process. Other known genes of interest include thyroid peroxidase (TPO), galectin-3 (LGALS3), calcitonin (CALCA), tissue inhibitor of metalloproteinase (TIMP), angiopoietin-2 (ANGPT2), and telomerase reverse transcriptase(TERT), all genes that have been shown to be implicated in thyroid cancer. In this example, the classifier uses signals from approximately 100-200 genes to achieve high accuracy. The molecular test described in this example can, thus, use high-density genomic information to extract meaningful signal from challenging samples and complement, or optionally replace, routine cytopathological and clinical assessment of thyroid nodules, enabling a more accurate classification of the nodule as benign.

**Claims**

1. A method for evaluating a thyroid tissue sample comprising:

   (a) determining an expression level for one or more gene expression products from said thyroid tissue sample, wherein said one or more gene expression products correspond to at least 5 genes selected from SNCA, STK32A, THRSP, TIMP1, TIMP2, TMSB10, TNFRSF17, TNFRSF1A, TXNDC12, VWA5A, WAS, WIPI1, and ZFYVE16; and
   (b) classifying the thyroid tissue sample as (i) benign or suspicious or (ii) benign or malignant, by comparing said expression level to gene expression data for at least two different sets of biomarkers, the gene expression data for each set of biomarkers comprising one or more reference gene expression levels correlated with a presence of one or more tissue types, wherein said expression level is compared to gene expression data for said at least two different sets of biomarkers sequentially.

2. The method of claim 1, wherein said comparing in (b) comprises comparing said expression level to gene expression data of a final set of biomarkers using a classifier.

3. The method of claim 2, wherein said classifier is trained with gene expression data correlated with a presence of one or more of the following tissue types: follicular thyroid adenoma, follicular thyroid carcinoma, nodular hyperplasia, papillary thyroid carcinoma, follicular variant of papillary carcinoma, Hurthle cell carcinoma, Hurthle cell adenoma, and lymphocytic thyroiditis.

4. The method of claim 2, wherein a first set of biomarkers of said at least two different sets of biomarkers comprises gene expression data correlated with a presence of one or more of the following tissue types: medullary thyroid carcinoma, renal carcinoma metastasis to the thyroid, parathyroid, breast carcinoma metastasis to the thyroid, and melanoma metastasis to the thyroid.

5. The method of claim 1, further comprising providing said thyroid tissue sample obtained from said subject for use in step (a).

6. The method of claim 1, the classifying comprises inputting said expression level to a computer system comprising said one or more reference gene expression levels.

7. The method of claim 1, wherein said gene expression data for said at least two different sets of biomarkers is correlated with a presence of:

(i) one or more tissue types selected from the group consisting of normal thyroid, follicular thyroid adenoma, nodular hyperplasia, lymphocytic thyroiditis, Hurthle cell adenoma, follicular thyroid carcinoma, papillary thyroid carcinoma, follicular variant of papillary carcinoma, medullary thyroid carcinoma, Hurthle cell carcinoma, anaplastic thyroid carcinoma, renal carcinoma metastasis to the thyroid, breast carcinoma metastasis to the thyroid, melanoma metastasis to the thyroid, B cell lymphoma metastasis to the thyroid, and parathyroid; or
(ii) one or more tissue types selected from the group consisting of follicular thyroid adenoma, follicular thyroid carcinoma, nodular hyperplasia, papillary thyroid carcinoma, follicular variant of papillary carcinoma, lymphocytic thyroiditis, Hurthle cell adenoma, and Hurthle cell carcinoma; or
(iii) Hurthle cell adenoma and/or Hurthle cell carcinoma.

8. The method of claim 1, wherein said gene expression data for said at least two different sets of biomarkers is correlated with a presence of one or more tissue types selected from the group consisting of medullary thyroid carcinoma, renal carcinoma metastasis to the thyroid, parathyroid, breast carcinoma metastasis to the thyroid, melanoma metastasis to the thyroid, Hurthle cell adenoma, and Hurthle cell carcinoma.

9. The method of claim 1, wherein gene expression data for a first set of biomarkers of said at least two different sets of biomarkers is correlated with a presence of one or more tissue types selected from the group consisting of medullary thyroid carcinoma, renal carcinoma metastasis to the thyroid, parathyroid, breast carcinoma metastasis to the thyroid, melanoma metastasis to the thyroid, Hurthle cell adenoma, and Hurthle cell; and a second of said at least two sets of biomarkers comprises one or more gene expression product levels correlated with the presence of one or more tissue types selected from the group consisting of follicular thyroid adenoma, follicular thyroid carcinoma, nodular hyperplasia, papillary thyroid carcinoma, follicular variant of papillary carcinoma, lymphocytic thyroiditis, Hurthle cell adenoma, and Hurthle cell carcinoma.

10. The method of claim 1, wherein said gene expression data comprises data obtained from a plurality of reference samples, wherein said plurality of reference samples comprise:

(i) at least 200 surgical biopsy samples; and/or
(ii) at least 200 fine needle aspiration samples.

11. The method of claim 1, wherein said classifying of (b) is at an NPV of at least 96%.

12. The method of claim 1, wherein said expression level for one or more gene expression products further comprises genes selected from the group consisting of AFF3, AIMP2, ALDH1B1, BRP44L, C5orf30, CD44, CPE, CYCS, DEFB1, EGF, EIF2AK1, FAH, FRK, FRMD3, GOT1, HSD17B6, HSPA9, IGF2BP2, IQCA1, ITGB3, KCNJ1, LOC100129258, MDH2, NUPR1, ODZ1, PDHA1, PFKFB2, PHYH, PPP2R2B, PVALB, PVRL2, RPL3, RRAGD, SDHA, SDHALP1, SDHALP2, SDHAP3, SLC16A1, SNORD63, ST3GAL5, ZBED2, ABCD2, ACER3, ACSL1, AHNAK, AIM2, ARSG, ASPN, AUTS2, BCL2L1, BTLA, C1lorf72, C4orf7, CC2D2B, CCL19, CCND1, CD36, CD52, CD96, CFH, CFHR1, CLDN1, CLDN16, CR2, CREM, CTNNA2, CXCL13, DAB2, DDI2, DNAJC13, DPP4, DPP6, DYNLT1, EAF2, EMR3, FABP4, FBXO2, F1142258, FN1, FPR2, FREM2, FXYD6, GOS2, GABRB2, GAL3ST4, GIMAP2, GMFG, GPHN, GPR174, GZMK, HCG11, HNRNPA3, IGHG1, IL7R, ITGB1, KCNA3, KLRG1, LCP1, LIPH, LOC100131599, LOC647979, LRP12, LRP1B, MAGI3, MAPK6, MATN2, MDK, MPPED2, MT1F, MT1G, MT1H, MT1P2, MYEF2, NDUFC2, NRCAM, OR10D1P, P2RY10, P2RY13, PARVG, PDE8A, PIGN, PIK3R5, PKHD1L1, PLA2G16, PLCB1, PLEK, PRKG1, PRNP, PROS1, PTPRC, PTPRE, PYGL, PYH1N1, PZP, RGS13, RIMS2, RNF24, ROS1, RXRG, SCEL, SCUBE3, SEMA3D, SERGEF, SERPINA1, SERPINA2, SHC1, SLAMF6, SLC24A5, SLC31A1, SLC34A2, SLC35B1, SLC43A3, SLC4A1, and SLC4A4.

**13.** The method of claim 1, wherein said thyroid tissue sample is a fine needle aspirate (FNA) thyroid tissue sample.

**14.** The method of claim 2, wherein said classifier has been trained using:

(i) greater than 200 clinical samples, or
(ii) samples derived from at least 5 different geographical locations.

**15.** The method of claim 1, wherein said classifying comprises using a machine learning algorithm.

**Patentansprüche**

**1.** Verfahren zur Bewertung einer Schilddrüsengewebeprobe umfassend:

(a) Bestimmen einer Expressionsebene für ein oder mehrere Genexpressionsprodukte aus der Schilddrüsengewebeprobe, wobei das eine oder die mehreren Genexpressionsprodukte mindestens 5 Genen entsprechen, ausgewählt aus SNCA, STK32A, THRSP, TIMP1, TIMP2, TMSB10, TNFRSF17, TNFRSF1A, TXNDC12, VWA5A, WAS, WIPI1 und ZFYVE16; und
(b) Klassifizieren der Schilddrüsengewebeprobe als (i) gutartig oder verdächtig oder (ii) gutartig oder bösartig durch Vergleichen der Expressionsebene mit Genexpressionsdaten für mindestens zwei verschiedene Sätze von Biomarkern, die Genexpressionsdaten für jeden Satz von Biomarkern umfassend eine oder mehrere Referenzgenexpressionsebenen, die mit einer Anwesenheit eines oder mehrerer Gewebetypen korreliert sind, wobei die Expressionebene nacheinander mit Genexpressionsdaten für die mindestens zwei verschiedenen Sätze von Biomarkern verglichen wird.

**2.** Verfahren nach Anspruch 1, wobei das Vergleichen in (b) Vergleichen der Expressionsebene mit Genexpressionsdaten eines endgültigen Satzes von Biomarkern unter Verwendung eines Klassierers umfasst.

**3.** Verfahren nach Anspruch 2, wobei der Klassierer mit Genexpressionsdaten trainiert wird, die mit einer Anwesenheit eines oder mehrerer der folgenden Gewebetypen korrelieren: follikuläres Schilddrüsenadenom, follikuläres Schilddrüsenkarzinom, noduläre Hyperplasie, papilläres Schilddrüsenkarzinom, follikuläre Variante des papillären Karzinoms, Hürthle-Zell-Karzinom, Hürthle-Zell-Adenom und lymphatische Thyreoiditis.

**4.** Verfahren nach Anspruch 2, wobei ein erster Satz von Biomarkern der mindestens zwei unterschiedlichen Sätze von Biomarkern Genexpressionsdaten umfasst, die mit der Anwesenheit eines oder mehrerer der folgenden Gewebetypen korreliert sind: medulläres Schilddrüsenkarzinom, Nierenkarzinom-Metastasierung der Schilddrüse. Nebenschilddrüse, Brustkrebs-Metastasierung der Schilddrüse und Melanom-Metastasierung der Schilddrüse.

**5.** Verfahren nach Anspruch 1, ferner umfassend Bereitstellen der von dem Subjekt erhaltenen Schilddrüsengewebeprobe zur Verwendung in Schritt (a).

**6.** Verfahren nach Anspruch 1, wobei das Klassifizieren das Eingeben der Expressionsebene in ein Computersystem umfasst, das die eine oder die mehreren Referenzgenexpressionsebenen umfasst.

**7.** Verfahren nach Anspruch 1, wobei die Genexpressionsdaten für die mindestens zwei unterschiedlichen Sätze von Biomarkern sind mit einer Anwesenheit von:

(i) einem oder mehrere Gewebetypen, ausgewählt aus der Gruppe bestehend aus normaler Schilddrüse, follikulärem Schilddrüsenadenom, nodulärer Hyperplasie, lymphatischer Thyreoiditis, Hürthle-Zell-Adenom follikulärem Schilddrüsenkarzinom, papillärem Schilddrüsenkarzinom, follikulärer Variante des papillären Karzinoms, medullärem Schilddrüsenkarzinomkarzinom, Hürthle-Zell-Karzinom, anaplastischem Schilddrüsenkarzinom, Nierenkarzinom-Metastasierung der Schilddrüse, Brustkarzinom-Metastasierung der Schilddrüse, Melanommetastasierung der Schilddrüse, B-Zell-Lymphommetastasierung der Schilddrüse und Nebenschilddrüse; oder
(ii) eines oder mehrerer Gewebetypen, ausgewählt aus der Gruppe bestehend aus follikulärem Schilddrüsenadenom, follikulärem Schilddrüsenkarzinom, nodulärer Hyperplasie, papillärem Schilddrüsenkarzinom, follikulärer Variante des papillären Karzinoms, lymphatischer Thyreoiditis, Hürthle-Zell-Adenom, und Hürthle-Zell-Karzinom; oder
(iii) Hürthle-Zell-Adenom und/oder Hürthle-Zell-Karzinom.

**8.** Verfahren nach Anspruch 1, wobei die Genexpressionsdaten für die mindestens zwei unterschiedlichen Sätze von Biomarkern mit einer Anwesenheit eines oder mehrerer Gewebetypen korreliert sind, ausgewählt aus der Gruppe bestehend aus medullärem Schilddrüsenkarzinom, Nierenkarzinom-Metastasierung der Schilddrüse, Nebenschilddrüse, Brustkrebs-Metastasierung der Schilddrüse. Melanom-Metastasierung der Schilddrüse, Hürthle-Zell-Adenom, und Hürthle-Zell-Karzinom.

**9.** Verfahren nach Anspruch 1, wobei die Genexpressionsdaten für einen ersten Satz von Biomarkern der mindestens zwei unterschiedlichen Sätze von Biomarkern mit einer Anwesenheit eines oder mehrerer Gewebetypen korreliert sind, ausgewählt aus der Gruppe bestehend aus medullärem Schilddrüsenkarzinom, Nierenkarzinom-Metastasierung der Schilddrüse, Nebenschilddrüse, Brustkrebs-Metastasierung der Schilddrüse, Melanom-Metastasierung der Schilddrüse, Hürthle-Zell-Adenom, und Hürthle-Zelle; und ein zweiter der mindestens zwei Sätzen von Biomarkern eine oder mehrere Genexpressionsproduktebenen umfasst, die mit der Anwesenheit eines oder mehrerer Gewebetypen korreliert sind, ausgewählt aus der Gruppe bestehend aus follikulärem Schilddrüsenadenom, follikulärem Schilddrüsenkarzinom, nodulärer Hyperplasie, papillärem Schilddrüsenkarzinom, follikulärer Variante des papillären Karzinoms, lymphatischer Thyreoiditis, Hürthle-Zell-Adenom, und Hürthle-Zell-Karzinom.

**10.** Verfahren nach Anspruch 1, wobei die Genexpressionsdaten Daten umfassen, die aus mehreren Referenzproben erhalten wurden, wobei die mehreren Referenzproben umfassen:

(i) mindestens 200 chirurgische Biopsieproben; und/oder
(ii) mindestens 200 Feinnadelaspirationsproben.

**11.** Verfahren nach Anspruch 1, wobei das Klassifizieren von (b) bei einem NPV von mindestens 96 % liegt.

**12.** Verfahren nach Anspruch 1, wobei die Expressionsebene für ein oder mehrere Genexpressionsprodukte ferner Gene umfasst, ausgewählt aus der Gruppe bestehend aus AFF3, AIMP2, ALDH1B1, BRP44L, C5orf30, CD44, CPE, CYCS, DEFB1, EGF, EIF2AK1, FAH, FRK, FRMD3, GOT1, HSD17B6, HSPA9, IGF2BP2, IQCA1, ITGB3, KCNJ1, LOC100129258, MDH2, NUPR1, ODZ1, PDHA1, PFKFB2, PHYH, PPP2R2B, PVALB, PVRL2, RPL3, RRAGD, SDHA, SDHALP1, SDHALP2, SDHAP3, SLC16A1, SNORD63, ST3GAL5, ZBED2, ABCD2, ACER3, ACSL1, AHNAK, AIM2, ARSG, ASPN, AUTS2, BCL2L1, BTLA, C1 lorf72, C4orf7, CC2D2B, CCL19, CCND1, CD36, CD52, CD96, CFH, CFHR1, CLDN1, CLDN16, CR2, CREM, CTNNA2, CXCL13, DAB2, DDI2, DNAJC13, DPP4, DPP6, DYNLT1, EAF2, EMR3, FABP4, FBXO2, F1142258, FN1, FPR2, FREM2, FXYD6, GOS2, GABRB2, GAL3ST4, GIMAP2, GMFG, GPHN, GPR174, GZMK, HCG11, HNRNPA3, IGHG1, IL7R, ITGB1, KCNA3, KLRG1, LCP1, LIPH, LOC100131599, LOC647979, LRP12, LRP1B, MAG13, MAPK6, MATN2, MDK, MPPED2, MT1F, MT1G, MT1H, MT1P2, MYEF2, NDUFC2, NRCAM, OR10D1P, P2RY10, P2RY13, PARVG, PDE8A, PIGN, PIK3R5, PKHD1L1, PLA2G16, PLCB1, PLEK, PRKG1, PRNP, PROS1, PTPRC, PTPRE, PYGL, PYH1N1, PZP, RGS13, RIMS2, RNF24, ROS1, RXRG, SCEL, SCUBE3, SEMA3D, SERGEF, SERPINA1, SERPINA2, SHC1, SLAMF6, SLC24A5, SLC31A1, SLC34A2, SLC35B1, SLC43A3, SLC4A1 und SLC4A4.

**13.** Verfahren nach Anspruch 1, wobei die Schilddrüsengewebeprobe eine Schilddrüsengewebeprobe mit feinem Nadelaspirat (FNA) ist.

**14.** Verfahren nach Anspruch 2, wobei der Klassierer trainiert wurde unter Verwendung von:

(i) mehr als 200 klinischen Proben oder
(ii) Proben von mindestens 5 verschiedenen geografischen Standorten.

**15.** Verfahren nach Anspruch 1, wobei das Klassifizieren Verwenden eines maschinellen Lernalgorithmus umfasst.

**Revendications**

**1.** Procédé d'évaluation d'un échantillon de tissu thyroïdien comprenant :

(a) la détermination d'un niveau d'expression pour au moins un produit d'expression génique issu dudit échantillon de tissu thyroïdien, ledit au moins un produit d'expression génique correspondant à au moins 5 gènes choisis parmi SNCA, STK32A, THRSP, TIMP1, TIMP2, TMSB10, TNFRSF17, TNFRSF1A, TXNDC12, VWA5A, WAS, WIPI1 et ZFYVE16 ; et

(b) la classification de l'échantillon de tissu thyroïdien comme étant (i) bénin ou suspect ou (ii) bénin ou malin, par la comparaison dudit niveau d'expression à des données d'expression génique pour au moins deux ensembles différents de biomarqueurs, les données d'expression génique pour chaque ensemble de biomarqueurs comprenant au moins un niveau d'expression génique de référence corrélé avec une présence d'au moins un type de tissu, ledit niveau d'expression étant comparé à des données d'expression génique pour lesdits au moins deux ensembles différents de biomarqueurs séquentiellement.

2. Procédé selon la revendication 1, dans lequel ladite comparaison dans (b) comprend la comparaison dudit niveau d'expression à des données d'expression génique d'un ensemble final de biomarqueurs à l'aide d'un classificateur.

3. Procédé selon la revendication 2, dans lequel ledit classificateur est formé avec des données d'expression génique corrélées avec une présence d'au moins un des types de tissu suivants : adénome folliculaire thyroïdien, carcinome folliculaire thyroïdien, hyperplasie nodulaire, carcinome papillaire de la thyroïde, variante folliculaire du carcinome papillaire, carcinome à cellules de Hürthle, adénome à cellules de Hürthle et thyroïdite lymphocytaire.

4. Procédé selon la revendication 2, dans lequel un premier ensemble de biomarqueurs desdits au moins deux ensembles différents de biomarqueurs comprend des données d'expression génique corrélées avec une présence d'au moins un des types de tissu suivants : carcinome médullaire de la thyroïde, métastase de carcinome rénal dans la thyroïde, la parathyroïde, métastase de carcinome du sein dans la thyroïde et métastase de mélanome dans la thyroïde.

5. Procédé selon la revendication 1, comprenant en outre la production dudit échantillon de tissu thyroïdien prélevé sur ledit sujet pour une utilisation dans l'étape (a).

6. Procédé selon la revendication 1, la classification comprenant l'entrée dudit niveau d'expression dans un système informatique comprenant ledit au moins un niveau d'expression génique de référence.

7. Procédé selon la revendication 1, dans lequel lesdites données d'expression génique pour lesdits au moins deux ensembles différents de biomarqueurs sont corrélées avec une présence de :

(i) au moins un type de tissu choisi dans le groupe constitué par la thyroïde normale, l'adénome folliculaire thyroïdien, l'hyperplasie nodulaire, la thyroïdite lymphocytaire, l'adénome à cellules de Hürthle, le carcinome folliculaire thyroïdien, le carcinome papillaire de la thyroïde, la variante folliculaire ou le carcinome papillaire, le carcinome médullaire de la thyroïde, le carcinome à cellules de Hürthle, le carcinome anaplasique de la thyroïde, la métastase du carcinome rénal dans la thyroïde, la métastase du carcinome du sein dans la thyroïde, la métastase de mélanome dans la thyroïde, la métastase de lymphome de cellules B dans la thyroïde et la parathyroïde ; ou
(ii) au moins un type de tissu choisi dans le groupe constitué par l'adénome folliculaire thyroïdien, le carcinome folliculaire thyroïdien, l'hyperplasie nodulaire, le carcinome papillaire de la thyroïde, la variante folliculaire du carcinome papillaire, la thyroïdite lymphocytaire, l'adénome à cellules de Hürthle et le carcinome à cellules de Hürthle ; ou
(iii) l'adénome à cellules de Hürthle et/ou le carcinome à cellules de Hürthle.

8. Procédé selon la revendication 1, dans lequel lesdites données d'expression génique pour lesdits au moins deux ensembles différents de biomarqueurs sont corrélées avec une présence d'au moins un type de tissu choisi dans le groupe constitué par le carcinome médullaire de la thyroïde, la métastase de carcinome rénal dans la thyroïde, la parathyroïde, la métastase de carcinome du sein dans la thyroïde, la métastase de mélanome dans la thyroïde, l'adénome à cellules de Hürthle et le carcinome à cellules de Hürthle.

9. Procédé selon la revendication 1, dans lequel des données d'expression génique pour un premier ensemble de biomarqueurs desdits au moins deux ensembles différents de biomarqueurs sont corrélées avec une présence d'au moins un type de tissu choisi dans le groupe constitué par le carcinome médullaire de la thyroïde, la métastase de carcinome rénal dans la thyroïde, la parathyroïde, la métastase de carcinome du sein dans la thyroïde, la métastase de mélanome dans la thyroïde, l'adénome à cellules de Hürthle et la cellule de Hürthle ; et un deuxième élément desdits au moins deux ensembles de biomarqueurs comprenant au moins un niveau de produit d'expression génique corrélé avec la présence d'au moins un type de tissu choisi dans le groupe constitué par l'adénome folliculaire thyroïdien, le carcinome folliculaire thyroïdien, l'hyperplasie nodulaire, le carcinome papillaire de la thyroïde, la variante folliculaire du carcinome papillaire, la thyroïdite lymphocytaire, l'adénome à cellules de Hürthle et le carcinome à cellules de Hürthle.

**10.** Procédé selon la revendication 1, dans lequel lesdites données d'expression génique comprennent des données obtenues à partir d'une pluralité d'échantillons de référence, ladite pluralité d'échantillons de référence comprenant :

(i) au moins 200 échantillons de biopsie chirurgicale ; et/ou
(ii) au moins 200 échantillons d'aspiration à l'aiguille fine.

**11.** Procédé selon la revendication 1, dans lequel ladite classification de (b) se situe à une valeur prédictive négative de 96 %.

**12.** Procédé selon la revendication 1, dans lequel ledit niveau d'expression pour au moins un produit d'expression génique comprend en outre des gènes choisis dans le groupe constitué par AFF3, AIMP2, ALDH1B1, BRP44L, C5orf30, CD44, CPE, CYCS, DEFB1, EGF, EIF2AK1, FAH, FRK, FRMD3, GOT1, HSD17B6, HSPA9, IGF2BP2, IQCA1, ITGB3, KCNJ1, LOC100129258, MDH2, NUPR1, ODZ1, PDHA1, PFKFB2, PHYH, PPP2R2B, PVALB, PVRL2, RPL3, RRAGD, SDHA, SDHALP1, SDHALP2, SDHAP3, SLC16A1, SNORD63, ST3GAL5, ZBED2, ABCD2, ACER3, ACSL1, AHNAK, AIM2, ARSG, ASPN, AUTS2, BCL2L1, BTLA, CI1orf72, C4orf7, CC2D2B, CCL19, CCND1, CD36, CD52, CD96, CFH, CFHR1, CLDN1, CLDN16, CR2, CREM, CTNNA2, CXCL13, DAB2, DDI2, DNAJC13, DPP4, DPP6, DYNLT1, EAF2, EMR3, FABP4, FBXO2, F1142258, FN1, FPR2, FREM2, FXYD6, GOS2, GABRB2, GAL3ST4, GIMAP2, GMFG, GPHN, GPR174, GZMK, HCG11, HNRNPA3, IGHG1, IL7R, ITGB1, KCNA3, KLRG1, LCP1, LIPH, LOC100131599, LOC647979, LRP12, LRP1B, MAG13, MAPK6, MATN2, MDK, MPPED2, MT1F, MT1G, MT1H, MT1P2, MYEF2, NDUFC2, NRCAM, OR10D1P, P2RY10, P2RY13, PARVG, PDE8A, PIGN, PIK3R5, PKHD1L1, PLA2G16, PLCB1, PLEK, PRKG1, PRNP, PROS1, PTPRC, PTPRE, PYGL, PYH1N1, PZP, RGS13, RIMS2, RNF24, ROS1, RXRG, SCEL, SCUBE3, SEMA3D, SERGEF, SERPINA1, SERPINA2, SHC1, SLAMF6, SLC24A5, SLC31A1, SLC34A2, SLC35B1, SLC43A3, SLC4A1 et SLC4A4.

**13.** Procédé selon la revendication 1, dans lequel ledit échantillon de tissu thyroïdien est un échantillon de tissu thyroïdien d'aspirats à l'aiguille fine.

**14.** Procédé selon la revendication 2, dans lequel ledit classificateur a été formé à l'aide :

(i) plus de 200 échantillons cliniques, ou
(ii) des échantillons dérivés d'au moins 5 localisations géographiques différentes.

**15.** Procédé selon la revendication 1, dans lequel ladite classification comprend l'utilisation d'un algorithme d'apprentissage automatique.

FIGURE 1A

**FIGURE 1B**

*103* Microarray scan

*104* CEL and checksum files

*105*
1. Normalization and summarization
2. Classification
3. Reporting

*106* Result file and other outputs

*107* Classifier Cassette Execution List
1. MTC
2. RCC
3. PTA
4. BCA
5. MMM
6. HC
7. BS

1. to 6. are specialized cassettes, mostly for metastatic cancers.

7. is the SVM cassette for thyroid cancers.

*108* Classifier & parameter files

*102* Supervisor

*101* Command line interface

# FIGURE 1C

## FIGURE 2

### Gene Biomarker Panels for Diagnosing a Thyroid Condition

| 1 | Normal Thyroid (NML) |
| 2 | Lymphocytic, Autoimmune Thyroiditis (LCT) |
| 3 | Nodular Hyperplasia (NHP) |
| 4 | Follicular Thyroid Adenoma (FA) |
| 5 | Hurthle Cell Thyroid Adenoma (HA) |
| 6 | Parathyroid (non thyroid tissue) (PTA) |
| 7 | Anaplastic Thyroid Carcinoma (ATC) |
| 8 | Follicular Thyroid Carcinoma (FC) |
| 9 | Hurthle Cell Thyroid Carcinoma (HC) |
| 10 | Papillary Thyroid Carcinoma (PTC) |
| 11 | Follicular Variant of Papillary Carcinoma (FVPTC) |
| 12 | Medullary Thyroid Carcinoma (MTC) |
| 13 | Renal Carcinoma metastasis to the Thyroid (RCC) |
| 14 | Melanoma metastasis to the Thyroid (MMN) |
| 15 | B cell Lymphoma metastasis to the Thyroid (BCL) |
| 16 | Breast Carcinoma metastasis to the Thyroid (BCA) |

**FIGURE 3**

**Optimized Gene Classification Panels of Diagnostic Utility in Thyroid FNA**

| Classification Panel | Subtype-specific Classifier Components | Number of Markers Per Panel |
|---|---|---|
| 1 | MTC | 5 |
| 2 | RCC | 5 |
| 3 | PTA | 5 |
| 4 | BCA | 5 |
| 5 | MMN | 5 |
| 6 | HA & HC[1] | 33 |
| 7 | Benign/Suspicious[2] | 142 |

[1] denotes the combination of two panels -HA and HC- into a single panel.

[2] denotes the combination of eight panels -FA, FC, NHP, PTC, FVPTC, LCT, HA, HC- into a single panel.

**FIGURE 4A**

| TCID | GENE | Gene Description | Classification Panel |
|---|---|---|---|
| 3364127 | CALCA | calcitonin-related polypeptide alpha | MTC |
| 3834341 | CEACAM5 | carcinoembryonic antigen-related cell adhesion molecule 5 | MTC |
| 3594003 | SCG3 | secretogranin III | MTC |
| 2585400 | SCN2A | sodium channel, voltage-gated, type II, alpha subunit | MTC |
| 2585400 | SCN9A | sodium channel, voltage-gated, type IX, alpha subunit | MTC |
| 3805614 | SYT4 | synaptotagmin IV | MTC |
| 2923928 | FABP7 | fatty acid binding protein 7, brain | RCC |
| 3393446 | FXYD2 | FXYD domain containing ion transport regulator 2 | RCC |
| 2883317 | HAVCR1 | hepatitis A virus cellular receptor 1 | RCC |
| 2883317 | LOC100101266 | hepatitis A virus cellular receptor 1 pseudogene | RCC |
| 3428225 | NR1H4 | nuclear receptor subfamily 1, group H, member 4 | RCC |
| 2479698 | PREPL | prolyl endopeptidase-like | RCC |
| 2479698 | SLC3A1 | solute carrier family 3 (cystine, dibasic and neutral amino acid transporters, activator of cystine, dibasic and neutral amino acid transport), member 1 | RCC |
| 3159754 | DMRT2 | doublesex and mab-3 related transcription factor 2 | PTA |
| 2941690 | GCM2 | glial cells missing homolog 2 (Drosophila) | PTA |
| 3363686 | KIDINS220 | kinase D-interacting substrate, 220kDa | PTA |
| 3484895 | KL | klotho | PTA |
| 3363686 | PTH | parathyroid hormone | PTA |
| 2894790 | SYCP2L | synaptonemal complex protein 2-like | PTA |
| 3039830 | AGR3 | anterior gradient homolog 3 (Xenopus laevis) | BCA |
| 3264997 | C10orf81 | chromosome 10 open reading frame 81 | BCA |
| 2926802 | MYB | v-myb myeloblastosis viral oncogene homolog (avian) | BCA |
| 3912079 | SYCP2 | synaptonemal complex protein 2 | BCA |
| 2430163 | VTCN1 | V-set domain containing T cell activation inhibitor 1 | BCA |
| 3811949 | CDH19 | cadherin 19, type 2 | MMN |

**FIGURE 4B**

| TCID | GENE | Gene Description | Classification Panel |
|---|---|---|---|
| 3161261 | MLANA | melan-A | MMN |
| 3935486 | S100B | S100 calcium binding protein B | MMN |
| 3457336 | SILV | silver homolog (mouse) | MMN |
| 3343832 | TYR | tyrosinase (oculocutaneous albinism IA) | MMN |
| 3343832 | TYRL | tyrosinase-like (pseudogene) | MMN |
| 2566848 | AFF3 | AF4/FMR2 family, member 3 | HA & HC, Benign/Suspicious |
| 2988882 | AIMP2 | aminoacyl tRNA synthetase complex-interacting multifunctional protein 2 | HA & HC, Benign/Suspicious |
| 3169331 | ALDH1B1 | aldehyde dehydrogenase 1 family, member B1 | HA & HC, Benign/Suspicious |
| 2984616 | BRP44L | brain protein 44-like | HA & HC, Benign/Suspicious |
| 2822492 | C5orf30 | chromosome 5 open reading frame 30 | HA & HC, Benign/Suspicious |
| 3326635 | CD44 | CD44 molecule (Indian blood group) | HA & HC, Benign/Suspicious |
| 2750627 | CPE | carboxypeptidase E | HA & HC, Benign/Suspicious |
| 3042001 | CYCS | cytochrome c, somatic | HA & HC, Benign/Suspicious |
| 3122678 | DEFB1 | defensin, beta 1 | HA & HC, Benign/Suspicious |
| 2739308 | EGF | epidermal growth factor (beta-urogastrone) | HA & HC, Benign/Suspicious |
| 2988882 | EIF2AK1 | eukaryotic translation initiation factor 2-alpha kinase 1 | HA & HC, Benign/Suspicious |
| 3603932 | FAH | fumarylacetoacetate hydrolase (fumarylacetoacetase) | HA & HC, Benign/Suspicious |
| 2970897 | FRK | fyn-related kinase | HA & HC, Benign/Suspicious |
| 3212008 | FRMD3 | FERM domain containing 3 | HA & HC, Benign/Suspicious |
| 3302990 | GOT1 | glutamic-oxaloacetic transaminase 1, soluble (aspartate aminotransferase 1) | HA & HC, Benign/Suspicious |
| 3417703 | HSD17B6 | hydroxysteroid (17-beta) dehydrogenase 6 homolog (mouse) | HA & HC, Benign/Suspicious |
| 2877508 | HSPA9 | heat shock 70kDa protein 9 (mortalin) | HA & HC, Benign/Suspicious |

## FIGURE 4C

| TCID | GENE | Gene Description | Classification Panel |
|---|---|---|---|
| 2708922 | IGF2BP2 | insulin-like growth factor 2 mRNA binding protein 2 | HA & HC, Benign/Suspicious |
| 2604998 | IQCA1 | IQ motif containing with AAA domain 1 | HA & HC, Benign/Suspicious |
| 3724545 | ITGB3 | integrin, beta 3 (platelet glycoprotein IIIa, antigen CD61) | HA & HC, Benign/Suspicious |
| 3397774 | KCNJ1 | potassium inwardly-rectifying channel, subfamily J, member 1 | HA & HC, Benign/Suspicious |
| 2604998 | LOC100129258 | hypothetical protein LOC100129258 | HA & HC, Benign/Suspicious |
| 3009299 | MDH2 | malate dehydrogenase 2, NAD (mitochondrial) | HA & HC, Benign/Suspicious |
| 3654699 | NUPR1 | nuclear protein, transcriptional regulator, 1 | HA & HC, Benign/Suspicious |
| 4020655 | ODZ1 | odz, odd Oz/ten-m homolog 1(Drosophila) | HA & HC, Benign/Suspicious |
| 3970833 | PDHA1 | pyruvate dehydrogenase (lipoamide) alpha 1 | HA & HC, Benign/Suspicious |
| 2377094 | PFKFB2 | 6-phosphofructo-2-kinase/fructose-2,6-biphosphatase 2 | HA & HC, Benign/Suspicious |
| 3278198 | PHYH | phytanoyl-CoA 2-hydroxylase | HA & HC, Benign/Suspicious |
| 2880051 | PPP2R2B | protein phosphatase 2 (formerly 2A), regulatory subunit B, beta isoform | HA & HC, Benign/Suspicious |
| 3959862 | PVALB | parvalbumin | HA & HC, Benign/Suspicious |
| 2688499 | PVRL2 | poliovirus receptor-related 2 (herpesvirus entry mediator B) | HA & HC, Benign/Suspicious |
| 2604998 | RPL3 | ribosomal protein L3 | HA & HC, Benign/Suspicious |
| 2964231 | RRAGD | Ras-related GTP binding D | HA & HC, Benign/Suspicious |
| 2798538 | SDHA | succinate dehydrogenase complex, subunit A, flavoprotein (Fp) | HA & HC, Benign/Suspicious |
| 2798538 | SDHALP1 | succinate dehydrogenase complex, subunit A, flavoprotein pseudogene 1 | HA & HC, Benign/Suspicious |
| 2798538 | SDHALP2 | succinate dehydrogenase complex, subunit A, flavoprotein pseudogene 2 | HA & HC, Benign/Suspicious |

# FIGURE 4D

| TCID | GENE | Gene Description | Classification Panel |
|---|---|---|---|
| 2798538 | SDHAP3 | succinate dehydrogenase complex, subunit A, flavoprotein pseudogene 3 | HA & HC, Benign/Suspicious |
| 2428501 | SLC16A1 | solute carrier family 16, member 1 (monocarboxylic acid transporter 1) | HA & HC, Benign/Suspicious |
| 2877508 | SNORD63 | small nucleolar RNA, C/D box 63 | HA & HC, Benign/Suspicious |
| 2562529 | ST3GAL5 | ST3 beta-galactoside alpha-2,3-sialyltransferase 5 | HA & HC, Benign/Suspicious |
| 2688499 | ZBED2 | zinc finger, BED-type containing 2 | HA & HC, Benign/Suspicious |
| 3450861 | ABCD2 | ATP-binding cassette, sub-family D (ALD), member 2 | Benign/Suspicious |
| 3341061 | ACER3 | alkaline ceramidase 3 | Benign/Suspicious |
| 2796553 | ACSL1 | acyl-CoA synthetase long-chain family member 1 | Benign/Suspicious |
| 3375735 | AHNAK | AHNAK nucleoprotein | Benign/Suspicious |
| 2439554 | AIM2 | absent in melanoma 2 | Benign/Suspicious |
| 3768474 | ARSG | arylsulfatase G | Benign/Suspicious |
| 3214845 | ASPN | asporin | Benign/Suspicious |
| 3006572 | AUTS2 | autism susceptibility candidate 2 | Benign/Suspicious |
| 3902489 | BCL2L1 | BCL2-like 1 | Benign/Suspicious |
| 2688717 | BTLA | B and T lymphocyte associated | Benign/Suspicious |
| 2708855 | C11orf72 | chromosome 11 open reading frame 72 | Benign/Suspicious |
| 2730303 | C4orf7 | chromosome 4 open reading frame 7 | Benign/Suspicious |
| 3259367 | CC2D2B | coiled-coil and C2 domain containing 2B | Benign/Suspicious |
| 3204285 | CCL19 | chemokine (C-C motif) ligand 19 | Benign/Suspicious |
| 3338192 | CCND1 | cyclin D1 | Benign/Suspicious |
| 3010503 | CD36 | CD36 molecule (thrombospondin receptor) | Benign/Suspicious |
| 2326463 | CD52 | CD52 molecule | Benign/Suspicious |
| 2635741 | CD96 | CD96 molecule | Benign/Suspicious |
| 2373336 | CFH | complement factor H | Benign/Suspicious |
| 2373336 | CFHR1 | complement factor H-related 1 | Benign/Suspicious |
| 2710599 | CLDN1 | claudin 1 | Benign/Suspicious |
| 2657808 | CLDN16 | claudin 16 | Benign/Suspicious |

## FIGURE 4E

| TCID | GENE | Gene Description | Classification Panel |
|------|------|------------------|---------------------|
| 2377283 | CR2 | complement component (3d/Epstein Barr virus) receptor 2 | Benign/Suspicious |
| 3242353 | CREM | cAMP responsive element modulator | Benign/Suspicious |
| 2490351 | CTNNA2 | catenin (cadherin-associated protein), alpha 2 | Benign/Suspicious |
| 2732508 | CXCL13 | chemokine (C-X-C motif) ligand 13 | Benign/Suspicious |
| 2854445 | DAB2 | disabled homolog 2, mitogen-responsive phosphoprotein (Drosophila) | Benign/Suspicious |
| 2321911 | DDI2 | DDI1, DNA-damage inducible 1, homolog 2 (S. cerevisiae) | Benign/Suspicious |
| 2642791 | DNAJC13 | DnaJ (Hsp40) homolog, subfamily C, member 13 | Benign/Suspicious |
| 2584018 | DPP4 | dipeptidyl-peptidase 4 | Benign/Suspicious |
| 3032647 | DPP6 | dipeptidyl-peptidase 6 | Benign/Suspicious |
| 2981874 | DYNLT1 | dynein, light chain, Tctex-type 1 | Benign/Suspicious |
| 2638676 | EAF2 | ELL associated factor 2 | Benign/Suspicious |
| 3852832 | EMR3 | egf-like module containing, mucin-like, hormone receptor-like 3 | Benign/Suspicious |
| 3142381 | FABP4 | fatty acid binding protein 4, adipocyte | Benign/Suspicious |
| 2396750 | FBXO2 | F-box protein 2 | Benign/Suspicious |
| 3338192 | FLJ42258 | FLJ42258 protein | Benign/Suspicious |
| 2526806 | FN1 | fibronectin 1 | Benign/Suspicious |
| 2598261 | FN1 | fibronectin 1 | Benign/Suspicious |
| 3839910 | FPR2 | formyl peptide receptor 2 | Benign/Suspicious |
| 3486096 | FREM2 | FRAS1 related extracellular matrix protein 2 | Benign/Suspicious |
| 3393479 | FXYD6 | FXYD domain containing ion transport regulator 6 | Benign/Suspicious |
| 2378068 | G0S2 | G0/G1switch 2 | Benign/Suspicious |
| 2884845 | GABRB2 | gamma-aminobutyric acid (GABA) A receptor, beta 2 | Benign/Suspicious |
| 3063795 | GAL3ST4 | galactose-3-O-sulfotransferase 4 | Benign/Suspicious |
| 3031556 | GIMAP2 | GTPase, IMAP family member 2 | Benign/Suspicious |
| 3861948 | GMFG | glia maturation factor, gamma | Benign/Suspicious |
| 3540862 | GPHN | gephyrin | Benign/Suspicious |
| 3982612 | GPR174 | G protein-coupled receptor 174 | Benign/Suspicious |
| 2809793 | GZMK | granzyme K (granzyme 3; tryptase II) | Benign/Suspicious |
| 2638676 | HCG11 | HLA complex group 11 | Benign/Suspicious |
| 2352609 | HNRNPA3 | heterogeneous nuclear ribonucleoprotein A3 | Benign/Suspicious |
| 3375735 | IGHG1 | immunoglobulin heavy constant gamma 1 (G1m marker) | Benign/Suspicious |
| 2806468 | IL7R | interleukin 7 receptor | Benign/Suspicious |

## FIGURE 4F

| TCID | GENE | Gene Description | Classification Panel |
|------|------|------------------|----------------------|
| 3852832 | ITGB1 | integrin, beta 1 (fibronectin receptor, beta polypeptide, antigen CD29 includes MDF2, MSK12) | Benign/Suspicious |
| 2427619 | KCNA3 | potassium voltage-gated channel, shaker-related subfamily, member 3 | Benign/Suspicious |
| 3404030 | KLRG1 | killer cell lectin-like receptor subfamily G, member 1 | Benign/Suspicious |
| 3512874 | LCP1 | lymphocyte cytosolic protein 1 (L-plastin) | Benign/Suspicious |
| 2708855 | LIPH | lipase, member H | Benign/Suspicious |
| 3875642 | LOC100131599 | hypothetical protein LOC100131599 | Benign/Suspicious |
| 2638676 | LOC647979 | hypothetical LOC647979 | Benign/Suspicious |
| 3147985 | LRP12 | low density lipoprotein-related protein 12 | Benign/Suspicious |
| 2578790 | LRP1B | low density lipoprotein-related protein 1B (deleted in tumors) | Benign/Suspicious |
| 2352609 | MAGI3 | membrane associated guanylate kinase, WW and PDZ domain containing 3 | Benign/Suspicious |
| 3111561 | MAPK6 | mitogen-activated protein kinase 6 | Benign/Suspicious |
| 3108526 | MATN2 | matrilin 2 | Benign/Suspicious |
| 3329343 | MDK | midkine (neurite growth-promoting factor 2) | Benign/Suspicious |
| 3367673 | MPPED2 | metallophosphoesterase domain containing 2 | Benign/Suspicious |
| 3662201 | MT1F | metallothionein 1F | Benign/Suspicious |
| 3692999 | MT1G | metallothionein 1G | Benign/Suspicious |
| 3662201 | MT1H | metallothionein 1H | Benign/Suspicious |
| 3662201 | MT1P2 | metallothionein 1 pseudogene 2 | Benign/Suspicious |
| 3622934 | MYEF2 | myelin expression factor 2 | Benign/Suspicious |
| 3341497 | NDUFC2 | NADH dehydrogenase (ubiquinone) 1, subcomplex unknown, 2, 14.5kDa | Benign/Suspicious |
| 3067478 | NRCAM | neuronal cell adhesion molecule | Benign/Suspicious |
| 3353914 | OR10D1P | olfactory receptor, family 10, subfamily D, member 1 pseudogene | Benign/Suspicious |
| 3982560 | P2RY10 | purinergic receptor P2Y, G-protein coupled, 10 | Benign/Suspicious |
| 2701071 | P2RY13 | purinergic receptor P2Y, G-protein coupled, 13 | Benign/Suspicious |
| 3948047 | PARVG | parvin, gamma | Benign/Suspicious |
| 3606034 | PDE8A | phosphodiesterase 8A | Benign/Suspicious |
| 3811086 | PIGN | phosphatidylinositol glycan anchor biosynthesis, class N | Benign/Suspicious |
| 3744680 | PIK3R5 | phosphoinositide-3-kinase, regulatory subunit 5 | Benign/Suspicious |
| 3111561 | PKHD1L1 | polycystic kidney and hepatic disease 1 (autosomal recessive)-like 1 | Benign/Suspicious |
| 3376529 | PLA2G16 | phospholipase A2, group XVI | Benign/Suspicious |

| TCID | GENE | Gene Description | Classification Panel |
|---|---|---|---|
| 3875642 | PLCB1 | phospholipase C, beta 1 (phosphoinositide-specific) | Benign/Suspicious |
| 2486811 | PLEK | pleckstrin | Benign/Suspicious |
| 3246888 | PRKG1 | protein kinase, cGMP-dependent, type 1 | Benign/Suspicious |
| 3874751 | PRNP | prion protein | Benign/Suspicious |
| 2685304 | PROS1 | protein S (alpha) | Benign/Suspicious |
| 2373842 | PTPRC | protein tyrosine phosphatase, receptor type, C | Benign/Suspicious |
| 3270270 | PTPRE | protein tyrosine phosphatase, receptor type, E | Benign/Suspicious |
| 3564210 | PYGL | phosphorylase, glycogen, liver | Benign/Suspicious |
| 2362351 | PYHIN1 | pyrin and HIN domain family, member 1 | Benign/Suspicious |
| 3443464 | PZP | pregnancy-zone protein | Benign/Suspicious |
| 2372812 | RGS13 | regulator of G-protein signaling 13 | Benign/Suspicious |
| 3110395 | RIMS2 | regulating synaptic membrane exocytosis 2 | Benign/Suspicious |
| 3895795 | RNF24 | ring finger protein 24 | Benign/Suspicious |
| 2721959 | ROS1 | c-ros oncogene 1 , receptor tyrosine kinase | Benign/Suspicious |
| 2442008 | RXRG | retinoid X receptor, gamma | Benign/Suspicious |
| 3494629 | SCEL | sciellin | Benign/Suspicious |
| 2904485 | SCUBE3 | signal peptide, CUB domain, EGF-like 3 | Benign/Suspicious |
| 3059667 | SEMA3D | sema domain, immunoglobulin domain (Ig), short basic domain, secreted, (semaphorin) 3D | Benign/Suspicious |
| 3365136 | SERGEF | secretion regulating guanine nucleotide exchange factor | Benign/Suspicious |
| 3577612 | SERPINA1 | serpin peptidase inhibitor, clade A (alpha-1 antiproteinase, antitrypsin), member 1 | Benign/Suspicious |
| 3577612 | SERPINA2 | serpin peptidase inhibitor, clade A (alpha-1 antiproteinase, antitrypsin), member 2 | Benign/Suspicious |
| 3759006 | SHC1 | SHC (Src homology 2 domain containing) transforming protein 1 | Benign/Suspicious |
| 2440258 | SLAMF6 | SLAM family member 6 | Benign/Suspicious |
| 3622934 | SLC24A5 | solute carrier family 24, member 5 | Benign/Suspicious |
| 3185522 | SLC31A1 | solute carrier family 31 (copper transporters), member 1 | Benign/Suspicious |
| 2721959 | SLC34A2 | solute carrier family 34 (sodium phosphate), member 2 | Benign/Suspicious |
| 3761959 | SLC35B1 | solute carrier family 35, member B1 | Benign/Suspicious |
| 3373845 | SLC43A3 | solute carrier family 43, member 3 | Benign/Suspicious |
| 3759006 | SLC4A1 | solute carrier family 4, anion exchanger, member 1 (erythrocyte membrane protein band 3, Diego blood group) | Benign/Suspicious |
| 2730746 | SLC4A4 | solute carrier family 4, sodium bicarbonate cotransporter, member 4 | Benign/Suspicious |

## FIGURE 4H

| TCID | GENE | Gene Description | Classification Panel |
|---|---|---|---|
| 2777714 | SNCA | synuclein, alpha (non A4 component of amyloid precursor) | Benign/Suspicious |
| 2834282 | STK32A | serine/threonine kinase 32A | Benign/Suspicious |
| 3341497 | THRSP | thyroid hormone responsive (SPOT14 homolog, rat) | Benign/Suspicious |
| 3976341 | TIMP1 | TIMP metallopeptidase inhibitor 1 | Benign/Suspicious |
| 3772661 | TIMP2 | TIMP metallopeptidase inhibitor 2 | Benign/Suspicious |
| 2491271 | TMSB10 | thymosin beta 10 | Benign/Suspicious |
| 3648391 | TNFRSF17 | tumor necrosis factor receptor superfamily, member 17 | Benign/Suspicious |
| 3441849 | TNFRSF1A | tumor necrosis factor receptor superfamily, member 1A | Benign/Suspicious |
| 2412668 | TXNDC12 | thioredoxin domain containing 12 (endoplasmic reticulum) | Benign/Suspicious |
| 3353914 | VWA5A | von Willebrand factor A domain containing 5A | Benign/Suspicious |
| 3976766 | WAS | Wiskott-Aldrich syndrome (eczema-thrombocytopenia) | Benign/Suspicious |
| 3768474 | WIPI1 | WD repeat domain, phosphoinositide interacting 1 | Benign/Suspicious |
| 2817731 | ZFYVE16 | zinc finger, FYVE domain containing 16 | Benign/Suspicious |
| 4027585 | unknown | | Benign/Suspicious |

**FIGURE 5A**

| | Malignant State | | | Benign State | | |
|---|---|---|---|---|---|---|
| **Marker** | **Subtype 1** | **Subtype 2** | **Subtype 3** | **Subtype 4** | **Subtype 5** | **Subtype 6** |
| Gene 1 | **up-regulated** | *down-regulated* | not differentially expressed | *down-regulated* | not differentially expressed | *down-regulated* |
| Gene 2 | **up-regulated** | *down-regulated* | not differentially expressed | *down-regulated* | not differentially expressed | *down-regulated* |
| Gene 3 | **up-regulated** | *down-regulated* | not differentially expressed | *down-regulated* | not differentially expressed | *down-regulated* |
| Gene 4 | **up-regulated** | *down-regulated* | **up-regulated** | *down-regulated* | not differentially expressed | *down-regulated* |
| Gene 5 | **up-regulated** | *down-regulated* | **up-regulated** | *down-regulated* | not differentially expressed | not differentially expressed |
| Gene 6 | **up-regulated** | *down-regulated* | **up-regulated** | *down-regulated* | not differentially expressed | not differentially expressed |
| Gene 7 | **up-regulated** | *down-regulated* | *down-regulated* | not differentially expressed | not differentially expressed | not differentially expressed |
| Gene 8 | **up-regulated** | not differentially expressed | *down-regulated* | not differentially expressed | **up-regulated** | not differentially expressed |
| Gene 9 | **up-regulated** | not differentially expressed | *down-regulated* | not differentially expressed | **up-regulated** | not differentially expressed |
| Gene 10 | **up-regulated** | not differentially expressed | not differentially expressed | not differentially expressed | *down-regulated* | **up-regulated** |
| Gene 11 | **up-regulated** | not differentially expressed | not differentially expressed | not differentially expressed | *down-regulated* | **up-regulated** |

FIGURE 5B

| Marker | Malignant State | | | Benign State | | |
|---|---|---|---|---|---|---|
| | Subtype 1 | Subtype 2 | Subtype 3 | Subtype 4 | Subtype 5 | Subtype 6 |
| Gene 12 | **up-regulated** | **up-regulated** | not differentially expressed | not differentially expressed | *down-regulated* | **up-regulated** |
| Gene 13 | not differentially expressed | **up-regulated** | **up-regulated** | not differentially expressed | *down-regulated* | not differentially expressed |
| Gene 14 | not differentially expressed | **up-regulated** | **up-regulated** | not differentially expressed | *down-regulated* | not differentially expressed |
| Gene 15 | not differentially expressed | **up-regulated** | **up-regulated** | not differentially expressed | *down-regulated* | not differentially expressed |
| Gene 16 | not differentially expressed | **up-regulated** | **up-regulated** | not differentially expressed | not differentially expressed | not differentially expressed |
| Gene 17 | *down-regulated* | **up-regulated** | **up-regulated** | not differentially expressed | not differentially expressed | not differentially expressed |
| Gene 18 | *down-regulated* | **up-regulated** | **up-regulated** | not differentially expressed | not differentially expressed | not differentially expressed |
| Gene 19 | *down-regulated* | **up-regulated** | **up-regulated** | **up-regulated** | not differentially expressed | not differentially expressed |
| Gene 20 | *down-regulated* | **up-regulated** | **up-regulated** | **up-regulated** | not differentially expressed | not differentially expressed |

**FIGURE 6A**

| Marker | Malignant State | | | Benign State | | |
|---|---|---|---|---|---|---|
| | Subtype 1 | Subtype 2 | Subtype 3 | Subtype 4 | Subtype 5 | Subtype 6 |
| Gene 21 | up-regulated | *down-regulated* | not differentially expressed | *down-regulated* | not differentially expressed | *down-regulated* |
| Gene 22 | up-regulated | *down-regulated* | not differentially expressed | *down-regulated* | not differentially expressed | *down-regulated* |
| Gene 23 | up-regulated | *down-regulated* | not differentially expressed | *down-regulated* | not differentially expressed | *down-regulated* |
| Gene 24 | up-regulated | *down-regulated* | up-regulated | *down-regulated* | not differentially expressed | *down-regulated* |
| Gene 25 | up-regulated | *down-regulated* | up-regulated | *down-regulated* | not differentially expressed | not differentially expressed |
| Gene 26 | up-regulated | *down-regulated* | up-regulated | *down-regulated* | not differentially expressed | not differentially expressed |
| Gene 27 | up-regulated | *down-regulated* | *down-regulated* | not differentially expressed | not differentially expressed | not differentially expressed |
| Gene 28 | up-regulated | not differentially expressed | *down-regulated* | not differentially expressed | up-regulated | not differentially expressed |
| Gene 29 | up-regulated | not differentially expressed | *down-regulated* | not differentially expressed | up-regulated | not differentially expressed |
| Gene 30 | up-regulated | not differentially expressed | not differentially expressed | not differentially expressed | *down-regulated* | up-regulated |
| Gene 31 | up-regulated | not differentially expressed | not differentially expressed | not differentially expressed | *down-regulated* | up-regulated |

FIGURE 6B

| Marker | Malignant State | | | Benign State | | |
|--------|-----------|-----------|-----------|-----------|-----------|-----------|
| | Subtype 1 | Subtype 2 | Subtype 3 | Subtype 4 | Subtype 5 | Subtype 6 |
| Gene 32 | up-regulated | up-regulated | not differentially expressed | not differentially expressed | *down-regulated* | up-regulated |
| Gene 33 | not differentially expressed | up-regulated | up-regulated | not differentially expressed | *down-regulated* | not differentially expressed |
| Gene 34 | not differentially expressed | up-regulated | up-regulated | not differentially expressed | *down-regulated* | not differentially expressed |
| Gene 35 | not differentially expressed | up-regulated | up-regulated | not differentially expressed | *down-regulated* | not differentially expressed |
| Gene 36 | not differentially expressed | up-regulated | up-regulated | not differentially expressed | not differentially expressed | not differentially expressed |
| Gene 37 | *down-regulated* | up-regulated | up-regulated | not differentially expressed | not differentially expressed | not differentially expressed |
| Gene 38 | *down-regulated* | up-regulated | up-regulated | not differentially expressed | not differentially expressed | not differentially expressed |
| Gene 39 | *down-regulated* | up-regulated | up-regulated | up-regulated | not differentially expressed | not differentially expressed |
| Gene 40 | *down-regulated* | up-regulated | up-regulated | up-regulated | not differentially expressed | not differentially expressed |

**FIGURE 7A**

| Marker | Malignant State | | | Benign State | | |
|---|---|---|---|---|---|---|
| | Subtype 1 | Subtype 2 | Subtype 3 | Subtype 4 | Subtype 5 | Subtype 6 |
| Gene 41 | not differentially expressed | up-regulated | *down-regulated* | up-regulated | not differentially expressed | not differentially expressed |
| Gene 42 | not differentially expressed | up-regulated | *down-regulated* | up-regulated | not differentially expressed | not differentially expressed |
| Gene 43 | not differentially expressed | up-regulated | *down-regulated* | up-regulated | not differentially expressed | not differentially expressed |
| Gene 44 | up-regulated | up-regulated | *down-regulated* | *down-regulated* | not differentially expressed | up-regulated |
| Gene 45 | *down-regulated* | up-regulated | *down-regulated* | up-regulated | not differentially expressed | up-regulated |
| Gene 46 | *down-regulated* | up-regulated | *down-regulated* | up-regulated | not differentially expressed | up-regulated |
| Gene 47 | *down-regulated* | not differentially expressed | *down-regulated* | up-regulated | up-regulated | up-regulated |
| Gene 48 | *down-regulated* | not differentially expressed | *down-regulated* | up-regulated | up-regulated | up-regulated |
| Gene 49 | *down-regulated* | not differentially expressed | up-regulated | up-regulated | up-regulated | *down-regulated* |
| Gene 50 | not differentially expressed | not differentially expressed | up-regulated | up-regulated | up-regulated | *down-regulated* |
| Gene 51 | not differentially expressed | down-regulated | up-regulated | up-regulated | up-regulated | *down-regulated* |

FIGURE 7B

| Marker | Malignant State | | | Benign State | | |
|---|---|---|---|---|---|---|
| | Subtype 1 | Subtype 2 | Subtype 3 | Subtype 4 | Subtype 5 | Subtype 6 |
| Gene 52 | not differentially expressed | *down-regulated* | **up-regulated** | **up-regulated** | **up-regulated** | not differentially expressed |
| Gene 53 | not differentially expressed | *down-regulated* | not differentially expressed | *down-regulated* | **up-regulated** | *down-regulated* |
| Gene 54 | not differentially expressed | *down-regulated* | not differentially expressed | *down-regulated* | not differentially expressed | *down-regulated* |
| Gene 55 | not differentially expressed | *down-regulated* | not differentially expressed | *down-regulated* | not differentially expressed | *down-regulated* |
| Gene 56 | **up-regulated** | *down-regulated* | not differentially expressed | not differentially expressed | **up-regulated** | *down-regulated* |
| Gene 57 | **up-regulated** | *down-regulated* | not differentially expressed | not differentially expressed | **up-regulated** | *down-regulated* |
| Gene 58 | **up-regulated** | *down-regulated* | **up-regulated** | not differentially expressed | **up-regulated** | *down-regulated* |
| Gene 59 | **up-regulated** | not differentially expressed | **up-regulated** | not differentially expressed | **up-regulated** | *down-regulated* |
| Gene 60 | **up-regulated** | not differentially expressed | **up-regulated** | not differentially expressed | **up-regulated** | *down-regulated* |

# FIGURE 8

| Gene Symbol na30 hg19 | TCID | Probeset ID | Repetability Score | BH adjusted LIMMA p-value | Gene Expression Effect Size | Final Rank |
|---|---|---|---|---|---|---|
| DEFB1 | 3122678 | 3122688 | 1 | 2.68E-09 | 2.14 | 2 |
| PVALB | 3959862 | 3959869 | 1 | 1.09E-08 | 2.30 | 3 |
| ALDH1B1 | 3169331 | 3169333 | 1 | 4.39E-10 | 1.40 | 4 |
| EGF | 2739308 | 2739364 | 1 | 4.72E-11 | 1.15 | 5 |
| KCNJ1 | 3397774 | 3397776 | 1 | 4.72E-11 | 1.08 | 6 |
| ITGB3 | 3724545 | 3724571 | 1 | 2.68E-09 | -1.57 | 7 |
| PFKFB2 | 2377094 | 2377112 | 1 | 1.53E-09 | 1.41 | 8 |
| NUPR1 | 3654699 | 3654707 | 1 | 6.46E-09 | 1.63 | 11 |
| HSD17B6 | 3417703 | 3417718 | 1 | 5.63E-06 | -2.33 | 12 |
| IGF2BP2 | 2708922 | 2708962 | 1 | 8.96E-08 | -1.70 | 16 |
| CD44 | 3326635 | 3326705 | 1 | 1.58E-09 | -1.17 | 17 |
| FRK | 2970897 | 2970926 | 1 | 1.07E-08 | 1.30 | 21 |
| SDHA | 2798538 | 2798548 | 1 | 3.87E-09 | 0.96 | 22 |
| SDHALP1 | 2798538 | 2798548 | 1 | 3.87E-09 | 0.96 | 22 |
| SDHALP2 | 2798538 | 2798548 | 1 | 3.87E-09 | 0.96 | 22 |
| SDHAP3 | 2798538 | 2798548 | 1 | 3.87E-09 | 0.96 | 22 |
| ODZ1 | 4020655 | 4020661 | 1 | 3.44E-05 | -1.91 | 23 |
| ST3GAL5 | 2562529 | 2562540 | 1 | 3.21E-08 | -1.24 | 25 |
| GOT1 | 3302990 | 3303003 | 1 | 8.96E-08 | 1.29 | 26 |
| RRAGD | 2964231 | 2964240 | 1 | 1.73E-07 | 1.27 | 27 |
| CPE | 2750627 | 2750676 | 1 | 9.64E-07 | -1.35 | 28 |
| PPP2R2B | 2880051 | 2880105 | 1 | 3.04E-09 | 0.78 | 29 |
| IQCA1 | 2604998 | 2605065 | 1 | 3.13E-08 | -1.05 | 31 |
| LOC100129258 | 2604998 | 2605065 | 1 | 3.13E-08 | -1.05 | 31 |
| RPL3 | 2604998 | 2605065 | 1 | 3.13E-08 | -1.05 | 31 |
| HSPA9 | 2877508 | 2877521 | 1 | 5.90E-09 | 0.85 | 32 |
| SNORD63 | 2877508 | 2877521 | 1 | 5.90E-09 | 0.85 | 32 |
| PHYH | 3278198 | 3278220 | 1 | 4.44E-08 | 1.13 | 33 |
| C5orf30 | 2822492 | 2822501 | 1 | 3.13E-08 | 0.98 | 35 |
| AIMP2 | 2988882 | 2988893 | 1 | 1.39E-08 | 0.92 | 39 |
| EIF2AK1 | 2988882 | 2988893 | 1 | 1.39E-08 | 0.92 | 39 |
| PVRL2 | 2688499 | 2688512 | 1 | 1.89E-06 | -1.35 | 40 |
| ZBED2 | 2688499 | 2688512 | 1 | 1.89E-06 | -1.35 | 40 |
| PDHA1 | 3970833 | 3970874 | 1 | 4.39E-09 | 0.74 | 41 |
| SLC16A1 | 2428501 | 2428504 | 1 | 3.12E-07 | 1.14 | 42 |
| FAH | 3603932 | 3603960 | 1 | 3.87E-09 | 0.65 | 43 |
| MDH2 | 3009299 | 3009305 | 1 | 2.28E-08 | 0.87 | 44 |
| AFF3 | 2566848 | 2566923 | 1 | 2.30E-09 | 0.40 | 46 |
| BRP44L | 2984616 | 2984647 | 1 | 3.21E-08 | 0.90 | 48 |
| CYCS | 3042001 | 3042003 | 1 | 6.55E-08 | 0.93 | 49 |
| FRMD3 | 3212008 | 3212070 | 1 | 1.44E-05 | -1.42 | 50 |

## FIGURE 9

**A** — Sensitivity

**B** — Specificity

**C**

|  | Post-surgical Histopathology | | | | | | | |
|---|---|---|---|---|---|---|---|---|
| Classifier Prediction | NHP | LCT | FA | BLN | PTC | FVPTC | HC | MLN |
| Benign | 12 | 3 | 13 | 1 | 0 | 1 | 0 | 0 |
| Suspicious | 3 | 1 | 1 | 0 | 6 | 4 | 2 | 1 |
| Total | 15 | 4 | 14 | 1 | 6 | 5 | 2 | 1 |

**D**

|  | Post-surgical Histopathology | | | | | |
|---|---|---|---|---|---|---|
| Classifier Prediction | NHP | LCT | FA | PTC | FVPTC | HC |
| Benign | 5 | 2 | 6 | 0 | 0 | 0 |
| Suspicious | 2 | 1 | 1 | 2 | 3 | 2 |
| Total | 7 | 3 | 7 | 2 | 3 | 2 |

**FIGURE 10**

# FIGURE 11

| Subtype | Tissue Training Set (n=178) | FNA Training Set (n=137) | Independent FNA Test Set Benign, Malignant, & Indeterminate (n=48) | Independent FNA Test Set Indeterminate only (n=24) |
|---|---|---|---|---|
| NHP | 23 | 67 | 15 | 7 |
| LCT | 40 | 18 | 4 | 3 |
| FA | 26 | 9 | 14 | 7 |
| HA | 0 | 2 | 0 | 0 |
| BLN | 0 | 0 | 1 | 0 |
| **Total Benign** | **89** | **96**[a] | **34**[b] | **17** |
| HC | 23 | 0 | 2 | 2 |
| FC | 19 | 3 | 0 | 0 |
| FVPTC | 21 | 4 | 5 | 3 |
| PTC | 26 | 34 | 6 | 2 |
| MLN | 0 | 0 | 1 | 0 |
| **Total Malignant** | **89** | **41**[c] | **14** | **7** |

A subset of samples did not have post-surgical histopathology labels; (a) 68/96, (b) 6/34, and (c) 4/41. Abbreviations: FA, follicular adenoma; FC, follicular carcinoma; FVPTC, follicular variant of papillary carcinoma; HA, follicular adenoma; LCT, lymphocytic thyroiditis; NHP, nodular hyperplasia; PTC, papillary thyroid carcinoma; BLN, benign lymph node; MLN, malignant lymph node.

## FIGURE 12

## FIGURE 13

# FIGURE 14

| KEGG Pathways ORA | | | |
|---|---|---|---|
| Subcategory | Number of Genes Expected | Number of Genes Observed | p-value (FDR) |
| Cell adhesion molecules (CAMs) | 8 | 28 | 4.17e-07 |
| Olfactory transduction | 24 | **3** | **1.80e-06** |
| Focal adhesion | 12 | 29 | 0.0003 |
| Adherens junction | 5 | 15 | 0.0006 |
| Arrhythmic right ventricular cardiomyopathy (ARVC) | 5 | 15 | 0.0006 |
| ECM-receptor interaction | 5 | 16 | 0.0006 |
| Metabolic pathways | 66 | **39** | **0.0006** |
| Tight junction | 8 | 21 | 0.0006 |
| Leukocyte transendothelial migration | 7 | 19 | 0.0009 |
| Complement coagulation cascades | 4 | 13 | 0.0021 |
| GO Pathways ORA | | | |
| Subcategory | Number of Genes Expected | Number of Genes Observed | p-value (FDR) |
| Plasma membrane | 113 | 220 | 2.82e-22 |
| Cell adhesion | 26 | 77 | 8.04e-16 |
| Defense response | 22 | 59 | 1.09e-09 |
| Nuclear part | 75 | **28** | **3.63e-09** |
| Organelle part | 150 | **87** | **1.40e-08** |
| Response to wounding | 17 | 47 | 1.97e-08 |
| Nucleus | 144 | **87** | **2.41e-07** |
| Extracellular region part | 26 | 59 | 2.41e-07 |
| Intracellular | 339 | **266** | **3.24e-07** |
| Locomotion | 17 | 44 | 6.47e-07 |

Numbers in regular font refer to pathways that are over-represented by top differentially expressed genes; those in bold refer to pathways that are under-represented

FIGURE 15

201
instructions

203
kit

200

202
container

205
reagents

FIGURE 16

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 3645691 A
- US 7335762 B
- US 7323305 B
- US 7264929 B
- US 7244559 B
- US 7211390 B
- US 7361488 B
- US 7300788 B
- US 7280922 B
- US 7358061 B
- US 7319011 B
- US 5965360 A
- US 6436642 B
- US 20030186248 A
- US 20050042222 A
- US 20030190602 A

- US 20050048533 A
- US 20050266443 A
- US 20060035244 A
- US 2006083744 A
- US 20060088851 A
- US 20060105360 A
- US 20060127907 A
- US 20070020657 A
- US 20070037186 A
- US 20070065833 A
- US 20070161004 A
- US 20070238119 A
- US 20080044824 A
- US 20080131892 A
- US 2006019615 A
- US 2009006162 W

**Non-patent literature cited in the description**

- **DURAND et al.** *Journal of Clinical Endocrinology & Metabolism,* vol. 93, 1195-1202
- *Bioinformatics,* 01 October 2007, vol. 23 (19), 2507-17
- *Cancer Inform.,* 2008, vol. 6, 77-97
- **COOPER et al.** *Thyroid,* 2006, vol. 16 (2
- **RAMZY ; IBRAHIM.** *Clinical Cytopathology and Aspiration Biopsy,* 2001
- **SAMBROOK.** Molecular Cloning a Laboratory Manual. 2001
- **BALDI, P. ; HATFIELD, W.G.** *DNA Microarrays*
- *Gene Expression,* 2002
- **IRIZARRY et al.** *Biostatistics,* April 2003, vol. 4 (2), 249-64
- **BOLSTAD et al.** *Bioinformatics,* 2003
- **TUKEY, J.W.** *Exploratory Data Analysis.,* 1977
- Limma: linear models for microarray data. **SMYTH, G. K.** Bioinformatics and Computational Biology Solutions using R and Bioconductor. Springer, 2005, 397-420

- **FISHEL ; KAUFMAN et al.** *Bioinformatics,* 2007, vol. 23 (13), 1599-606
- **SMYTH, G.K.** *Stat. Appl. Genet. Mol. Biol.,* 2004, vol. 3
- **SMYTH.** *Statistical applications in genetics and molecular biology,* 2004, vol. 3
- **DIAZ-URIARTE ; ALVAREZ DE ANDRES.** *BMC Bioinformatics,* 2006, vol. 7 (3
- **VARMA ; SIMON.** *BMC Bioinformatics,* 2006, vol. 7 (91
- **DIMITRIADOU et al.** *Misc Functions of the Department of Statistics,* 2009, e1071
- **CORTES ; VAPNIK.** *Machine Learning,* 2005, vol. 20, 273-297
- **PAUL.** *Fundamental Immunology,* 2008
- **BACKES et al.** *Nucleic Acids Research,* 2007, vol. 35, W186-192
- **KANEHISA et al.** *Nucleic Acids Research,* 2010, vol. 38, D355-360
- **ASHBURNER et al.** *Nature Genetics,* 2000, vol. 25, 25-29